# EUROPEAN PATENT APPLICATION

(11) **EP 2 018 862 A1**
(43) Date of publication of application: **28.01.2009**
(21) Application number: 08161040.4
(22) Date of filing: 24.07.2008
(51) Int. Cl.: A61K 31/404, A61K 31/5375, A61P 25/28, A61P 33/06, A61P 31/18

(54) **Substituted piperidines as therapeutic compounds**

(30) Priority: 25.07.2007 EP 07113144
(71) Applicant: Speedel Experimenta AG, 4123 Allschwil (CH)
(72) Inventor: Herold, Peter, 4123, Allschwil (CH); Mah, Robert, 4123, Allschwil (CH); Tschinke, Vincenzo, 4123, Allschwil (CH); Behnke, Dirk, 4123, Allschwil (CH); Jotterand, Nathalie, 4123, Allschwil (CH); Stojanovic, Aleksandar, 4123, Allschwil (CH); Stutz, Stefan, 4123, Allschwil (CH); Jelakovich, Stjepan, 4123, Allschwil (CH)
(74) Representative: Pommerenke, Alexander

(57) **Abstract**

Described are compounds of the general formula (I) 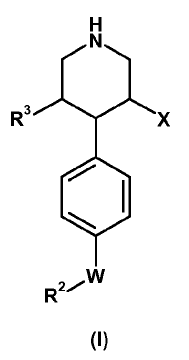 and pharmaceutically acceptable salt thereof, in which R², R³, W,and X have the definitions illustrated in detail in the description, as beta-secretase, cathepsin D, plasmepsin II and/or HIV protease inhibitors.

## Description

### Field of the Invention

The present invention relates to the use of substituted piperidines as beta-secretase-, cathepsin D-, plasmepsin II- and/or HIV-protease-inhibitors.

### Background of the Invention

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound C-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the proression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias.

### Alzheimer's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and

Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/ pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment.

It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity. As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data detailing both the catalytic mechanism and the molecular basis for substrate selection.

### Detailed Description of the Invention

Firstly, the present invention relates to compounds of the general formula 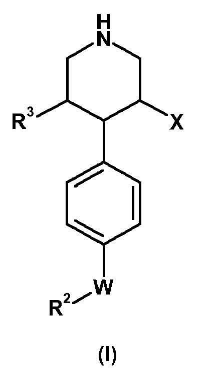 and their pharmaceutically acceptable salts for the inhibition of beta-secretase-, cathepsin D-, plasmepsin II- and/or HIV-protease, in which
(A) R¹ is heterocyclyl substituted by oxo or oxide or as indicated under (B) or (C), in particular azepanyl, benzo[1,3]dioxolyl, benzofuranyl, benzoimidazolyl, 4H-benzo[1,4]oxazinyl, benzooxazolyl, 4H-benzo[1,4]thiazinyl, quinolinyl, chromenyl, dihydro-benzo[e][1,4]diazepinyl, dihydrobenzofuranyl, 3,4-dihydro-2H-benzo[1,4]oxazinyl, dihydro-3H-benzo[1,4]oxazinyl, dihydro-benzo[d][1,3]oxazinyl, 3,4-dihydro-2H-benzo[1,4]thiazinyl, dihydro-2H-1λ6-benzo[1,4]thiazinyl, dihydro-1H-quinazolinyl, 1a,7b-dihydro-1H-cyclo-propa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1H-pyrido[2,3-b][1,4]oxazinyl, pyridyl, 1H-pyrrolizinyl, 1H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydrocyclopropa[c]chromenyl, tetrahydropyranyl or triazinyl; or
(B) R¹ is aryl which is substituted by 1-4-acetamidinyl-C₁₋₈alkyl, acyl-C₁₋₈alkoxy-C₁₋₈ alkyl, (N-acyl)-C₁₋₈alkoxy-C₁₋₈alkylamino, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkyl-carbamoyl, C₁₋₈alkoxy-C₁₋₈alkylcarbonyl, C₁₋₈alkoxy-C₁₋₈alkylcarbonylamino, 1-C₁₋₈alkoxy-C₁₋₈alkylimidazol-2-yl, 2-C₁₋₈alkoxy-C₁-₈alkyl-4-oxolmldazol-1-yl, 1-C₁₋₈alkoxy-C₁₋₈-alkyltetrazol-5-yl, 5-C₁₋₈alkoxy-C₁₋₈-alkyltetrazol-1-yl, 6-alkoxy-aminocarbonyl-C₁₋₈alkoxy, C₁₋₈alkoxyaminocarbonyl-C₁₋₈alkyl, C₁₋₈alkoxycarbonyl, C₁₋₈alkoxycarbonyl-C₁₋₈alkoxy, C₁₋₈alkoxycarbonyl-C₁₋₈alkyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₁₋₈alkoxy-C₁₋₈alkylcarbamoyl, (N-C₁₋₈alkyl)-C₁₋₈alkoxy-C₁₋₈alkylcarbonylamino, (N-C₁₋₈alkyl)-C₁₋₈alkoxycarbonylamino, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₁₋₈alkylsulfonylamino-C₁₋₈alkoxy, (N-C₁₋₈alkyl)-C₁₋₈alkylsulfonylamino-C₁₋₈alkyl, C₁₋₈alkylamidinyl, C₁₋₈alkylaminocarbonyl-C₁₋₈alkoxy, di-C₁₋₈alkylaminocarbonyl-C₁₋₈alkoxy, C₁₋₈alkylaminocarbonyl-C₁₋₈alkoxy-C₁₋₈alkyl, C₁₋₈alkylaminocarbonyl-C₁₋₈alkyl, C₁₋₈alkylaminocarbonylamino-C₁₋₈alkoxy, C₁₋₈alkylaminocarbonylamino-C₁₋₈ alkyl, di-C₁₋₈alkylaminocarbonyl-C₁₋₈alkyl, C₁₋₈alkylamino-C₂₋₈alkoxy, di-C₁₋₈alkylamino-C₂₋₈alkoxy, C₁₋₈alkylamino-C₁₋₈alkyl, di-C₁₋₈alkylamino-C₁₋₈alkyl, C₁₋₈alkylcarbamoyl, di-C₁₋₈alkylcarbamoyl, C₀₋₈alkylcarbonylamino-C₁₋₈alkoxy, C₀₋₈alkylcarbonylamino, C₀₋₈alkylcarbonylamino-C₁₋₈alkyl, C₁₋₈alkylcarbonyloxy-C₁₋₈alkoxy, C₁₋₈alkylcarbonyloxy-C₁₋₈alkyl, C₁₋₈alkylsulfonyl, C₁₋₈alkylsulfonyl-C₁₋₈alkoxy, C₁₋₈alkylsulfonyl-C₁₋₈alkyl, C₁₋₈alkylsulfonylamino-C₁₋₈alkoxy, C₁₋₈alkylsulfonylamino-C₁₋₈alkyl, carbamoyl, carbamoyl-C₁₋₈alkoxy, carbamoyl-C₁₋₈alkyl, carboxy-C₁₋₈alkoxy, carboxy-C₁₋₈alkoxy-C₁₋₈alkyl, carboxy-C₁₋₈alkyl, cyano, cyano-C₁₋₈alkoxy, cyano-C₁₋₈alkyl, C₃₋₈cycloalkyl-C₁₋₈alkoxy, C₃₋₈cycloalkyl-C₁₋₈alkyl, C₃₋₈cycloalkylcarbonylamino-C₁₋₈alkoxy, C₃₋₈cycloalkylcarbonylamino-C₁₋₈alkyl, O,N-dimethylhydroxylamino-C₁₋₈alkyl, halogen, hydroxy-C₁₋₈alkoxy-C₁₋₈alkoxy, hydroxy-C₁₋₈alkoxy-C₁₋₈alkyl, hydroxy-C₁₋₈alkyl, (N-hydroxy)-C₁₋₈alkylaminocarbonyl-C₁₋₈alkoxy, (N-hydroxy)-C₁₋₈alkylaminocarbonyl-C₁₋₈alkyl, (N-hydroxy)aminocarbonyl-C₁₋₈alkoxy, (N-hydroxy)aminocarbonyl-C₁₋₈alkyl, 2-oxooxazolidinyl-C₁₋₈alkoxy, 2-oxooxazolidinyl-C₁₋₈alkyl, O-methyloximyl-C₁₋₈alkyl, polyhalo-C₁₋₈alkoxy or polyhalo-C₁₋₈alkyl; or
(C) R¹ is aryl which is substituted by 3-acetamidomethylpyrrolidinyl, 3-C₁₋₈alkoxy-C₁₋₈alkyl-pyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolyl-alkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]-triazol-1-yl-alkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl or thiomorpholinyl; or
(D) R¹ is aryl when X is -O-CHR⁶-CO-NR⁴-R¹ or -O-CHR⁶-CO-NR⁴-Z, where Z is Alk-R¹ where Alk is C₁₋₈alkylene; or
(E) R¹ is aryl when X is -O-Z, where Z is Alk-NR⁴-R¹ or X is -Z, where Z is -Alk-NR⁴-R¹, where Alk is C₁₋₈alkylene;

R²
   a) is absent when W is cyano; or
   b) is C₁₋₈alkyl, C₂₋₈alkenyl, C₂₋₈alkynyl, C₁₋₈alkoxy-C₁₋₈alkyl, C₁₋₈alkoxy-C₃₋₈cycloalkyl-C₁₋₈-alkyl, C₁₋₈alkylsulfanyl-C₁₋₈alkyl, C₁₋₈alkylsulfonyl-C₁₋₈alkyl when W is -O- or -S-;
R³
   a) is halogen- and/or hydroxy-substituted C₁₋₈alkoxy, halogen- and/or hydroxy-substituted C₁₋₈alkoxy-C₁₋₈alkoxy, branched C₁₋₈alkoxy-C₁₋₈alkoxy, optionally N-mono- or N,N-di-C₁₋₈-alkylated amino-C₁₋₈alkoxy, optionally N-C₁₋₈alkylated C₁₋₈alkoxy-C₁₋₈alkylamino-C₁₋₈alkoxy, optionally N-mono- or N,N-di-C₁₋₈alkylated amino-C₀₋₈-alkylcarbonyl-C₁₋₈alkoxy, hydroxy-C₀₋₈-alkylcarbonyl-C₀₋₈alkoxy, C₁₋₈alkoxy-C₀₋₈alkylcarbonyl-C₀₋₈alkoxy, C₁₋₈-alkylcarbonylamino-C₁₋₈alkoxy, cyano-C₁₋₈alkoxy, substituted C₃₋₈clcloalkyl-C₀₋₈alkoxy, heterocyclyl-C₀₋₈alkoxy, optionally N-C₁₋₈alkylated heterocyclyl-C₀₋₈alkylamino-C₀₋₈alkylcarbonyl-C₀₋₈alkoxy, C₁₋₈alkylsulfonyl-C₁₋₈alkoxy, C₂₋₈alkynyloxy, heterocyclyl-C₂₋₈alkynyloxy, optionally N-mono- or N,N-di-C₁₋₈alkylated amino-C₂₋₈alkynyloxy, N-mono- or N,N-di-C₁₋₈alkylated aminocarbonyl-C₂₋₈alkynyloxy, heterocyclylcarbonyl-C₀₋₈alkoxy, optionally N-mono- or N,N-di-C₁₋₈alkylated amino-C₁₋₈alkyl, optionally N-C₁₋₈alkylated C₁₋₈alkoxy-C₁₋₈alkylamino-C₁₋₈alkyl, optionally N-mono- or N,N-di-C₁₋₈alkylated and optionally hydroxy-substituted amino-C₀₋₈alkyl-carbonyl-C₀₋₈alkyl, optionally N-C₁₋₈alkylated heterocyclyl-C₀₋₈alkylamino-C₀₋₈alkylcarbonyl-C₀₋₈alkyl, optionally halogen- or hydroxy-substituted C₁₋₈alkoxy-C₁₋₈alkyl, optionally halogen- or hydroxy-substituted hydroxy-C₁₋₈alkyl, optionally N-C₁₋₈alkylated hydroxy-C₁₋₈alkylamino-C₁₋₈alkyl, heterocyclylcarbonyl-C₀₋₈alkyl, heterocyclylcarbonyl-C₀₋₈alkylamino-C₁₋₈alkyl, heterocyclyl-C₁₋₈alkyl, C₁₋₈alkoxycarbonylamino-C₁₋₈alkyl, optionally halogen-substituted heterocyclyl-C₀₋₈alkylcarbonylamino-C₁₋₈alkyl, optionally halogen-substituted C₃₋₈cycloalkyl-C₀₋₈alkylcarbonylamino-C₁₋₈alkyl or optionally halogen-substituted C₁₋₈alkylcarbonylamino-C₁₋₈alkyl; or additionally
   b) is hydroxy, unsubstituted C₁₋₈alkoxy, unsubstituted, unbranched C₁₋₈alkoxy-C₁₋₈-alkoxy or unsubstituted C₃₋₈cycloalkyl-C₀₋₈alkoxy if -W-R² is not C₁₋₈alkoxy;
R⁴ is acyl, C₁₋₈alkoxy-C₁₋₈alkyl, C₁₋₈alkyl, aryl-C₁₋₈alkyl, C₃₋₈cycloalkyl-C₀₋₈alkyl, or hydrogen;
R⁵ is C₁₋₈alkoxycarbonyl-C₁₋₈alkyl, C₁₋₈alkyl, carboxy-C₁₋₈alkyl or hydrogen;
R⁶ is acyl, C₂₋₈alkenyl, C₁₋₈alkyl, aryl-C₁₋₈alkyl or hydrogen;
X is Z, -O-Z or -S-Z, where the bond originating from an oxygen or sulfur atom leads to a saturated C atom of the group Z, or is a group -CHR⁶-Z, -CHOR⁴-Z, -O-CO-Z, -O-CO-R¹ -CO-Z, -C=NOR⁵-Z, -O-CHR⁶-Z, -O-CHR⁶-CO-NR⁴-Z, -O-CHR⁶-CO-NR⁴-R¹, or -O-CHR⁶-R¹;
W is -O-, -S- or cyano;
Z is C₁₋₈-Alk-R¹, C₂₋₈alkenylene-R¹, hydroxy-substituted -Alk-R¹, -O-R¹, -S-R¹, -O-Alk-R¹, -S-Alk-R¹, -Alk-O-R¹, -Alk-S-R¹ or -Alk-NR⁴-R¹, where Alk is C₁₋₈alkylene; and where
   (a) X is -CH-R⁶-Z if Z is -O-R¹ or -S-R¹
   (b) X is -CH-R⁶-Z if Z is -O-Alk-R¹ or -S-Alk-R¹; and
   (c) Z is C₂₋₈alkenylene-R¹, -Alk-O-R¹, -Alk-S-R¹ or -Alk-NR⁴-R¹ if X is Z.
C₁₋₈Alkyl and alkoxy radicals may be linear or branched. Examples of C₁₋₈alkyl and alkoxy radicals are methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy. C₁₋₈Alkylenedioxy radicals are preferably methylenedioxy, ethylenedioxy and propylenedioxy. Examples of C₁₋₈alkanoyl radicals are acetyl, propionyl and butyryl. Cycloalkyl is a saturated, cyclic hydrocarbon radical having 3 to 12 carbon atoms, for example cyclopropyl, cyclobutyl, cyclopentyl, cyclohexyl, cycloheptyl, bicyclo[2.2.1]heptyl, cyclooctyl, bicyclo[2.2.2]octyl and adamantyl. Cycloalkyl may be unsubstituted or substituted one or more times, e.g. substituted once or twice by C₁₋₈alkanoyl, C₂₋₈alkenyl, C₂₋₈alkynyl, C₁₋₈alkoxy, C₁₋₈alkoxy-C₁₋₈alkoxy, C₁₋₈alkoxy-C₁₋₈alkyl, C₁₋₈alkoxycarbonylamino, C₁₋₈alkyl, C₀₋₈alkylcarbonylamino, C₁₋₈alkylcarbonyloxy, C₁₋₈alkylenedioxy, optionally N-mono-or N,N-di-C₁₋₈alkylated amino, aryl, optionally N-mono- or N,N-di-C₁₋₈alkylated carbamoyl, optionally esterified carboxy, cyano, C₃₋₈cycloalkoxy, halogen, heteroaryl, heterocyclyl, hydroxy, oxo, polyhalo-C₁₋₈alkoxy or polyhalo-C₁₋₈alkyl. C₁₋₈Alkylene radicals may be linear or branched and are, for example, methylene, ethylene, propylene, 2-methylpropylene, 2-methylbutylene, 2-methylpropyl-2-ene, butyl-2-ene, butyl-3-ene, propyl-2-ene, tetra-, penta- and hexamethylene; C₂₋₈alkenylene radicals are, for example, vinylene and propenylene; C₂₋₈alkynylene radicals is, for example, ethynylene; acyl radicals are alkanoyl radicals, preferably C₁₋₈alkanoyl radicals, or aroyl radicals such as benzoyl. Aryl refers to mono- or polynuclear aromatic radicals which may be substituted one or more times, such as, for example, phenyl, substituted phenyl, naphthyl, substituted naphthyl, tetrahydronaphthyl or substituted tetrahydronaphthyl. Examples of substituents on such aryl radicals are C₁₋₈alkyl, polyhalo-C₁₋₈alkoxy, polyhalo-C₁₋₈alkyl, nitro, amino, C₁₋₈alkenyl, C₁₋₈alkoxy, C₁₋₈alkylcarbonyloxy, hydroxy, halogen, cyano, carbamoyl, carboxy and C₁₋₈alkylenedioxy, and optionally halogen-, C₁₋₈alkyl-, C₁₋₈alkoxy- or dihydroxy-C₁₋₈alkylaminocarbonylsubstituted phenyl, phenoxy, phenylthio, phenyl-C₁₋₈alkyl or phenyl-C₁₋₈alkoxy. Further examples of substituents on aryl or heterocyclyl radicals are C₁₋₈alkoxycarbonylphenyl, hydroxy-C₁₋₈alkylphenyl, benzyloxy, pyridylcarbonylamino-C₁₋₈alkyl, C₂₋₈alkenyloxy, C₁₋₈alkoxy-C₁₋₈alkoxy, C₁₋₈alkoxy-C₁₋₈alkoxy-C₁₋₈alkyl, methoxybenzyloxy, hydroxybenzyloxy, phenethyloxy, methylenedioxybenzyloxy, dioxolanyl-C₁₋₈alkoxy, cyclopropyl-C₁₋₈alkyl, cyclopropyl-C₁₋₈alkoxy, hydroxy-C₁₋₈alkoxy, carbamoyloxy-C₁₋₈alkoxy, pyridylcarbamoyloxy-C₁₋₈alkoxy, benzoyloxy-C₁₋₈alkoxy, C₁₋₈alkoxycarbonyl, C₀₋₈alkylcarbonylamino, C₀₋₈alkylcarbonylamino-C₁₋₈alkyl, C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₀₋₈alkylcarbonylamino-C₁₋₈alkoxy, C₃₋₈-cycloalkylcarbonylamino-C₁₋₈alkyl, C₃₋₈cycloalkylcarbonylamino-C₁₋₈alkoxy, C₁₋₈alkoxy-C₁₋₈alkyl, hydroxy-C₁₋₈alkyl, hydroxy-C₁₋₈alkoxy-C₁₋₈alkyl, hydroxy-C₁₋₈alkoxy-C₁₋₈alkoxy, C₁₋₈alkoxycarbonylamino-C₁₋₈alkyl, C₁₋₈alkoxycarbonylamino-C₁₋₈alkoxy, C₁₋₈alkylaminocarbonylamino-C₁₋₈alkyl, C₁₋₈alkylaminocarbonylamino-C₁₋₈alkoxy, C₁₋₈alkylaminocarbonyl-C₁₋₈alkyl, C₁₋₈alkylaminocarbonyl-C₁₋₈alkoxy, C₁₋₈alkylaminocarbonyl-C₁₋₈alkoxy-C₁₋₈alkyl, di-C₁₋₈alkylaminocarbonyl-C₁₋₈alkyl, di-C₁₋₈alkylaminocarbonyl-C₁₋₈alkoxy, C₁₋₈alkylcarbonyloxy-C₁₋₈alkyl, C₁₋₈alkylcarbonyloxy-C₁₋₈alkoxy, cyano-C₁₋₈alkyl, cyano-C₁₋₈alkoxy, 2-oxooxazolidinyl-C₁₋₈alkyl, 2-oxooxazolidinyl-C₁₋₈alkoxy, C₁₋₈alkoxycarbonyl-C₁₋₈alkyl, C₁₋₈alkoxycarbonyl-C₁₋₈alkoxy, C₁₋₈alkylsulfonylamino-C₁₋₈alkyl, C₁₋₈alkylsulfonylamino-C₁₋₈alkoxy, (N-C₁₋₈alkyl)-C₁₋₈alkylsulfonylamino-C₁₋₈alkyl, (N-C₁₋₈alkyl)-C₁₋₈alkylsulfonylamino-C₁₋₈alkoxy, C₁₋₈alkylamino-C₁₋₈alkyl, C₁₋₈alkylamino-C₂₋₈alkoxy, di-C₁₋₈alkylamino-C₁₋₈alkyl, di-C₂₋₈alkylamino-C₂₋₈alkoxy, C₁₋₈alkylsulfonyl-C₁₋₈alkyl, C₁₋₈alkylsulfonyl-C₁₋₈alkoxy, carboxy-C₁₋₈alkyl, carboxy-C₁₋₈alkoxy, carboxy-C₁₋₈alkoxy-C₁₋₈alkyl, C₁₋₈alkoxy-C₁₋₈alkylcarbonyl, acyl-C₁₋₈alkoxy-C₁₋₈alkyl, (N-C₁₋₈alkyl)-C₁₋₈alkoxycarbonylamino, (N-hydroxy)-C₁₋₈alkylaminocarbonyl-C₁₋₈alkyl, (N-hydroxy)-C₁₋₈alkylaminocarbonyl-C₁₋₈alkoxy, (N-hydroxy)aminocarbonyl-C₁₋₈alkyl, (N-hydroxy)aminocarbonyl-C₁₋₈alkoxy, C₁₋₈alkoxyaminocarbonyl-C₁₋₈alkyl, 6-alkoxyaminocarbonyl-C₁₋₈alkoxy, (N-C₁₋₈alkoxy)-C₁₋₈alkylaminocarbonyl-C₁₋₈alkyl, (N-C₁₋₈alkoxy)-C₁₋₈alkylaminocarbonyl-C₁₋₈alkoxy, (N-acyl)-C₁₋₈alkoxy-C₁₋₈alkylamino, C₁₋₈alkoxy-C₁₋₈alkylcarbamoyl, (N-C₁₋₈alkyl)-C₁₋₈alkoxy-C₁₋₈alkylcarbamoyl, C₁₋₈alkoxy-C₁₋₈alkylcarbonyl, C₁₋₈alkoxy-C₁₋₈alkylcarbonylamino, (N-C₁₋₈alkyl)-C₁₋₈alkoxy-C₁₋₈alkylcarbonylamino, 1-C₁₋₈alkoxy-C₁₋₈alkylimidazol-2-yl, 1-C₁₋₈alkoxy-C₁₋₈alkyltetrazol-5-yl, 5-C₁₋₈alkoxy-C₁₋₈alkyltetrazol-1-yl, 2-C₁₋₈alkoxy-C₁₋₈alkyl-4-oxoimidazol-1-yl, carbamoyl-C₁₋₈alkyl, carbamoyl-C₁₋₈alkoxy, C₁₋₈alkylcarbamoyl, di-C₁₋₈alkylcarbamoyl, C₁₋₈alkylsulfonyl, C₁₋₈alkylamidinyl, acetamidinyl-C₁₋₈alkyl, O-methyloximyl-C₁₋₈alkyl, O,N-dimethylhydroxylamino-C₁₋₈alkyl, C₃₋₈cycloalkyl-C₁₋₈alkanoyl, aryl-C₁₋₈alkanoyl, heterocyclyl-C₁₋₈alkanoyl; and optionally halogen-, C₁₋₈alkyl-, C₁₋₈alkoxy- or dihydroxy-C₁₋₈alkylaminocarbonyl-substituted pyridyl, pyridyloxy, pyridylthio, pyridylamino, pyridyl-C₁₋₈alkyl, pyridyl-C₁₋₈alkoxy, pyrimidinyl, pyrimidinyloxy, pyrimidinylthio, pyrimidinylamino, pyrimidinyl-C₁₋₈alkyl, pyrimidinyl-C₁₋₈alkoxy, thienyl, thienyl-C₁₋₈alkyl, thienyl-C₁₋₈alkoxy, furyl, furyl-C₁₋₈alkyl, furyl-C₁₋₈alkoxy.

The term heterocyclyl refers to mono-, bi- or polycyclic, saturated and unsaturated heterocyclic radicals having 1 to 4 nitrogen and/or 1 or 2 sulfur or oxygen atoms, which may be substituted one or more times, in particular once, twice or three times. The term heterocyclyl further encompasses the above oxo-substituted radicals. Heterocyclyl radicals which comprise a nitrogen atom may be linked either via the N atom or via a C atom to the remainder of the molecule.

Examples of unsaturated heterocyclyl radicals are benzo[1,3]dioxolyl, benzofuranyl, benzoimidazolyl, benzooxazolyl, benzothiazolyl, benzo[b]thienyl, quinazolinyl, quinolyl, quinoxalinyl, chromenyl, dihydrobenzofuranyl, 1,3-dihydrobenzoimidazolyl, 3,4-dihydro-2H-benzo[1,4]oxazinyl, dihydro-3H-benzo[1,4]oxazinyl, 1,4-dihydrobenzo[d][1,3]oxazinyl, dihydro-2H-benzo[1,4]thiazinyl, 3,4-dihydro-1H-quinazolinyl, 3,4-dihydro-1H-quinolinyl, 2,3-dihydroindolyl, dihydro-1H-pyrido[2,3-b][1,4]oxazinyl, 1,1-dioxodihydro-2H-benzo[1,4]thiazinyl, furyl, imidazolyl, imidazo[1,5-a]pyridinyl, imidazo[1,2-a]pyrimidinyl, indazolyl, indolyl, isobenzofuranyl, isoquinolyl, [1,5]naphthyridyl, oxazolyl, 1-oxidopyridyl, 2-oxobenzoimidazolyl, 3-oxo-4H-benzo[1,4]oxazinyl, 2-oxobenzooxazolyl, 3-oxo-4H-benzo[1,4]thiazinyl, 2-oxo-1H-quinolinyl, 2-oxo-chromenyl, 2-oxodihydrobenzo[e][1,4]diazepinyl, 2-oxo-1,3-dihydrobenzoimidazole, 2-oxodihydrobenzo[d][1,3]oxazinyl, 2-oxo-3,4-dihydro-1H-quinazolinyl, 2-oxo-3,4-dihydro-1H-quinolinyl, 4-oxo-dihydroimidazolyl, 2-oxo-1,3-dihydroindolyl, 1-oxo-3H-isobenzofuranyl, 2-oxo-1H-pyrido[2,3-b][1,4]oxazinyl, 2-oxo-1,3,4,5-tetrahydrobenzo[b]azepinyl, 2-oxotetrahydrobenzo[e][1,4]diazepinyl, 4-oxo-3H-thieno[2,3-d]pyrimidinyl, 5-oxo-4H-[1,2,4]triazinyl, C₁₋₈alkylenedioxy-substituted phenyl, phthalazinyl, pyranyl, pyrazinyl, pyrazolyl, pyridyl, pyrimidinyl, 1H-pyrrolizinyl, pyrrolo[3,2-c]pyridinyl, pyrrolo[2,3-c]pyridinyl, pyrrolo[3,2-b]pyridinyl, 1H-pyrrolo[2,3-b]pyridyl, pyrrolyl, 1,3,4,5-tetrahydrobenzo[b]azepinyl, tetrahydroquinolinyl, tetrahydroquinoxalinyl, tetrahydroisoquinolinyl, thiazolyl, thienyl, triazinyl, triazolyl, 1,1,3-trioxodihydro-2H-1λ6-benzo[1,4]thiazinyl, [1,2,3]triazolo[1,5-a]pyridinyl or [1,2,4]triazolo[4,3-a]pyridinyl.

The term saturated heterocyclyl refers to 3-16-membered, mono-, bi- or polycyclic saturated heterocyclic radicals having 1 to 4 nitrogen and/or 1 or 2 sulfur or oxygen atoms. Preference is given to 3-8-membered, particularly preferably 5- or 6-membered, monocyclic radicals which optionally have a 3-8-membered fused-on ring which may be carbocyclic or heterocyclic. A further preferred group of heterocyclic radicals are bi- or polycyclic heterocycles which optionally have a spirocyclic or bridged ring. Preferred heterocyclic radicals have in each ring 1 nitrogen, oxygen or sulfur atom, 1-2 nitrogen atoms and 1-2 oxygen atoms or 1-2 nitrogen atoms and 1-2 sulfur atoms, with at least one, preferably 1-7, carbon atoms being present in each ring.

Examples of saturated heterocyclyl radicals are azepanyl, azetidinyl, aziridinyl, 3,4-dihydroxypyrrolidinyl, 2,6-dimethylmorpholinyl, 3,5-dimethylmorpholinyl, dioxanyl, [1,4]dioxepanyl, dioxolanyl, 4,4-di-oxothiomorpholinyl, dithianyl, dithiolanyl, 2-hydroxymethylpyrrolidinyl, 4-hydroxypiperidinyl, 3-hydroxypyrrolidinyl, 4-methylpiperazinyl, 1-methylpiperidinyl, 1-methylpyrrolidinyl, morpholinyl, oxathianyl, oxepanyl, 2-oxoazepanyl, 2-oxoimidazolidinyl, 2-oxooxazolidinyl, 2-oxopiperidinyl, 4-oxopiperidinyl, 2-oxopyrrolidinyl, 2-oxotetrahydropyrimidinyl, 4-oxothiomorpholinyl, piperazinyl, piperidinyl, pyrrolidinyl, tetrahydrofuranyl, tetrahydropyranyl, tetrahydrothiophenyl, tetrahydrothiopyranyl, thiepanyl or thiomorpholinyl.

Examples of bi- or polycyclic heterocyclyl radicals are 2,5-dioxabicyclo[4.1.0]heptanyl, 2-oxa-bicyclo[2.2.1]heptanyl, 2-oxabicyclo[4.1.0]heptanyl, 3-oxabicyclo[4.1.0]heptanyl, 7-oxa-bicyclo[2.2.1]heptanyl, 2-oxabicyclo[3.1.0]hexanyl, 3-oxabicyclo[3.1.0]hexanyl, 1-oxa-spiro[2.5]octanyl, 6-oxaspiro[2.5]octanyl, 3-oxabicyclo[3.3.1]nonanyl, 2-oxo-1a,7b-dihydro-1H-cyclopropa[c]chromenyl or 1,1a,2,7b-tetrahydrocyclopropa[c]chromenyl.

Heterocyclyl may be unsubstituted or substituted one or more times, e.g. once or twice, by C₁₋₈alkanoyl, C₂₋₈alkenyl, C₂₋₈alkynyl, C₁₋₈alkoxy, C₁₋₈alkoxy-C₁₋₈alkoxy, C₁₋₈alkoxy-C₁₋₈alkyl, C₁₋₈alkoxycarbonylamino, C₁₋₈alkyl, C₀₋₈alkylcarbonylamino, C₁₋₈alkylcarbonyloxy, C₁₋₈alkylenedioxy, optionally N-mono- or N,N-di-C₁₋₈alkylated amino, aryl, optionally N-mono- or N,N-di-C₁₋₈alkylated carbamoyl, optionally esterified carboxy, cyano, C₃₋₈cycloalkoxy, halogen, heteroaryl, heterocyclyl, hydroxy, nitro, oxide, oxo, polyhalo-C₁₋₈alkoxy or polyhalo-C₁₋₈alkyl.

The aryl and heterocyclyl radicals in the case of R¹ may additionally be substituted also by heterocyclylalkyl, heterocyclylalkoxy, heterocyclylalkoxyalkyl or heterocyclyl such as, for example, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]-triazol-1-ylalkyl, [1,2,4]-triazol-1-ylalkoxy, [1,2,4]-triazol-4-yl-alkyl, [1,2,4]-triazol-4-ylalkoxy, [1,2,4]-oxadiazol-5-ylalkyl, [1,2,4]-oxadiazol-5-ylalkoxy, 3-methyl-[1,2,4]-oxadiazol-5-ylalkyl, 3-methyl-[1,2,4]-oxadiazol-5-ylalkoxy, 5-methyl-[1,2,4]-oxadiazol-3-ylalkyl, 5-methyl-[1,2,4]-oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyl-tetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy N-methylpiperazinoalkyl, N-methyl-piperazinoalkoxy, N-methylpiperazinoalkoxyalkyl, and also alkylaminoalkyl, alkylaminoalkoxy, alkylaminoalkoxyalkyl, mono- and polyhydroxyalkyl, mono- and polyhydroxyalkoxy, mono- and polyhydroxyalkoxyalkyl, mono- and polyhydroxyalkoxyalkoxy, carbamoylalkyloxy, C₁₋₈alkoxy, amino-C₁₋₈alkoxy, hydroxy-C₁₋₈alkoxy, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₈alkoxy-C₁₋₈alkylpyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl and the like or by the radical -O-CH₂CH(OH)CH₂NRₓ, where NRₓ is a mono- or di-C₁₋₈alkylamino, piperidino, morpholino, piperazino or N-methylpiperazino radical.

Halogen- and/or hydroxy-substituted C₁₋₈alkoxy may be for example hydroxy-C₁₋₈alkoxy or else polyhydroxy-C₁₋₈alkoxy.

The term polyhydroxyalkyl refers to C₁₋₈alkyl radicals which may be substituted by 2-8 hydroxy groups, such as, for example, glyceryl, arabityl, sorbityl etc. An analogous statement applies to radicals derived therefrom, such as polyhydroxy-C₁₋₈alkoxy.

The compounds of the formula (I) have at least three asymmetric carbon atoms and may therefore exist in the form of optically pure diastereomers, mixtures of diastereomers, diastereomeric racemates, mixtures of diastereomeric racemates or as meso compounds. The invention encompasses all these forms.

Mixtures of diastereomers, diastereomeric racemates or mixtures of diastereomeric racemates can be fractionated by conventional methods, e.g. by column chromatography, thin-layer chromatography, HPLC and the like.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of the formula (I).

Such salts are formed for example by compounds of the formula (I) having an acidic group, e.g. a carboxy or sulfo group, and are for example their salts with suitable bases, such as non-toxic metal salts derived from metals of group Ia, Ib, IIa and IIb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as optionally hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N,N-di-lower-alkyl-N-(hydroxy-lower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formula I having a basic group, e.g. an amino group, can form acid addition salts, e.g. with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulfonic acid, ethanesulfonic acid, 2-hydroxyethanesulfonic acid, ethane-1,2-disulfonic acid, benzenesulfonic acid, 4-toluenesulfonic acid, naphthalene-2-sulfonic acid, 2- or 3-phosphoglycerate, glucose 6-phosphate, N-cyclohexylsulfamic acid (to form cyclamates) or with other acidic organic compounds such as ascorbic acid. Compounds of the formula (I) having acidic and basic groups may also form inner salts.

Pharmaceutically unsuitable salts may also be used for isolation and purification.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate. The definitions mentioned apply within the scope of general chemical principles such as, for example, the usual valencies of atoms.

The compounds of the formula (I) also include those compounds in which one or more atoms are replaced by their stable, non-radioactive isotopes; for example, a hydrogen atom by deuterium.

Preferred compounds according to the invention are those of the general formula (IA) and the pharmaceutically acceptable salts thereof 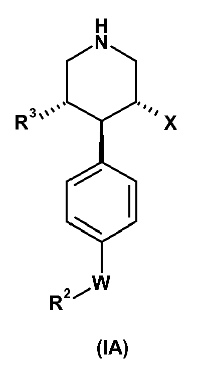 in which R², R³, W and X have the meaning indicated above for the compounds of the formula (I).

A further preferred group of compounds of the formula (I), and particularly preferably of the formula (IA), and the pharmaceutically acceptable salts thereof, are compounds in which
R¹ is heterocyclyl substituted by oxo or oxide or as indicated under (B) or (C), where heterocyclyl is particularly preferably selected from azepanyl, benzo[1,3]dioxolyl, benzofuranyl, benzoimidazolyl, 4H-benzo[1,4]oxazinyl, benzooxazolyl, 4H-benzo[1,4]thiazinyl, quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, 3,4-dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c] chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1 H-pyrido[2,3-b][1,4]oxazinyl, pyridyl, 1H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydro-cyclo-propa[c]chromenyl, tetrahydropyranyl or triazinyl.

Particularly preferred radicals R¹ are azepanyl, benzo[1,3]dioxolyl, benzofuranyl, benzoimidazolyl, 4H-benzo[1,4]oxazinyl, benzooxazolyl, 4H-benzo[1,4]thiazinyl, quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo-[d][1,3]oxazinyl, 3,4-dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1 H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydro-cyclopropa[c]chromenyl, tetrahydropyranyl or triazinyl substituted by 1-3 C₁₋₈alkanoyl, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkylcarbonyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, C₀₋₈alkylcarbonylamino-C₁₋₈alkoxy, C₀₋₈alkylcarbonylamino-C₁₋₈alkyl, halogen, oxide, oxo, polyhalo-C₁₋₈alkoxy, polyhalo-C₁₋₈alkyl, [1,2,4]-triazol-1-ylalkyl, [1,2,4]-triazol-4-ylalkyl, tetrazol-1-ylalkyl, tetrazol-2-ylalkyl, tetrazol-5-ylalkyl or thiazol-4-ylalkyl.

R¹ is very particularly preferably chromenyl, 3,4-dihydro-2H-benzo[1,4]oxazinyl, 3,4-dihydro-2H-benzo[1,4]thiazinyl or 1,3-dihydroindolyl substituted by 1-3 C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, C₁₋₈alkoxy-C₁₋₈alkylcarbonyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, C₀₋₈alkylcarbonylamino-C₁₋₈alkoxy, C₀₋₈alkylcarbonylamino-C₁₋₈alkyl, halogen, oxo, polyhalo-C₁₋₈alkoxy or polyhalo-C₁₋₈alkyl.

A further preferred group of compounds of the formula (I), or particularly preferably of the formula (IA), and the pharmaceutically acceptable salts thereof, are compounds in which
R¹ has the meaning as indicated for (A) or (B), particularly preferably as indicated for (A);
R² has the meaning as indicated for (a) or (b);
R³ has the meaning as indicated for (a) or (b);
R⁴ is C₁₋₈alkyl or hydrogen;
R⁵ is C₁₋₈alkyl or hydrogen;
R⁶ is C₁₋₈alkyl or hydrogen;
X is -CHR⁶-Alk-R¹, -Alk-NR⁴-R¹, -Alk-O-R¹, -Alk-S-R¹, C₂₋₈-Alkenylen-R¹, -CH(OR⁴)-Alk-R¹, -CHR⁶-Alk-R¹, -CHR⁶-O-R¹, -CHR⁶-O-Alk-R¹, -CHR⁶-S-R¹, -CHR⁶-S-Alk-R¹, -CO-Alk-R¹, -C(=NOR⁵)-Alk-R¹, -O-Alk-NR⁴-R¹, -O-Alk-R¹, -O-Alk-O-R¹, -O-CO-R¹, -O-CO-Alk-R¹, -O-CHR⁶-R¹, -O-CHR⁶-Alk-R¹, -O-CHR⁶-CO-NR⁴-R¹ or -O-CHR⁶-CO-NR⁴-Alk-R¹, where Alk is C₁₋₈alkylene; and
W is -O-, -S- or cyano.

A further preferred group of compounds of the formula (I), or particularly preferably of the formula (IA), and the pharmaceutically acceptable salts thereof, are compounds in which
R¹ has the meaning as indicated for (A) or (B), particularly preferably as indicated for (A);
R² has the meaning as indicated for (a) or (b);
R³ has the meaning as indicated for (a) or (b);
R⁴ is C₁₋₈alkyl or hydrogen;
R⁵ is C₁₋₈alkyl or hydrogen;
R⁶ is C₁₋₈alkyl or hydrogen;
X is -CHR⁶-Alk-R¹, -CH(OR⁴)-Alk-R¹, -O-Alk-R¹, -O-CHR⁶-R¹ or -O-CHR⁶-CO-NR⁴-R¹, where Alk is C₁₋₈alkylene; and
W is -O-, -S- or cyano.

Preference is furthermore given to compounds of the formulae (I) and (IA) and the pharmaceutically acceptable salts thereof, in which X is preferably -CHR⁶-Alk-R¹, -CH(OR⁴)-Alk-R¹, -O-Alk-R¹, -O-CHR⁶-R¹ or -O-CHR⁶-CO-NR⁴-R¹.

Preference is furthermore given to compounds of the formulae (I) and (IA) and the pharmaceutically acceptable salts thereof, in which
R³
   a) is halogen- and/or hydroxy-substituted C₁₋₈alkoxy, halogen- and/or hydroxy-substituted C₁₋₈alkoxy-C₁₋₈alkoxy, branched C₁₋₈alkoxy-C₁₋₈alkoxy, optionally N-mono- or N,N-di-C₁₋₈alkylated amino-C₁₋₈alkoxy, optionally N-mono- or N,N-di-C₁₋₈alkylated amino-C₀₋₈alkylcarbonyl-C₁₋₈alkoxy, substituted C₃₋₈cycloalkyl-C₀₋₈alkoxy, optionally C₁₋₈alkoxy or hydroxy-substituted heterocyclyl-C₀₋₈alkoxy, heterocyclylcarbonyl-C₀₋₈alkoxy, heterocyclylcarbonyl-C₀₋₈alkyl, optionally halogen-substituted heterocyclyl-C₀₋₈alkyl-carbonylamino-C₁₋₈alkyl, optionally halogen-substituted C₃₋₈cycloalkyl-C₀₋₈alkylcarbonyl-amino-C₁₋₈alkyl or optionally halogen-substituted C₁₋₈alkylcarbonylamino-C₁₋₈alkyl; or additionally
   b) hydroxy, unsubstituted C₁₋₈alkoxy, unsubstituted, unbranched C₁₋₈alkoxy-C₁₋₈alkoxy or unsubstituted C₃₋₈cycloalkyl-C₀₋₈alkoxy if -W-R² is not C₁₋₈alkoxy.
R³ is very particularly preferably
   a) hydroxy-substituted C₁₋₈alkoxy, optionally hydroxy-substituted C₁₋₈alkoxy-C₁₋₈alkoxy, branched C₁₋₈alkoxy-C₁₋₈alkoxy or C₁₋₈alkylcarbonylamino-C₁₋₈alkyl; or additionally
   b) hydroxy, unsubstituted C₁₋₈alkoxy or unsubstituted, unbranched C₁₋₈alkoxy-C₁₋₈alkoxy if -W-R² is not C₁₋₈alkoxy.

Preference is furthermore given to compounds of the formulae (I) and (IA) and the pharmaceutically acceptable salts thereof, in which
R² is C₁₋₈alkyl, C₂₋₈alkenyl, C₁₋₈alkoxy-C₁₋₈alkyl, C₁₋₈alkoxy-C₃₋₈cycloalkyl-C₁₋₈alkyl, C₃₋₈-cycloalkyl-C₀₋₈alkoxy-C₁₋₈alkyl-, C₁₋₈alkylsulfanyl-C₁₋₈alkyl, C₁₋₈alkylsulfonyl-C₁₋₈alkyl;
R³ is hydroxy-substituted C₁₋₈alkoxy, optionally hydroxy-substituted C₁₋₈alkoxy-C₁₋₈alkoxy, hydroxy or C₁₋₈alkylcarbonylamino-C₁₋₈alkyl; and
W is -S-.

Preference is furthermore given to compounds of the formulae (I) and (IA) and the pharmaceutically acceptable salts thereof, in which
R² is C₁₋₈alkyl, C₂₋₈alkenyl, C₁₋₈alkoxy-C₁₋₈alkyl, C₁₋₈alkoxy-C₃₋₈cycloalkyl-C₁₋₈alkyl, C₃₋₈cycloalkyl-C₀₋₈alkoxy-C₁₋₈alkyl-, C₁₋₈alkylsulfanyl-C₁₋₈alkyl, C₁₋₈alkylsulfonyl-C₁₋₈alkyl;
R³
   a) hydroxy-substituted C₁₋₈alkoxy, hydroxy-substituted C₁₋₈alkoxy-C₁₋₈alkoxy, branched C₁₋₈alkoxy-C₁₋₈alkoxy or C₁₋₈alkylcarbonylamino-C₁₋₈alkyl; or additionally
   b) hydroxy, unsubstituted C₁₋₈alkoxy or unsubstituted, unbranched C₁₋₈alkoxy-C₁₋₈alkoxy if R² is not C₁₋₈alkyl;
   and
W is -O-.

Particularly preferred radicals R² are C₁₋₈alkyl, C₂₋₈alkenyl, C₁₋₈alkoxy-C₁₋₈alkyl, C₃₋₈cycloalkyl-C₀₋₈alkoxy-C₁₋₈alkyl, C₁₋₈alkylsulfanyl-C₁₋₈alkyl or C₁₋₈alkylsulfonyl-C₁₋₈alkyl when W is -O- or -S-.

Very particular preference is given to compounds and the pharmaceutically acceptable salts thereof, of the formulae (I) and (IA) in which
R¹ is substituted chromenyl or 3,4-dihydro-2H-benzo[1,4]oxazinyl;
R² is C₁₋₈alkyl, C₂₋₈alkenyl, C₁₋₈alkoxy-C₁₋₈alkyl, C₁₋₈alkoxy-C₃₋₈cycloalkyl-C₁₋₈alkyl, C₃₋₈cycloalkyl-C₀₋₈alkoxy-C₁₋₈alkyl, C₁₋₈alkylsulfanyl-C₁₋₈alkyl;
R³
   a) hydroxy-substituted C₁₋₈alkoxy, hydroxy-substituted C₁₋₈alkoxy-C₁₋₈alkoxy, branched C₁₋₈alkoxy-C₁₋₈alkoxy or C₁₋₈alkylcarbonylamino-C₁₋₈alkyl; or additionally
   b) hydroxy, unsubstituted C₁₋₈alkoxy or unsubstituted, unbranched C₁₋₈alkoxy-C₁₋₈ alkoxy if R² is not C₁₋₈alkyl;
R⁶ is C₁₋₈alkyl or hydrogen;
X is -CHR⁶-Alk-R¹ or -O-Alk-R¹, where Alk is C₁₋₈alkylene; and
W is -O-.

The groups of compounds mentioned above are not to be regarded as closed; on the contrary, it is possible for parts of these groups of compounds to be interchanged or replaced by the definitions or preferences given, or omitted, in a worthwhile manner, e.g. to replace general by more specific definitions. The definitions and preferences mentioned apply within the scope of general chemical principles such as, for example, the usual valencies of atoms.

The compounds of the formula (I) can be prepared in a manner analogous to preparation processes disclosed in the literature. Similar preparation processes are described for example in WO 97/09311. Details of the specific preparation variants can be found in the examples.

The compounds of the formula (I) can also be prepared in optically pure form. Separation into antipodes can take place by methods known per se, either preferably at an early stage in the synthesis by salt formation with an optically active acid such as, for example, (+)- or (-)-mandelic acid and separation of the diastereomeric salts by fractional crystallization or preferably at a rather late stage by derivatizing with a chiral auxiliary component such as, for example, (+)- or (-)-camphanoyl chloride, and separation of the diastereomeric products by chromatography and/or crystallization and subsequent cleavage of the linkage to the chiral auxiliary. The pure diastereomeric salts and derivatives can be analysed to determine the absolute configuration of the contained piperidine by conventional spectroscopic methods, with X-ray spectroscopy on single crystals representing a particularly suitable method.

The compounds of formula (I) and (IA), respectively, and their pharmaceutically acceptable salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 µl buffer, 10 µl inhibitor in DMSO, 10 µl peptide substrate in DMSO and 20 µl enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher (SEQ ID NO: 1) is obtained from Invitrogen, Carlsbad, CA, USA. The cathepsin D peptide substrate of the sequence DABCYL-Pro-Thr-Glu-Phe-Phe-Arg-Leu-OXL (SEQ ID NO: 2), the plasmepsin peptide substrate of the sequence DABCYL-Glu-Arg-Nle-Phe-Leu-Ser-Phe-Pro-OXL (SEQ ID NO: 3) and the HIV protease peptide substrate of the sequence DABCYL-His-Lys-Ala-Arg-Val-Leu-Tyr-Glu-Ala-Nle-Ser-EDANS (SEQ ID NO: 4) are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

### In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) or preferred formula (IA) and the pharmaceutically acceptable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) and pharmaceutically acceptable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (IA), respectively, and their pharmaceutically acceptable salts for the prevention, delay of progression or the treatment of Alzheimer Disease, malaria or HIV infection. Subject of the present invention is also the use of the compounds of formula (I) and (IA), respectively, and their pharmaceutically acceptable salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formula (IA) or a pharmaceutically acceptable salt thereof is applied.

Subject of the present invention is also a pharmaceutical preparation that contains for the inhibition of beta-secretase, cathepsin D, plasmepsin and/or HIV-protease a compound of the general formula (I), or preferred of formula (IA) or a pharmaceutically acceptable salt thereof as well as commonly used ingredients.

Subject of the present invention is also a pharmaceutical preparation for the prevention, delay of progression or treatment of Alzheimer Disease, malaria and HIV infection that contains a compound of the general formula (I), or preferred of formula (IA) or a pharmaceutically acceptable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The following examples illustrate the present invention. All temperatures are stated in degrees Celsius and pressures in mbar. Unless mentioned otherwise, the reactions take place at room temperature. The abbreviation "Rf = xx (A)" means for example that the Rf xx was found in solvent system A. The ratio of amounts of solvents to one another is always indicated in proportions by volume. Chemical names for final products and intermediates were generated with the aid of the AutoNom 2000 (Automatic Nomenclature) program.

| **No.** | **Structure** | **Appearance** | **R_{f} (System)** | **Rt (Method)** |
|---|---|---|---|---|
| 1 | 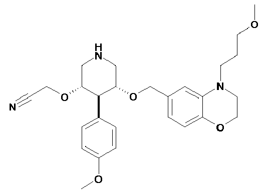 | yellowish resin | 0.31 (C) | 3.62 (I) |
| 2 | 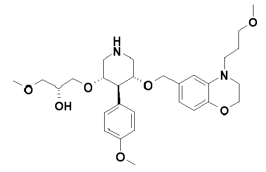 | yellowish oil | 0.17 (A) | 3.33 (I) |
| 3 | 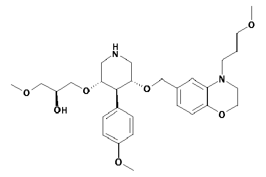 | yellowish oil | 0.17 (A) | 3.30 (I) |
| 5 | 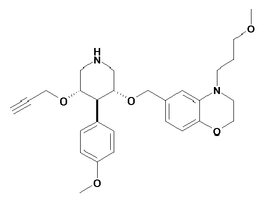 | yellowish resin | 0.21 (C) | 3.72 (I) |
| 6 | 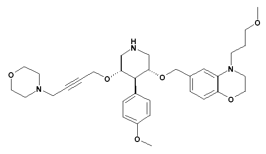 | yellowish resin | 0.28 (C) | 3.15 (I) |
| 7 | 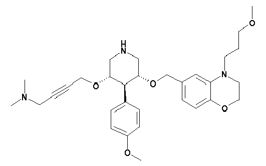 | violet oil | 0.09 (J) | 3.13 (I) |
| 8 | 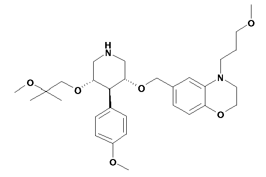 | yellowish oil | 0.1 (C) | 3.79 (I) |
| 9 | 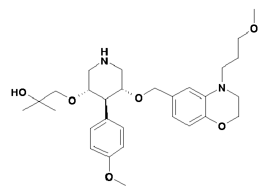 | yellowish oil | 0.22 (C) | 3.51 (I) |
| 10 | 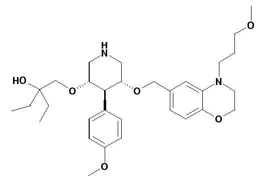 | yellow oil | 0.15 (C) | 3.85 (I) |
| 11 | 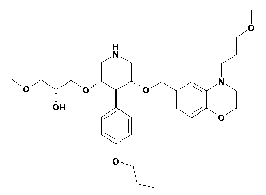 | colourless oil | 0.12 (C) | 3.83 (I) |
| 12 | 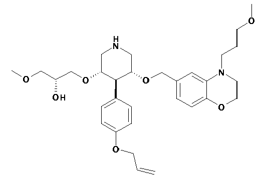 | yellowish oil | 0.31 (A) | 3.66 (I) |
| 13 | 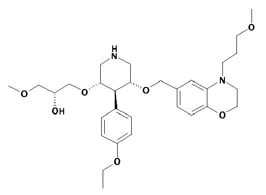 | pale yellow cloudy resin | 0.194 (A) | 3.55 (I) |
| 14 | 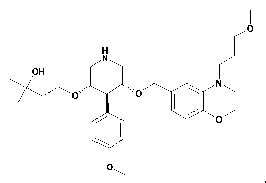 | yellow oil | 0.13 (C) | 3.51 (I) |
| 16 | 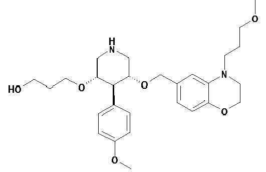 | yellowish oil | 0.08 (C) | 3.29 (I) |
| 17 | 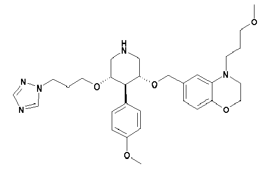 | cloudy oil | 0.08 (J) | 3.25 (I) |
| 18 | 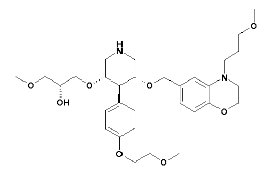 | yellowish oil | 0.33 (A) | 3.31 (I) |
| 19 | 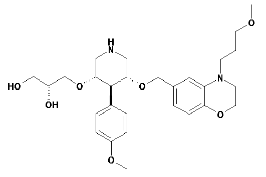 | colourless wax | 0.22 (A) | 3.11 (I) |
| 20 | 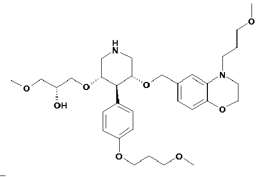 | yellow resin | 0.25 (A) | 3.50 (I) |
| 21 | 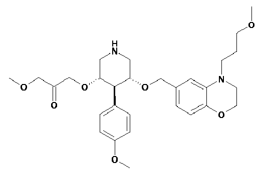 | dark yellow oil | 0.22 (A) | 3.49 (I) |
| 22 | 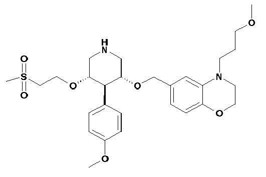 | yellowish resin | 0.08 (C) | 3.40 (I) |
| 23 | 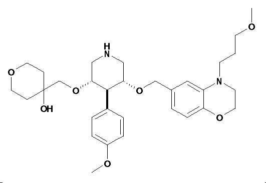 | yellowish resin | 0.19 (A) | 3.32 (I) |
| 24 | 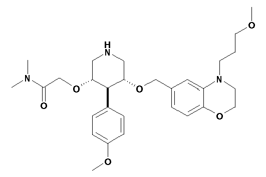 | yellowish, cloudy oil | 0.1 (C) | 3.41 (I) |
| 25 | 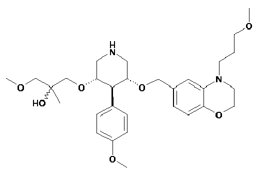 | yellowish oil | 0.27 (A) | 3.52 (I) |
| 26 | 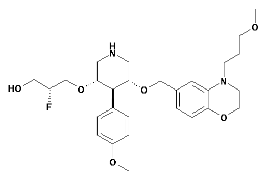 | yellow wax | 0.25 (A) | 3.36 (I) |
| 27 | 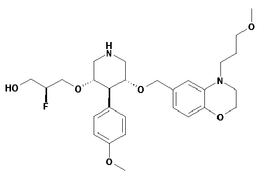 | yellow wax | 0.26 (A) | 3.35 (I) |
| 28 | 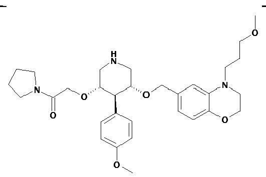 | yellow oil | 0.61 (H) | 3.56 (I) |
| 29 | 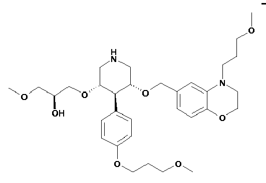 | black oil | 0.25 (A) | 3.47 (I) |
| 30 | 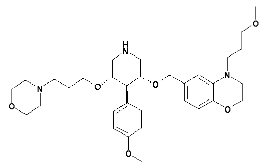 | yellowish resin | 0.37 (A) | 3.05 (I) |
| 31 | 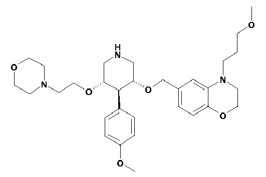 | yellowish resin | 0.37 (A) | 3.04 (I) |
| 32 | 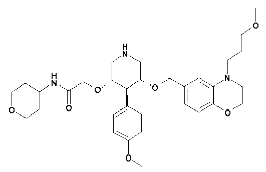 | yellow resin | 0.31 (C) | 3.38 (I) |
| 33 | 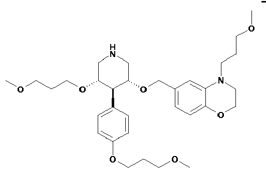 | yellowish liquid | 0.48 (A) | 3.80 (I) |
| 34 | 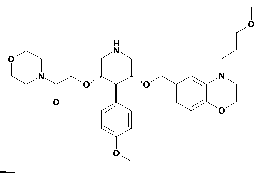 | colourless oil | 0.25 (A) | 3.38 (I) |
| 35 | 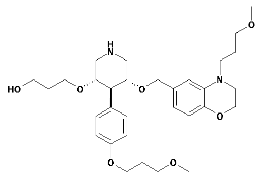 | yellow oil | 0.26 (A) | 3.43 (I) |
| 36 | 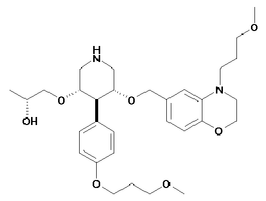 | colourless oil | 0.26 (A) | 3.51 (I) |
| 37 | 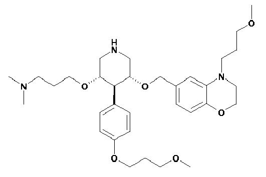 | pale yellowish oil | 0.05 (C) | 3.13 (II) |
| 38 | 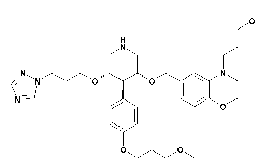 | pale yellowish oil | 0.28 (A) | 3.38 (I) |
| 39 | 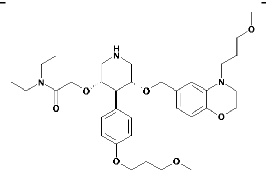 | colourless oil | 0.24 (A) | 3.82 (I) |
| 40 | 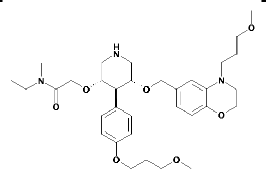 | colourless oil | 0.21 (A) | 3.69 (I) |
| 41 | 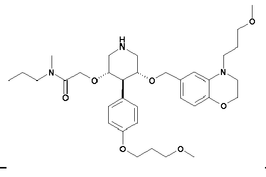 | colourless oil | 0.24 (A) | 3.85 (I) |
| 42 | 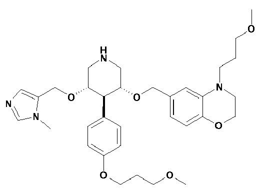 | colourless oil | 0.21 (A) | 3.11 (I) |
| 43 | 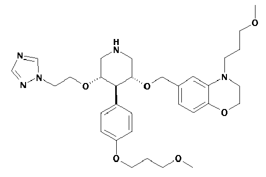 | pale yellow resin | 0.27 (A) | 3.30 (I) |
| 44 | 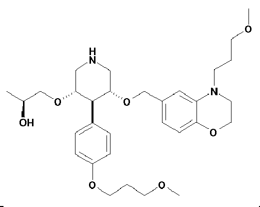 | colourless oil | 0.24 (A) | 3.52 (I) |
| 45 | 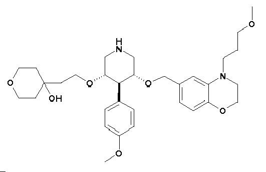 | yellowish oil | 0.25 (A) | 3.32 (I) |
| 46 | 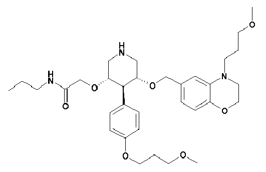 | yellowish resin | 0.44 (G) | 3.79 (II) |
| 47 | 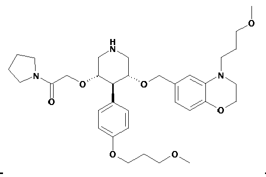 | colourless oil | 0.21 (A) | 3.67 (I) |
| 48 | 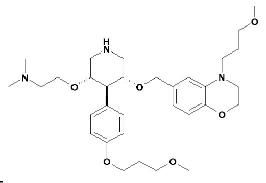 | brown resin | 0.33 (H) | 3.12 (I) |
| 49 | 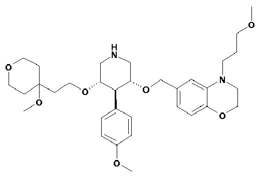 | yellowish oil | 0.1 (C) | 3.57 (I) |
| 50 | 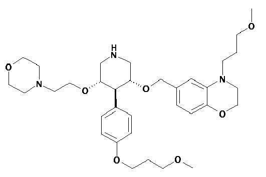 | yellowish oil | 0.29 (I) | 3.19 (I) |
| 51 | 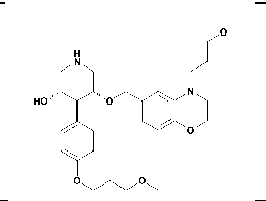 | yellow resin | 0.20 (A) | 3.40 (I) |
| 52 | 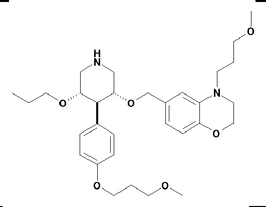 | pale yellow resin | 0.38 (A) | 4.06 (I) |
| 53 | 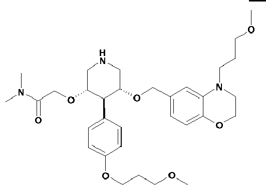 | yellowish oil | 0.31 (I) | 3.57 (I) |
| 54 | 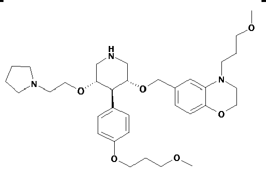 | yellowish oil | 0.12 (I) | 3.24 (I) |
| 55 | 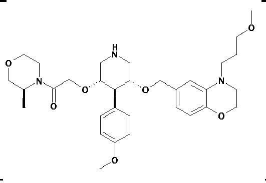 | yellow resin | 0.48 (G) | 3.51 (I) |
| 56 | 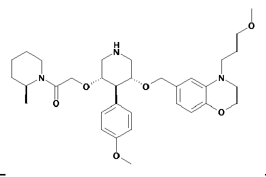 | orange resin | 0.42 (G) | 3.94 (I) |
| 57 | 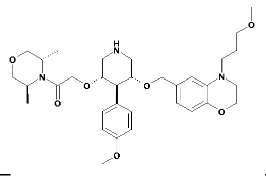 | orange resin | 0.32 (G) | 3.59 (I) |
| 58 | 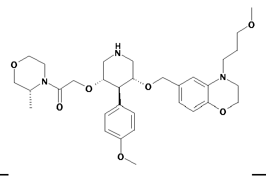 | yellow oil | 0.27 (A) | 3.48 (I) |
| 59 | 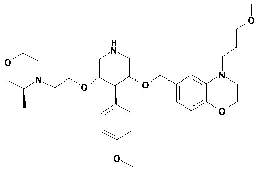 | yellow oil | 0.17 (A) | 3.10 (I) |
| 60 | 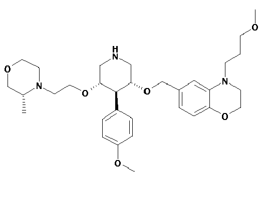 | yellow oil | 0.16 (A) | 3.14 (I) |
| 61 | 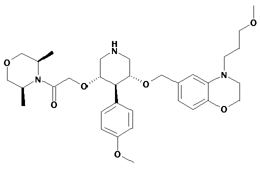 | yellow oil | 0.31 (A) | 3.61 (I) |
| 62 | 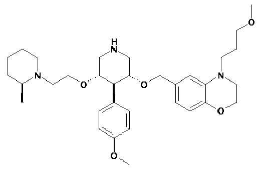 | yellow oil | 0.13 (H) | 3.27 (I) |
| 63 | 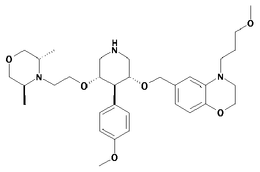 | yellowish resin | 0.25 (D) | 3.17 (I) |
| 64 | 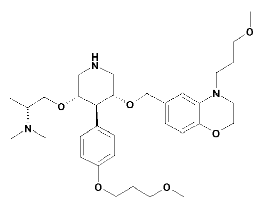 | yellow oil | 0.11 (A) | 3.17 (I) |
| 65 | 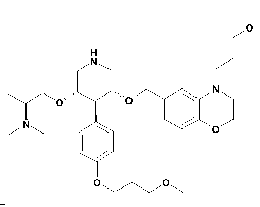 | yellow oil | 0.11 (A) | 3.18 (I) |
| 66 | 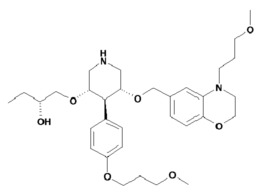 | pale yellow resin | 0.16 (A) | 3.70 (I) |
| 67 | 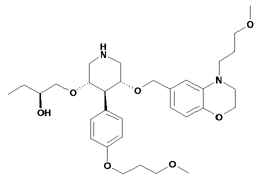 | pale yellow resin | 0.165 (A) | 3.67 (I) |
| 68 | 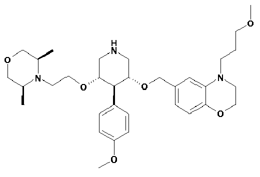 | yellowish resin | 0.17 (G) | 3.18 (I) |
| 69 | 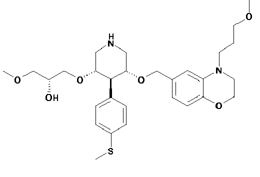 | yellow oil | 0.27 (A) | 3.58 (I) |
| 70 | 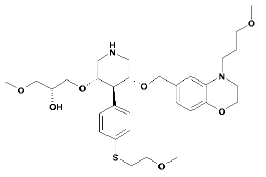 | yellow oil | 0.45 (D) | 3.53 (I) |
| 71 | 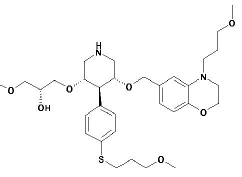 | yellow oil | 0.44 (D) | 3.71 (I) |
| 72 | 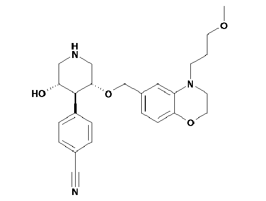 | colourless oil | 0.31 (A) | 3.14 (I) |
| 73 | 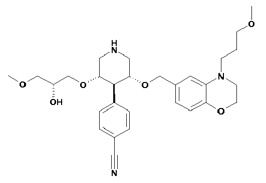 | colourless oil | 0.32 (A) | 3.24 (I) |
| 74 | 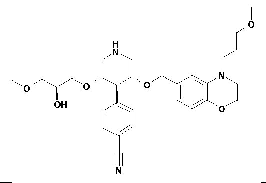 | yellow wax | 0.27 (A) | 3.22 (I) |
| 75 | 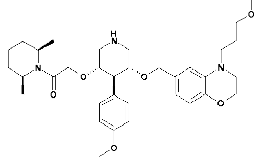 | yellowish resin | 0.38 (D) | 4.09 (I) |
| 76 | 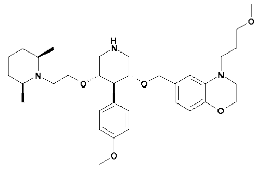 | yellow resin | 0.08 (A) | 3.34 |
| 77 | 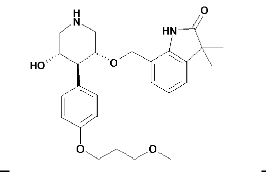 | white foam | 0.21 (A) | 3.17 (I) |
| 78 | 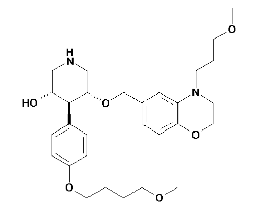 | yellowish oil | 0.08 (C) | 3.56 (I) |
| 79 | 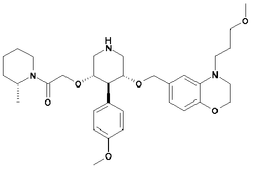 | yellowish resin | 0.35 (G) | 3.93 (I) |
| 80 | 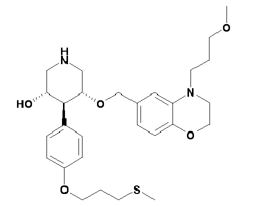 | yellowish resin | 0.18 (G) | 3.79 (I) |
| 81 | 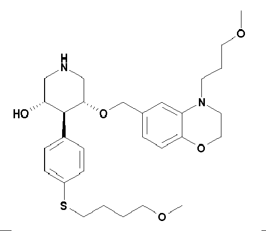 | yellowish oil | 0.22 (A) | 3.76 (I) |
| 82 | 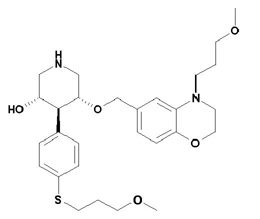 | yellowish resin | 0.26 (A) | 3.63 (I) |
| 83 | 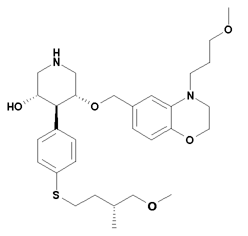 | yellowish oil | 0.22 (A) | 4.01 (I) |
| 87 | 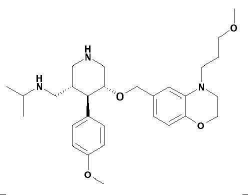 | beige foam | 0.04 (H) | 3.09 (I) |
| 91 | 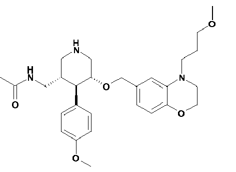 | yellowish foam | 0.71 (K) | 3.30 (I) |
| 92 | 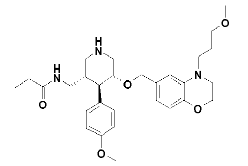 | pale yellow oil | 0.21 (I) | 3.43 (I) |
| 94 | 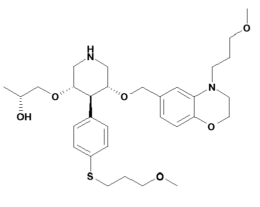 | yellow oil | 0.19 (A) | 3.75 (I) |
| 95 | 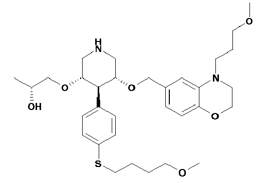 | yellow oil | 0.18 (A) | 3.87 (I) |
| 96 | 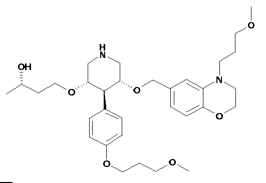 | yellow oil | 0.19 (A) | 3.58 (I) |
| 97 | 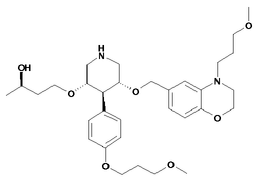 | violet resin | 0.40 (G) | 3.55 (I) |
| 98 | 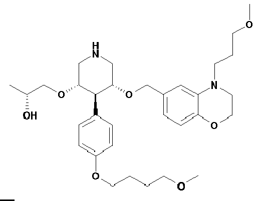 | yellowish oil | 0.27 (A) | 3.65 (I) |
| 99 | 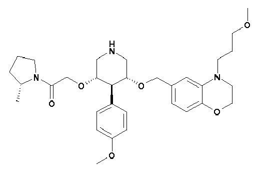 | yellowish resin | 0.39 (G) | 3.74 (I) |
| 100 | 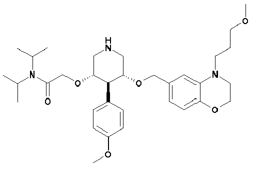 | yellowish resin | 0.44 (G) | 4.12 (I) |
| 101 | 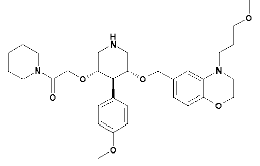 | yellowish oil | 0.40 (G) | 3.80 (I) |
| 102 | 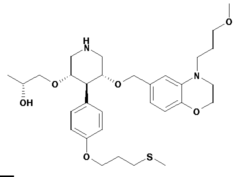 | yellow oil | 0.19 (A) | 3.89 (I) |
| 103 | 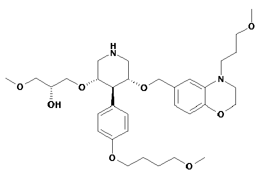 | colourless oil | 0.32(A) | 3.65 (I) |
| 104 | 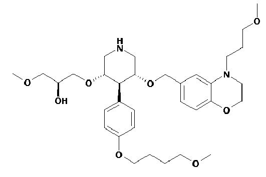 | colourless oil | 0.32 (A) | 3.63 (I) |
| 105 | 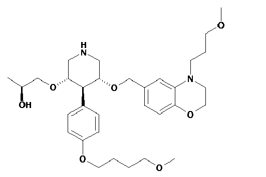 | yellow oil | 0.30 (A) | 3.68 (I) |
| 106 | 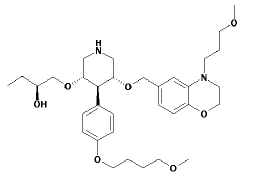 | yellow oil | 0.21 (A) | 3.84 (I) |
| 107 | 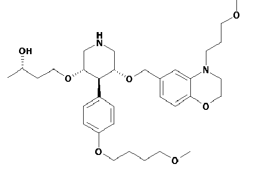 | beige oil | 0.26 (A) | 3.71 (I) |
| 108 | 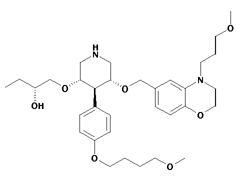 | yellowish oil | 0.25 (A) | 3.86 (I) |
| 109 | 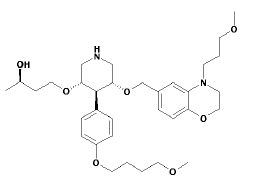 | yellowish oil | 0.45 (A) | 3.70 (I) |
| 110 | 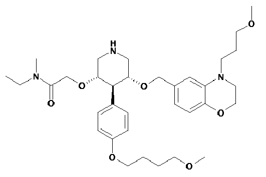 | colourless oil | 0.23 (A) | 3.86 (I) |
| 111 | 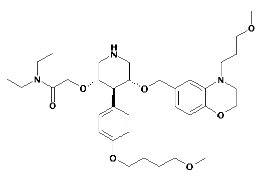 | colourless oil | 0.23 (A) | 3.98 (I) |
| 112 | 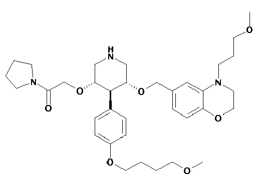 | colourless oil | 0.22 (A) | 3.87 (I) |
| 113 | 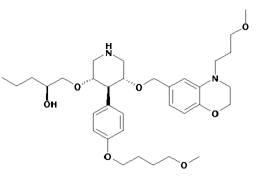 | beige oil | 0.20 (A) | 4.01 (I) |
| 114 | 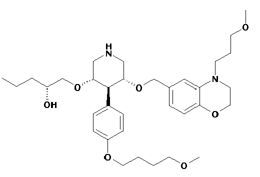 | yellowish oil | 0.41 (A) | 4.04 (I) |
| 116 | 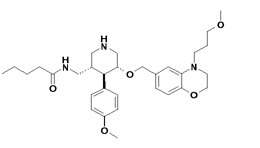 | pale yellow oil | 0.15 (A) | 3.81 (I) |
| 117 | 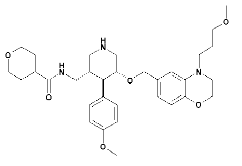 | pale yellow oil | 0.16 (I) | 3.38 (I) |
| 119 | 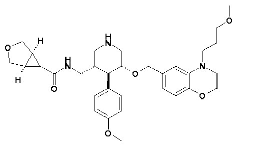 | white foam | 0.15 (I) | 3.42 (I) |
| 122 | 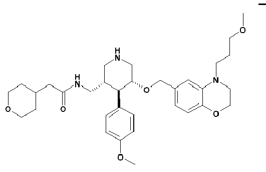 | colourless oil | 0.41 (A) | 3.40 (I) |
| 125 | 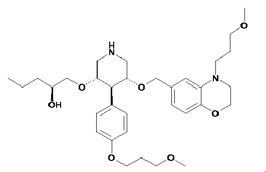 | colorless oil | 0.28 (A) | 3.82 (I) |
| 126 | 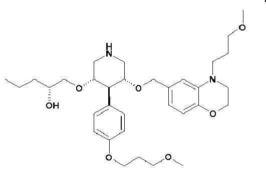 | colorless oil | 0.28 (A) | 3.84 (I) |
| 127 | 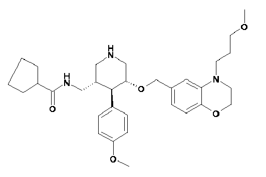 | pale yellow oil | 0.17 (I) | 3.80 (I) |
| 128 | 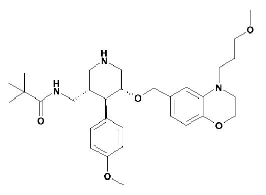 | pale yellow oil | 0.12 (I) | 3.81 (I) |
| 129 | 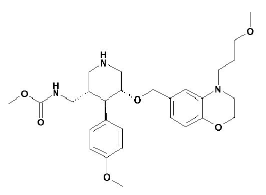 | pale yellow oil | 0.32 (I) | 3.53 (I) |
| 130 | 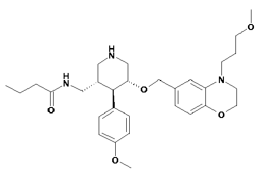 | pale yellow oil | 0.19 (I) | 3.57 (I) |
| 131 | 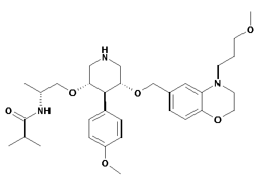 | pale yellow oil | 0.22 (A) | 3.71 (I) |
| 132 | 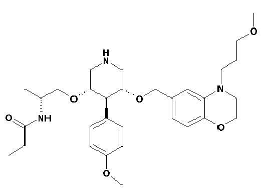 | pale yellow oil | 0.19 (A) | 3.57 (I) |
| 133 | 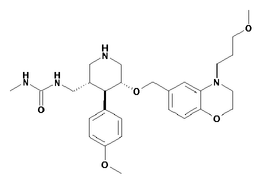 | pale yellow foam | 0.10 (I) | 3.29 (I) |
| 134 | 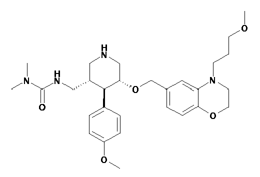 | pale yellow foam | 0.12 (I) | 3.44 (I) |
| 135 | 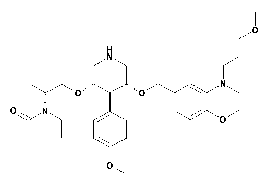 | pale yellow oil | 0.21 (A) | 3.67 (I) |
| 136 | 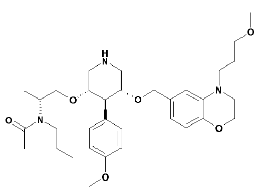 | pale yellow oil | 0.27 (A) | 3.85 (I) |
| 137 | 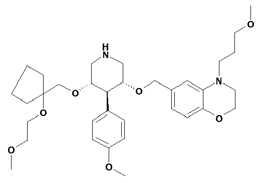 | yellow oil | 0.46 (A) | 4.10 (I) |
| 138 | 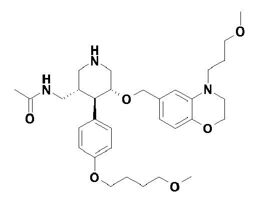 | pale yellow oil | 0.35 (A) | 3.61 (I) |
| 139 | 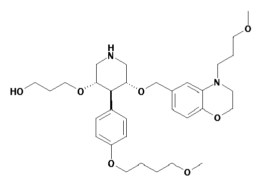 | pink oil | 0.35 (A) | 3.63 (I) |

Mobile phase systems for thin-layer chromatography:
- A: Dichloromethane-methanol-25% conc. ammonia = 200:20:1
- B: Dichloromethane-methanol-25% conc. ammonia = 200:20:0.5
- C: Dichloromethane-methanol-25% conc. ammonia = 200:10:1
- D: Dichloromethane-methanol-25% conc. ammonia = 90:10:1
- E: Dichloromethane-methanol-25% conc. ammonia = 60:10:1
- F: Dichloromethane-methanol-25% conc. ammonia = 200:30:1
- G: Dichloromethane-methanol = 9:1
- H: Dichloromethane-methanol-25% conc. ammonia = 200:40:1
- I: Dichloromethane-methanol-25% conc. ammonia = 100:10:1
- J: Dichloromethane-methanol = 20:1
- K: Dichloromethane-methanol-25% conc. ammonia = 40:10:1

HPLC gradient on Hypersil BDS C-18 (5 µm); column: 4 x 125 mm
(I) 90% water*/10% acetonitrile* to 0% water*/100% acetonitrile* in 5 minutes + 2.5 minutes (1.5 ml/min)
(II) 95% water*/5% acetonitrile* to 0% water*/100% acetonitrile* in 40 minutes (0.8 ml/min)
   HPLC gradient on Zorbax SB-C18 (3.5 µm); column: 2.1 x 30 mm
(III) 97.5% water*/2.5% acetonitrile* to 5% water*/95% acetonitrile* in 5.5 minutes + 2.4 minutes (0.5 ml/min)
* contains 0.1 % trifluoroacetic acid

The following abbreviations are used:
Rf ratio of distance migrated by a substance to the distance of the solvent front from the starting point in thin-layer chromatography
Rt retention time of a substance in HPLC (in minutes)
M.p. melting point (temperature)

### General Method A: (N-BOC deprotection)

15 ml of methanol and 2.5 ml of 2N HCl are successively added to a solution of 1 mmol "N-BOC derivative" in 5 ml of chloroform, and the mixture is stirred at 60°C for 18 hours. The reaction mixture is cooled to room temperature, poured into 1 M aqueous sodium bicarbonate solution (40 ml) and extracted with tert-butyl methyl ether (2 x 60 ml). The organic phases are washed with brine (1 x 60 ml), dried with sodium sulfate and evaporated. The title compound is obtained from the residue by flash chromatography (SiO₂ 60F).

### General Method B: (hydrogenation)

A solution of 1 mmol of "substrate" in 15 ml of tetrahydrofuran/methanol 1:1 is hydrogenated in the presence of 100-200 mg of Pd/C 10% at 15-20°C for 2-20 hours. The reaction mixture is clarified by filtration and the filtrate is evaporated. The title compound is obtained from the residue by flash chromatography (SiO₂ 60F).

### General Method C: (9-BBN reduction)

A solution of 1 mmol of "lactam" in 3 ml of tetrahydrofuran is mixed with 3.2-6.4 mmol of 9-BBN (0.5M in tetrahydrofuran) and stirred under reflux for 1-2 hours (conversion checked by HPLC). The reaction mixture is cooled to room temperature and, after addition of 3.2-6.4 mmol of ethanolamine, evaporated. The residue is stirred in ethyl acetate/heptane 1:1 (30 ml) at 0°C overnight and clarified by filtration, and the filtrate is evaporated. The title compound is obtained from the residue by flash chromatography (SiO 60F).

### General Method D: (O-alkylation)

1.1 mmol of sodium hydride (60% dispersion in oil) are added to a solution of 1 mmol of "alcohol", 1.0-2.0 mmol of "benzyl halide" in 2.0 ml of N,N-dimethylformamide while stirring at -10°C. The reaction mixture is stirred at -10°C for 1 hour and at room temperature for 18 hours. The mixture is poured into 1 M aqueous sodium bicarbonate solution (50 ml) and extracted with tert-butyl methyl ether (2 x 50 ml). The organic phases are washed successively with water (1 x 50 ml) and brine (1 x 60 ml), dried with sodium sulfate and evaporated. The title compound is obtained from the residue by flash chromatography (SiO₂ 60F).

### General Method E: (chlorination)

A solution of 40 mmol of "benzyl alcohol" in 6.40 ml of pyridine and 100 ml of dichloromethane is slowly added dropwise to a solution, precooled to 0-5°C, of 7.65 ml of thionyl chloride in 20 ml of dichloromethane. The reaction mixture is stirred at 0°C and then at room temperature for 1 hour each, and then poured into 200 ml of ice-water. The mixture is extracted with dichloromethane (2 x 200 ml). The organic phases are washed successively with 1 M aqueous sodium bicarbonate solution (2 x 200 ml) and brine, dried with sodium sulfate and evaporated. The title compound is obtained from the residue by flash chromatography (SiO₂ 60F).

### General Method F: (phenol alkylation I)

A mixture of 20 mmol of "phenol" in 60 ml of N,N-dimethylformamide with 4.15 g of potassium carbonate and 30 mmol of "halide" or "tosylate" is stirred at 100°C for 24 hours. The reaction mixture is then evaporated. The residue is mixed with 1 M aqueous sodium bicarbonate solution (40 ml) and extracted with ethyl acetate (2 x 60 ml). The organic phases are washed with brine (1 x 60 ml), dried with sodium sulfate and evaporated. The title compound is obtained from the residue by flash chromatography (SiO 60F).

### General Method G: (phenol alkylation II)

A suspension of 1 mmol of "tosylate", 2 mmol "phenol", 2 mmol of potassium carbonate and 20 ml of acetonitrile is stirred at 90°C for 24 h. The reaction mixture is then evaporated. The residue is mixed with saturated aqueous sodium bicarbonate solution and extracted with ethyl acetate (2x). The organic phases are washed with brine, dried with sodium sulfate and evaporated. The title compound is obtained from the residue by flash chromatography (SiO₂ 60F).

### General Method H: (tosylation)

A solution of 12 mmol of p-toluenesulfonyl chloride in 15 ml of dichloromethane is added dropwise to a solution of 10 mmol of "alcohol", 15 mmol of triethylamine, 1 mmol of 4-dimethylaminopyridine in 90 ml of dichloromethane at 0°C. The reaction mixture is stirred at room temperature for 2-18 hours. The reaction mixture is diluted with dichloromethane and then washed with water and brine, dried with sodium sulfate and evaporated. The title compound is obtained from the residue by flash chromatography (SiO₂ 60F).

### General Method I: (phenol alkylation III)

A suspension of 1 mmol of "phenol", 1.0-1.5 mmol of "tosylate" or "bromide", 1.5 mmol of caesium carbonate and 2 ml of acetonitrile is stirred at 80°C for 2 hours. The reaction mixture is cooled, poured into water and extracted with ethyl acetate (2x). The organic phases are washed with brine, dried with sodium sulfate and evaporated. The title compound is obtained from the residue by flash chromatography (SiO₂ 60F).

### General Method J (alcohol desilylation)

A solution of 1 mmol of "silyl ether" in 5 ml of tetrahydrofuran is mixed with 1.5-2.0 mmol of tetrabutylammonium fluoride (1 M solution in tetrahydrofuran), and the solution is stirred at room temperature for 1-2 hours. The reaction solution is then diluted with water and extracted 2x with tert-butyl methyl ether. The combined organic phases are dried with sodium sulfate and evaporated. The title compound is obtained from the residue by flash chromatography (SiO 60F).

### General Method K (borane reduction)

A solution of 1 mmol of "lactam" in 3 ml of tetrahydrofuran is mixed with 3.0-6.0 mmol of borane-tetrahydrofuran complex (1 M in tetrahydrofuran) and stirred at room temperature for 1-3 hours (conversion checked by HPLC or TLC). The reaction mixture is cooled to room temperature, mixed with 3.0-6.0 mmol of methanol and evaporated. The title compound is obtained from the residue by flash chromatography (SiO₂ 60F).

### General Method L (N-Tos deprotection I)

0.44 mmol of sodium dihydrogen phosphate and 0.90 mmol of sodium amalgam (10% Na) are successively added at room temperature to a solution of 0.09 mmol of "tosylamide" in 10 ml of methanol. The reaction mixture is stirred for 2-18 hours, diluted with water and extracted with ethyl acetate. The organic phase is separated off and washed with brine, dried with sodium sulfate and evaporated. The title compound is obtained from the residue by flash chromatography (SiO₂ 60F).

### General Method M (O-alkylation II)

1 mmol of methylmagnesium bromide (35% solution in diethyl ether) is added to a solution of 1 mmol of "secondary alcohol" in 5 ml of tetrahydrofuran at room temperature. The reaction solution is heated to reflux for 5 minutes and then a solution of 2.2 mmol of "oxirane" in 1 ml of THF is added. The reaction mixture is heated to reflux for 1-5 hours and poured into saturated aqueous sodium bicarbonate solution, and the mixture is extracted with tert-butyl methyl ether. The combined organic phases are dried over sodium sulfate and evaporated. The title compound is obtained from the residue by flash chromatography (SiO₂ 60F).

### General Method N (N-Tos deprotection II)

0.5 ml of a bluish green sodium naphthalenide stock solution (from 0.04 g of sodium and 0.22 g of naphthalene in 5 ml of dimethoxyethane) is added to a solution of 0.1 mmol of "tosylamide" in 2 ml of dimethoxyethane at -60°C. After 3-6 hours, the reaction mixture is diluted with water and extracted with dichloromethane (2x). The combined organic phases are washed with brine, dried with sodium sulfate and evaporated. The title compound is obtained from the residue by flash chromatography (SiO₂ 60F).

### Example 1

### {(3S,4R,5R)-4-(4-Methoxyphenyl)-5-[4-(3-methoxyproipyl)-3,4-dihydro-2H-benzo[1,4]oxazin-6-ylmethoxy]piperidin-3-yloxy}acetonitrile

The title compound is prepared in analogy to method L from 0.185 g of [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetonitrile.

The starting materials are prepared as follows:
a) [(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetonitrile
   0.164 g of sodium hydride (60% dispersion in oil) is added to a stirred solution of 0.50 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol in 4 ml of acetonitrile. The reaction mixture is stirred at room temperature for 1 hour. 0.762 g of bromoacetonitrile is added at -20°C, and the mixture is stirred at -20°C for 48 hours. The reaction mixture is poured into 1 M aqueous sodium bicarbonate solution (30 ml) and extracted with tert-butyl methyl ether (2 X 100 ml). The organic phases are dried with sodium sulfate and evaporated. The title compound is obtained as a yellowish resin from the residue by flash chromatography (SiO₂ 60F). Rf = 0.30 (EtOAc-heptane 1:1); Rt = 5.13 (Gradient I).
b) (3S,4S,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-ol
   14.74 g of tetrabutylammonium fluoride trihydrate are added to a solution of 23.2 g of 6-[(3R,4R,5S)-4-(4-methoxyphenyl)-1-(toluene-4-sulfonyl)-5-triisopropylsilanyloxypiperidin-3-yloxymethyl]-4-(3-methoxypropyl)-3,4-dihydro-2H-benzo[1,4]oxazine in 500 ml of tetrahydrofuran at room temperature. After 1 hour 10 ml of water are added to the reaction mixture, and the mixture is evaporated to dryness. The title compound is obtained as a yellowish oil from the residue by flash chromatography (SiO₂ 60F). Rf = 0.06 (EtOAc-heptane 1:2); Rt = 4.73 (Gradient I).
c) 6-[(3R,4R,5S)-4-(4-Methoxyphenyl)-1-(toluene-4-sulfonyl)-5-triisoiproipylsilanyloxy-piperidin-3-yloxymethyl]-4-(3-methoxypropyl)-3,4-dihydro-2H-benzo[1,4]oxazine
   24.99 g of 6-[(3R,4R,5S)-4-(4-methoxyphenyl)-1-(toluene-4-sulfonyl)-5-triisopropylsilanyloxy-piperidin-3-yloxymethyl]-4-(3-methoxypropyl)-4H-benzo[1,4]oxazin-3-one are reacted in analogy to method C. The title compound is obtained as a colourless resin. Rf = 0.10 (EtOAc-heptane 1:2).
d) 6-[(3R,4R,5S)-4-(4-Methoxyphenyl)-1-(toluene-4-sulfonyl)-5-triisoiproipylsilanyloxy-piperidin-3-yloxymethyl]-4-(3-methoxypropyl)-4H-benzo[1,4]oxazin-3-one
   19.0 g of (3R,4R,5S)-4-(4-methoxyphenyl)-1-(toluene-4-sulfonyl)-5-triisopropyl-silanyloxy-piperidin-3-ol and 11.52 g of 6-chloromethyl-4-(3-methoxypropyl)-4H-benzo[1,4]oxazin-3-one are reacted in analogy to method D. The title compound is obtained as a yellowish resin. Rf = 0.18 (EtOAc-heptane 1:2); Rt = 6.67 (Gradient I).
e) (3R,4R,5S)-4-(4-Methoxyphenyl)-1-(toluene-4-sulfonyl)-5-triisopropylsilanyloxypiperidin-3-ol
   A suspension of 55.64 g (3R,4R,5S)-4-(4-hydroxyphenyl)-1-(toluene-4-sulfonyl)-5-triisopropylsilanyloxypiperidin-3-ol, 15.0 g of dimethyl sulfate, 20.92 g of potassium carbonate and 750 ml of acetone is stirred at 80°C for 24 h. The reaction mixture is clarified by filtration and evaporated. The residue is diluted with 1.75 I of tert-butyl methyl ether, and 1 l of water is added. The aqueous phase is again extracted with 1 I of tert-butyl methyl ether. The combined organic phases are washed with 750 ml of brine, dried over sodium sulfate and evaporated. The crude title compound is obtained as a white foam from the residue. Rt = 6.27 (Gradient I).
f) (3R,4R,5S)-4-(4-Hydroxyphenyl)-1-(toluene-4-sulfonyl)-5-triisopropylsilanyloxypiperidin-3-ol
   26.08 g of toluenesulfonyl chloride are added to a mixture of 50 g of (3R,4R,5S)-4-(4-hydroxyphenyl)-5-triisopropylsilanyloxypiperidin-3-ol in 1 I of ethyl acetate and 1 I of 2N sodium carbonate solution at 0°C. After 4 hours at 0°C, the reaction mixture is stirred at room temperature for a further 16 hours. The phases are separated and the aqueous phase is extracted with 200 ml of ethyl acetate. The combined organic phases are washed with 200 ml of brine, dried with sodium sulfate and evaporated. The title compound is obtained in the form of white crystals. Rf = 0.31 (EtOAc-heptane 1:1.5); Rt = 5.77 (Gradient I).
g) (3R,4R,5S)-4-(4-Hydroxyphenyl)-5-triisopropylsilanyloxypiperidin-3-ol
   5.210 g of (3R,4R,5S)-4-(4-benzyloxyphenyl)-1-((R)-1-phenylethyl)-5-triisopropylsilanyloxy-piperidin-3-ol are reacted in analogy to method B. The title compound is obtained as a colourless solid. Rf = 0.19 (dichloromethane-methanol-25% conc. ammonia = 200:20:1); Rt = 3.80 (Gradient I).
h) (3R,4R,5S)-4-(4-Benzyloxyphenyl)-1-((R)-1-phenylethyl)-5-triisopropylsilanyloxypiperidin-3-ol
   150 ml of borane-tetrahydrofuran complex (1M in tetrahydrofuran) are added dropwise to a solution of 20.00 g of (S)-4-(4-benzyloxyphenyl)-1-((R)-1-phenylethyl)-3-triisopropylsilanyloxy-1,2,3,4-tetrahydropyridine in 280 ml of 1,2-dimethoxyethane at 0°C. The reaction solution is then stirred at 30°C for 3 hours. The solution is cooled to room temperature and quenched with 70 ml of water. After stirring for 5 minutes, 56.00 g of sodium percarbonate are added, and the suspension is stirred at 50°C for 1 hour. The reaction mixture is poured into 600 ml of water and extracted with ethyl acetate (2X). The combined organic phases are washed with 400 ml each of water and brine and evaporated. The title compound is obtained as a yellowish oil from the residue by flash chromatography (SiO F60). Rf = 0.23 (EtOAc-heptane 1:2); Rt = 5.75 (Gradient I).
i) (S)-4-(4-Benzyloxyphenyl)1-1((R)-1-phenylethyl)-3-triisopropylsilanyloxy-1,2,3,6 tetrahydropyridine
   6.80 ml of 2,6-lutidine are added to a suspension of 14.70 g of 4-(4-benzyloxyphenyl)-1-(1(R)-phenylethyl)-1,2,3,6-tetrahydropyridin-3(S)-ol [257928-45-3] in 250 ml of dichloromethane, and the mixture is cooled to 0°C. 12.60 ml of triisopropysilyl trifluoromethanesulfonate are added dropwise, and the reaction mixture is stirred at 0°C for 1 hour. The reaction solution is poured into 400 ml of water and the phases are separated. The aqueous phase is back-extracted with 200 ml of dichloromethane, and the combined organic phases are dried with sodium sulfate and evaporated. The title compound is obtained as a yellow-brown oil from the residue by flash chromatography (SiO₂ F60). Rf = 0.66 (EtOAc-heptane 1:2); Rt = 5.83 (Gradient I).
j) 6-Chloromethyl-4-(3-methoxypropyl)-4H-benzo[1,4]oxazin-3-one
   0.37 g of 6-hydroxymethyl-4-(3-methoxypropyl)-4H-benzo[1,4]oxazin-3-one is reacted in analogy to method E. The title compound is obtained as a colourless oil. Rf = 0.60 (EtOAc-heptane 2:1); Rt = 4.05 (Gradient I).
k) 6-Hydroxymethyl-4-(3-methoxypropyl)-4H-benzo[1,4]oxazin-3-one
   A suspension of 1.79 g of 6-hydroxymethyl-4H-benzo[1,4]oxazin-3-one, 2.20 ml of 1-chloro-3-methoxypropane, 10 g of potassium fluoride on alumina and 0.033 g of potassium iodide in 150 ml of acetonitrile is stirred under reflux for 72 hours. The reaction mixture is cooled and clarified by filtration, and the filtrate is evaporated to dryness. The title compound is obtained as a yellow oil from the residue by flash chromatography (SiO₂ 60F). Rf = 0.60 (dichloromethane-methanol 9:1); Rt = 2.74 (Gradient I).
m) 6-Hydroxymethyl-4H-benzo[1,4]oxazin-3-one
   A mixture of 6.9 g of methyl 3-oxo-3,4-dihydro-2H-benzo[1,4]oxazine-6-carboxylate [202195-67-3] and 230 ml of tetrahydrofuran is cooled to -40°C. 88.9 ml of diisobutylaluminium hydride (1.5M in toluene) are added dropwise at -40°C over the course of 30 minutes. The reaction mixture is stirred at -40°C to -20°C for 1.5 hours and then cautiously poured into 150 ml of 2N HCl (cold). The organic phase is separated off and the aqueous phase is extracted with tetrahydrofuran (5 X 100 ml). The organic phases are washed with brine (1 X 100 ml), filtered through cotton wool and evaporated. The title compound is obtained as beige crystals from the residue by crystallization (from ethanol). Rf = 0.16 (EtOAc-heptane 2:1); Rt = 2.23 (Gradient I); m.p.: 186-187°C.

### Example 2

### (R)-1-Methoxy-3-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}propan-2-ol

The title compound is prepared from 0.282 g of (R)-1-methoxy-3-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-propan-2-ol in analogy to method L.

The starting material is prepared as follows:
a) (R)-1-Methoxy-3-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propan-2-ol
   0.30 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) and 0.098 g of S-(+)-glycidyl methyl ether [64491-68-5] are reacted in analogy to method M. The title compound is obtained as a colourless oil. Rf = 0.19 (EtOAc-heptane 2:1); Rt = 4.81 (Gradient I).

The following compound is prepared in an analogous manner to the process described in Example 2:

### Example

### 3 (S)-1-Methoxy-3-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}propan-2-ol using R-(-)-glycidyl methyl ether [64491-70-9]

### Example 5

### 6-[(3R,4R,5S)-4-(4-Methoxyphenyl)-5-prop-2-ynyloxypiperidin-3-yloxymethyl]-4-(3-methoxy-propyl)-3,4-dihydro-2H-benzo[1,4]oxazine

0.236 g of sodium dihydrogenphosphate and 0.743 g of sodium amalgan (10% Na) are successively added to a solution of 0.209 g of 6-[(3R,4R,5S)-4-(4-methoxyphenyl)-5-prop-2-ynyloxy-1-(toluene-4-sulfonyl)piperidin-3-yloxymethyl]-4-(3-methoxypropyl)-3,4-dihydro-2H-benzo[1,4]oxazine in 20 ml of tetrahydrofuran at 0°C. The reaction mixture is stirred at 0°C for 1 hour and then at room temperature for 20 hours. The mixture is decanted and clarified by filtration through Hyflo. The filtrate is evaporated. The title compound is obtained from the residue by flash chromatography (SiO₂ 60F).

The starting materials are prepared as follows:
a) 6-[(3R,4R,5S)-4-(4-Methoxyphenyl)-5-prop-2-ynyloxy-1-(toluene-4-sulfonyl)piperidin-3-yloxymethyl]-4-(3-methoxypropyl)-3,4-dihydro-2H-benzo[1,4]oxazine
   0.263 g of sodium hydride (60% dispersion in oil) is added to a solution of 1.50 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) and 1.099 g of 3-bromo propyne in 25 ml of tetrahydrofuran. After stirring for 22 hours, the reaction mixture is mixed with 40 ml of saturated sodium bicarbonate solution and extracted with tert-butyl methyl ether (3 X 50 ml). The combined organic phases are washed with 40 ml of brine, dried with sodium sulfate and evaporated. The title compound is obtained as a yellowish resin from the residue by flash chromatography (SiO₂ 60F). Rf = 0.18 (EtOAc-heptane 1:2); Rt = 5.26 (Gradient I).

### Example 6

### 6-[(3R,4R,5S)-4-(4-Methoxyphenyl)-5-(4-morpholin-4-yl-but-2-ynyloxy)piperidin-3-yloxymethyl]-4-(3-methoxypropyl)-3,4-dihydro-2H-benzo[1,4]oxazine

0.194 g of sodium dihydrogenphosphate and 0.612 g of sodium amalgan (10% Na) are successively added to a solution of 0.199 g of 6-[(3R,4R,5S)-4-(4-methoxyphenyl)-5-(4-morpholin-4-ylbut-2-ynyloxy)-1-(toluene-4-sulfonyl)piperidin-3-yloxymethyl]-4-(3-methoxypropyl)-3,4-dihydro-2H-benzo[1,4]oxazine in 20 ml of tetrahydrofuran at 0°C. The reaction mixture is stirred at 0°C for 1 hour and then at room temperature for 24 hours. The mixture is decanted and clarified by filtration through Hyflo. The filtrate is evaporated. The title compound is obtained from the residue by flash chromatography (SiO₂ 60F).

The starting materials are prepared as follows:
a) 6-[(3R,4R,5S)-4-(4-Methoxyphenyl)-5-(4-moripholin-4-ylbut-2-ynyloxy)-1-(toluene-4-sulfonyl)piperidin-3-yloxymethyl]-4-(3-methoxypropyl)-3,4-dihydro-2H-benzo[1,4]oxazine
   A suspension of 0.038 g of paraformaldehyde and 0.032 g of morpholine in 4 ml of dioxane is heated until the solution is clear and then stirred at room temperature for 20 minutes.
   A solution of 0.21 g of 6-[(3R,4R,5S)-4-(4-methoxyphenyl)-5-prop-2-ynyloxy-1-(toluene-4-sulfonyl)piperidin-3-yloxymethyl]-4-(3-methoxypropyl)-3,4-dihydro-2H-benzo[1,4]oxazine (Example 5a) and 0.068 g of copper(II) acetate in 1 ml of dioxane is added to this solution. After 18 hours at 90°C, the reaction mixture is diluted with tert-butyl methyl ether and washed successively with water and brine. The organic phase is dried with sodium sulfate and evaporated. The title compound is obtained as a yellowish resin from the residue by flash chromatography (SiO₂ 60F). Rf = 0.19 (EtOAc-heptane 2:1); Rt = 4.55 (Gradient I).

The following compound is prepared in an analogous manner to the process described in Example 6:

### Example

### 7 (4-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-but-2-ynyl)dimethylamine

### Example 8

### 6-[(3R,4R,5S)-5-(2-Methoxy-2-methylpropoxy)-4-(4-methoxyphenyl)piperidin-3-yloxymethyl]-4-(3-methoxypropyl)-3,4-dihydro-2H-benzo[1,4]oxazine

The title compound is prepared from 0.20 g of 6-[(3R,4R,5S)-5-(2-methoxy-2-methylpropoxy)-4-(4-methoxyphenyl)-1-(toluene-4-sulfonyl)piperidin-3-yloxymethyl]-4-(3-methoxypropyl)-3,4-dihydro-2H-benzo[1,4]oxazine in analogy to method L.

The starting materials are prepared as follows:
a) 6-[(3R,4R,5S)-5-(2-Methoxy-2-methylpropoxy)-4-(4-methoxyphenyl)-1-(toluene-4-sulfonyl)piperidin-3-yloxymethyl]-4-(3-methoxypropyl)-3,4-dihydro-2H-benzo[1,4]oxazine
   0.03 g of sodium hydride (60% dispersion in oil) is added to a stirred solution of 0.20 g of 1-[(3S,4R,5R)-4-(4-methoxyphenyl)-5-[4-(3-methoxypropyl)-3,4-dihydro-2H-benzo-[1,4]oxazin-6-ylmethoxy]-1-(toluene-4-sulfonyl)piperidin-3-yloxy]-2-methylpropan-2-ol in 1.5 ml of N,N-dimethylformamide. After 45 minutes at room temperature, 0.129 g of methyl iodide is added to the mixture. After 3 hours at room temperature, the reaction mixture is diluted with tert-butyl methyl ether. The solution is washed successively with aqueous sodium bicarbonate solution, water and brine, dried with sodium sulfate and evaporated. The title compound is obtained as a yellowish oil from the residue by flash chromatography (SiO₂ 60F). Rt = 5.56 (Gradient I).
b) 1-[(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-2-methylpropan-2-ol
   0.558 ml of a solution of methylmagnesium bromide (3N in diethyl ether) is added to a solution of 0.23 g of methyl [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy] acetate in 1.5 ml of tetrahydrofuran. After 15 minutes at 50°C, the reaction mixture is cooled to room temperature and diluted with 50 ml of tert-butyl methyl ether. The solution is washed successively with 20 ml of saturated aqueous sodium bicarbonate solution and 10 ml of brine. The combined aqueous phases are extracted with 50 ml of tert-butyl methyl ether. The combined organic phases are dried with sodium sulfate and evaporated. The title compound is obtained as a white resin from the residue. Rt = 5.16 (Gradient I).
c) Methyl [(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy] acetate
   0.085 g of sodium hydride (60% dispersion in oil) is added to a solution of 0.513 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) in 8 ml of tetrahydrofuran while stirring. The reaction mixture is stirred at room temperature for 1 hour and then, at -20°C, 0.40 g of methyl bromoacetate is added. After 1 hour at -20°C and 3 hours at room temperature, the reaction mixture is diluted with 100 ml of tert-butyl methyl ether and washed with saturated aqueous sodium bicarbonate solution (30 ml). The aqueous phase is extracted with tert-butyl methyl ether (2 X 50 ml). The combined organic phases are dried with sodium sulfate and evaporated. The title compound is obtained as a yellowish resin from the residue by flash chromatography (SiO₂ 60F). Rf = 0.14 (EtOAc-heptane 1:2); Rt = 5.21 (Gradient I).

### Example 9

### 1-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-2-methylpropan-2-ol

The title compound is prepared from 0.125 g of 1-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-2-methylpropan-2-ol (Example 8b) in analogy to method L.

The following compound is prepared in an analogous manner to the process described in Example 9:

### Example

### 10 3-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxymethyl}pentan-3-ol

### Example 11

### (R)-1-Methoxy-3-[(3S,4R,5R)-5-[4-(3-methoxypropyl)-3,4-dihydro-2H-benzo[1,4]oxazin-6-ylmethoxy]-4-(4-propoxyphenyl)piperidin-3-yloxy]propan-2-ol

The title compound is prepared from 0.303 g of benzyl (3S,4R,5R)-4-(4-allyloxyphenyl)-3-((R)-2-hydroxy-3-methoxypropoxy)-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-4-(4-allyloxyphenyl)-3-((R)-2-hydroxy-3-methoxypropoxy)-5-[4-(3-methoxypropyl)-3,4-dihydro-2H-benzo[1,4]oxazin-6-ylmethoxy]piperidin-1-carboxylate
   The title compound is obtained as a colourless oil from 1.400 g of benzyl (3S,4S,5R)-4-(4-allyloxyphenyl)-3-hydroxy-5-[4-(3-methoxypropyl)-3,4-dihydro-2H-benzo[1,4]oxazin-6-ylmethoxy]piperidine-1-carboxylate and 0.460 g of S-(+)-glycidyl methyl ether [64491-68-5] in analogy to method M. Rf = 0.34 (EtOAc-heptane 3:1); Rt = 5.06 (Gradient I).
b) Benzyl (3S,4S,5R)-4-(4-allyloxyphenyl)-3-hydroxy-5-[4-(3-methoxypropyl)-3,4-dihydro-2H-benzo[1,4]oxazin-6-ylmethoxy]piperidin-1-carboxylate
   The title compound is obtained as a colourless resin from 1.860 g of benzyl (3R,4R,5S)-4-(4-allyloxyphenyl)-3-[4-(3-methoxypropyl)-3,4-dihydro-2H-benzo[1,4]oxazin-6-ylmethoxy]-5-triisopropylsilanyloxypiperidine-1-carboxylate in analogy to method J. Rf = 0.18 (EtOAc-heptane 1:1); Rt = 4.97 (Gradient I).
c) Benzyl (3R,4R,5S)-4-(4-allyloxyphenyl)-3-f4-(3-methoxypropyl)-3,4-dihydro-2H-benzo[1,4]oxazin-6-ylmethoxyl-5-triisopropylsilanyloxypiperidine-1-carboxylate
   0.820 g of potassium carbonate and 0.300 ml of allyl bromide are added to a solution of 2.090 g of benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate in 5 ml of N,N-dimethylformamide at room temperature. The reaction mixture is stirred at 40-60°C for 5 hours, poured into saturated aqueous sodium bicarbonate solution and extracted with tert-butyl methyl ether. The combined organic extracts are washed with brine, dried with sodium sulfate and evaporated. The title compound is obtained as a colourless oil from the residue by flash chromatography (SiO₂ 60F). Rf = 0.42 (EtOAc-heptane 1:1).
d) Benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate
   The title compound is obtained as a reddish resin from 5.010 g of benzyl (3R,4R,5S)-4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate in analogy to method K. Rf = 0.32 (EtOAc-heptane 1:1); Rt = 29.32 (Gradient II).
e) Benzyl (3R,4R,5S)-4-(4-hydroxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate
   1.620 g of palladium(0) tetrakistriphenylphosphine and 7.300 g of potassium carbonate are added to a solution of 13.88 g of benzyl (3R,4R,5S)-4-(4-allyloxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate in 100 ml of methanol. The reaction mixture is then stirred at room temperature for 3 hours. The solid is filtered off and the filtrate is evaporated. The title compound is obtained as a colourless oil from the residue by flash chromatography (SiO 60F). Rf = 0.23 (EtOAc-heptane 1:1); Rt = 6.37 (Gradient I).
f) Benzyl (3R,4R,5S)-4-(4-allyloxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate
   The title compound is obtained as a yellow resin from 16.50 g of benzyl (3R,4R,5S)-4-(4-allyloxyphenyl)-3-hydroxy-5-triisopropylsilanyloxypiperidine-1-carboxylate in analogy to method D. Rf = 0.18 (EtOAc-heptane 1:2); Rt = 7.07 (Gradient I).
g) Benzyl (3R,4R,5S)-4-(4-allyloxyphenyl)-3-hydroxy-5-triisopropylsilanyloxypiperidine-1-carboxylate
   16.66 g of benzyl (3R,4R,5S)-3-hydroxy-4-(4-hydroxyphenyl)-5-triisopropylsilanyloxypiperidine-1-carboxylate are reacted in analogy to method 11c. The title compound is obtained as a pale brown resin. Rf = 0.42 (EtOAc-heptane 1:1); Rt = 6.54 (Gradient I).
h) Benzyl (3R,4R,5S)-3-hydroxy-4-(4-hydroxyphenyl)-5-triisopropylsilanyloxypiperidine-1-carboxylate
   20.07 ml of benzyl chloroformate are slowly added to a two-phase mixture of 50 g of (3R,4R,5S)-4-(4-hydroxyphenyl)-5-triisopropylsilanyloxypiperidin-3-ol (Example 1g) in 1000 ml of saturated sodium bicarbonate solution and 1000 ml of ethyl acetate at 0°C. After 3 hours at 0°C and 15 hours at room temperature, the aqueous phase is separated and extracted with 200 ml of ethyl acetate. The combined organic phases are washed with 200 ml of brine, dried with sodium sulfate and evaporated. The title compound is obtained as a colourless oil from the residue by flash chromatography (SiO₂ 60F). Rf = 0.31 (EtOAc-heptane 1:1.5); Rt = 5.77 (Gradient I).

### Example 12

### (R)-1-3-{(3S,4R,5R)-4-(4-Allyloxyphenyl)-5-[4-(3-methoxypropyl)-3,4-dihydro-2H-benzo[1,4]oxazin-6-ylmethoxy]piperidin-3-yloxy}-3-methoxypropyl-2-ol

5 ml of a 40% aqueous potassium hydroxide solution are added to a solution of 0.375 g of benzyl (3S,4R,5R)-4-(4-allyloxyphenyl)-3-((R)-2-hydroxy-3-methoxypropoxy)-5-[4-(3-methoxypropyl)-3,4-dihydro-2H-benzo[1,4]oxazin-6-ylmethoxy]piperidine-1-carboxylate (Example 11a) in 10 ml of 1:1 methanol-dioxane. The mixture is heated in a closed flask at 80°C for 5 hours. The reaction solution is poured into water and extracted with tert-butyl methyl ether. The combined organic extracts are washed with brine, dried with sodium sulfate and evaporated. The title compound is obtained from the residue by flash chromatography (SiO₂ 60F).

### Example 13

### (R)-1-[(3S,4R,5R)-4-(4-Ethoxyphenyl)-5-[4-(3-methoxypropyl)-3,4-dihydro-2H-benzo[1,4]oxazin-6-ylmethoxy]piperidin-3-yloxy]-3-methoxypropan-2-ol

The title compound is prepared from 0.600 g of benzyl (3S,4R,5R)-4-(4-ethoxyphenyl)-3-((R)-2-hydroxy-3-methoxypropoxy)-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-4-(4-ethoxyphenyl)-3-((R)-2-hydroxy-3-methoxypropoxy)-5-[4-(3-methoxypropyl)-3,4-dihydro-2H-benzo[1,4]oxazin-6-ylmethoxy]piperidine-1-carboxylate
   The title compound is obtained as a yellowish resin from 0.800 g of benzyl (3S,4S,5R)-4-(4-ethoxyphenyl)-3-hydroxy-5-[4-(3-methoxypropyl)-3,4-dihydro-2H-benzo[1,4]oxazin-6-ylmethoxy]piperidine-1-carboxylate and 0.268 g of S-(+)-glycidyl methyl ether [64491-68-5] in analogy to method M. Rf = 0.24 (EtOAc-heptane 3:1); Rt = 4.98 (Gradient I).
b) Benzyl (3S,4S,5R)-4-(4-ethoxyphenyl)-3-hydroxy-5-[4-(3-methoxypropyl)-3,4-dihydro-2H-benzo[1,4]oxazin-6-ylmethoxy]piperidine-1-carboxylate
   The title compound is obtained as a yellow resin from 2.100 g of benzyl (3R,4R,5S)-4-(4-ethoxyphenyl)-3-[4-(3-methoxypropyl)-3,4-dihydro-2H-benzo[1,4]oxazin-6-ylmethoxy]-5-triisopropylsilanyloxypiperidine-1-carboxylate in analogy to method J. Rf = 0.27 (EtOAc-heptane 2:1); Rt = 4.88 (Gradient I).
c) Benzyl (3R,4R,5S)-4-(4-ethoxyphenyl)-3-[4-(3-methoxypropyl)-3,4-dihydro-2H-benzo[1,4]oxazin-6-ylmethoxy]-5-triisopropylsilanyloxypiperidine-1-carboxylate
   0.185 g of sodium hydride (60% dispersion in oil) is added to a solution of 2.500 g of benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 11d) and 0.56 ml of ethyl iodide in 35 ml of N,N-dimethylformamide at 0°C. The reaction mixture is stirred at room temperature for 3 hours, poured into saturated aqueous sodium bicarbonate solution and extracted with tert-butyl methyl ether. The combined organic extracts are washed with brine, dried with sodium sulfate and evaporated. The title compound is obtained as a yellow resin from the residue by flash chromatography (SiO₂ 60F). Rf = 0.23 (EtOAc-heptane 1:2).

### Example 14

### 4-{(3S,4S,5R)-4-(4-Methoxyphenyl)-5-[4-(3-methoxypropyl)-3,4-dihydro-2H-benzo[1,4]oxazin-6-ylmethoxy]piperidin-3-yloxy}-2-methylbutan-2-ol

The title compound is prepared from 0.044 g of 4-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-2-methylbutan-2-ol in analogy to method L.

The starting materials are prepared as follows:
a) 4-[(3S,4S,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-2-methylbutan-2-ol
   0.046 g of methyl 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propionate and 0.109 ml of methylmagnesium bromide solution (3N in diethyl ether) are reacted in analogy to Example 8b. The title compound is obtained as a colourless resin. Rt = 5.11 (Gradient I).
b) Methyl 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propionate
   0.043 g of methyl acrylate is added to a solution of 0.10 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1 b) and 0.013 g of 2,3,4,6,7,8,9,10-octahydropyrimido[1,2-a]azepine (DBU) in 0.5 ml of acetonitrile. After 18 hours at 45°C, 0.043 g of methyl acrylate is again added to the reaction solution. After 24 hours, the reaction mixture is evaporated. The title compound is obtained as a yellowish oil from the residue by flash chromatography (SiO₂ 60F). Rf = 0.47 (EtOAc-heptane 2:1); Rt = 5.23 (Gradient I).

### Example 16

### 3-{(3S,4S,5R)-4-(4-Methoxyphenyl)-5-[4-(3-methoxyproyl)-3,4-dihydro-2H-benzo[1,4]oxazin-6-ylmethoxy]piperidin-3-yloxy}propan-1-ol

The title compound is prepared from 0.088 g of 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)-piperidin-3-yloxy]propan-1-ol in analogy to method L.

The starting materials are prepared as follows:
a) 3-[(3S,4S,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propan-1-ol
   0.389 g of 6-[(3R,4S,5S)-4-(4-methoxyphenyl)-1-(toluene-4-sulfonyl)-5-(3-triisopropylsilanyloxypropoxy)piperidin-3-yloxymethyl]-4-(3-methoxypropyl)-3,4-dihydro-2H-benzo[1,4]oxazine is reacted in analogy to method J. The title compound is obtained as a colourless resin. Rf = 0.36 (EtOAc-heptane 4:1); Rt = 4.80 (Gradient I).
b) 6-[(3R,4S,5S)-4-(4-Methoxyphenyl)-1-(toluene-4-sulfonyl)-5-(3-triisopropylsilanyloxypropoxy)piperidin-3-yloxymethyl]-4-(3-methoxypropyl)-3,4-dihydro-2H-benzo[1,4]oxazine
   0.043 g of sodium hydride (60% dispersion in oil) is added to a solution of 0.50 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b), 0.268 g of (3-bromopropoxy)triisopropylsilane [215650-24-1 and 0.009 g of tetrabutylammonium iodide in 8 ml of tetrahydrofuran. After 15 hours at 50°C, the reaction mixture is diluted at room temperature with 200 ml of tert-butyl methyl ether. The solution is washed successively with 30 ml of aqueous sodium bicarbonate solution, 30 ml of water and 20 ml of brine, dried with sodium sulfate and evaporated. The title compound is obtained as a colourless oil from the residue by flash chromatography (SiO 60F). Rf = 0.31 (EtOAc-heptane 1:2).

### Example 17

### 6-[(3R,4S,5S)-4-(4-Methoxyphenyl)-5-(3-[1,2,4]triazol-1-yl-propoxy)piperidin-3-yloxymethyl]-4-(3-methoxypropyl)-3,4-dihydro-2H-benzo[1,4]oxazine

The title compound is prepared from 0.150 g of 6-[(3R,4S,5S)-4-(4-methoxyphenyl)-1-(toluene-4-sulfonyl)-5-(3-[1,2,4]triazol-1-yl-propoxy)piperidin-3-yloxymethyl]-4-(3-methoxypropyl)-3,4-dihydro-2H-benzo[1,4]oxazine in analogy to method L.

The starting materials are prepared as follows:
a) 6-[(3R,4S,5S)-4-(4-Methoxyphenyl)-1-(toluene-4-sulfonyl)-5-(3-[1,2,4]triazol-1-yl-propoxy)piperidin-3-yloxymethyl]-4-(3-methoxypropyl)-3,4-dihydro-2H-benzo[1,4]oxazine
   0.115g of 1,2,4-triazole sodium salt [41253-21-8] is added to a solution of 0.184 g of 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propyl toluene-4-sulfonate in 2 ml of N,N-dimethylformamide at 0°C. After 15 hours at room temperature, the reaction mixture is diluted with 100 ml of ethyl acetate and washed successively with 15 ml of saturated aqueous sodium bicarbonate solution and 15 ml of brine. The organic phase is dried with sodium sulfate and evaporated. The title compound is obtained as a yellowish resin from the residue by flash chromatography (SiO₂ 60F). Rf = 0.10 (EtOAc-heptane 4:1); Rt = 4.72 (Gradient I).
b) 3-[(3S,4S,5R)-4-(4-Methoxyphenyl)-5-[4-(3-methoxypropyl)-3,4-dihydro-2H-benzo[1,4]oxazin-6-ylmethoxyl-1-(toluene-4-sulfonyl)piperidin-3-yloxy]propyl toluene-4-sulfonate
   The title compound is obtained as a colourless oil from 0.195 g of 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propan-1-ol (Example 16a) in analogy to method H. Rf = 0.22 (EtOAc-heptane 1:1); Rt = 5.68 (Gradient I).

### Example 18

### (R)-1-Methoxy-3-{(3S,4R,5R)-4-[4-(2-methoxyethoxy)phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.200 g of benzyl (3S,4R,5R)-3-((R)-2-hydroxy-3-methoxypropoxy)-4-[4-(2-methoxyethoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-3-((R)-2-hydroxy-3-methoxypropoxy)-4-[4-(2-methoxyethoxy)-phenyl]-5-[4-(3-methoxypropyl)-3,4-dihydro-2H-benzo[1,4]oxazin-6-ylmethoxy]piperidine-1-carboxylate
   The title compound is obtained as a colourless oil from 0.580 g of benzyl (3S,4S,5R)-3-hydroxy-4-[4-(2-methoxyethoxy)phenyl]-5-[4-(3-methoxypropyl)-3,4-dihydro-2H-benzo[1,4]oxazin-6-ylmethoxy]piperidin-1-carboxylate and 0.185 g of S-(+)-glycidyl methyl ether [64491-68-5] in analogy to method M. Rf = 0.15 (EtOAc-heptane 2:1); Rt = 4.70 (Gradient I).
b) Benzyl (3S,4S,5R)-3-hydroxy-4-[4-(2-methoxyethoxy)phenyl]-5-[4-(3-methoxypropyl)-3,4-dihydro-2H-benzo[1,4]oxazin-6-ylmethoxy]piperidine-1-carboxylate
   The title compound is obtained as a colourless oil from 0.584 g of benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 11d) and 0.334 g of 2-methoxyethyl toluene-4-sulfonate [17178-10-8] in analogy to method I. Rf = 0.20 (EtOAc-heptane 2:1); Rt = 4.62 (Gradient I).

The following compounds are prepared in an analogous manner to the process described in Example 18:

### Examples

### 142 (R)-1-Methoxy-3-{(3S,4R,5R)-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidine-1-carboxylic acid benzyl ester (Example 140a)

### 143 (S)-1-Methoxy-3-{(3S,4R,5R)-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidine-1-carboxylic acid benzyl ester (Example 140a) using R-(-)-glycidyl methyl ether [64491-70-9]

### 147 (R)-1-Methoxy-3-{(3S,4R,5R)-4-[4-((R)-4-methoxy-pentyloxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((R)-4-methoxy-pentyloxy)-phenyl]-5-[4-(3-methoxy-propyl)-3,4-dihydro-2H-benzo[1,4]oxazin-6-ylmethoxy]-piperidine-1-carboxylic acid benzyl ester (Example 145a)

### 148 (S)-1-Methoxy-3-{(3S,4R,5R)-4-[4-((R)-4-methoxy-pentyloxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((R)-4-methoxy-pentyloxy)-phenyl]-5-[4-(3-methoxy-propyl)-3,4-dihydro-2H-benzo[1,4]oxazin-6-ylmethoxy]-piperidine-1-carboxylic acid benzyl ester (Example 145a) using R-(-)-glycidyl methyl ether [64491-70-9]

### Example 19

### (R)-3-{(3S,4R,5R)-4-[4-(2-Methoxyethoxy)phenyl]-5-[4-(3-methoxypropyl)-3,4-dihydro-2H-benzo[1,4]oxazin-6-ylmethoxy]piperidin-3-yloxy}propane-1,2-diol

The title compound is prepared from 0.225 g of benzyl (3S,4R,5R)-3-((R)-2,3-dihydroxy-propoxy)-4-(4-methoxyphenyl)-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-3-((R)-2,3-dihydroxypropoxy)-4-(4-methoxyphenyl)-5-[4-(3-methoxy-propyl)-3,4-dihydro-2H-benzo[1,4]oxazin-6-ylmethoxy]piperidine-1-carboxylate
   The title compound is obtained as a yellow oil from 4.600 g of benzyl (3S,4R,5R)-3-[(S)-3-(tert-butyldimethylsilanyloxy)-2-hydroxypropoxy)-4-(4-methoxyphenyl)-5-[4-(3-methoxypropyl)-3,4-dihydro-2H-benzo[1,4]oxazin-6-ylmethoxy]piperidine-1-carboxylate in analogy to method J. Rf = 0.11 (EtOAc-heptane 3:1); Rt = 4.38 (Gradient I).
b) Benzyl (3S,4R,5R)-3-[(S)-3-(tert-butyldimethylsilanyloxy)-2-hydroxypropoxy)-4-(4-methoxyphenyl)-5-[4-(3-methoxyproipyl)-3,4-dihydro-2H-benzo[1,4]oxazin-6-ylmethoxy]piperidine-1-carboxylate
   The title compound is obtained as a brownish oil from 2.210 g of benzyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate and 1.86 ml of tert-butyldimethyl((S)-1-oxiranylmethoxy)silane [123237-62-7] in analogy to method M. Rt = 6.14 (Gradient I).
c) Benzyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate
   The title compound is obtained as a pale yellow oil from 26.59 g of benzyl (3R,4R,5S)-3-hydroxy-4-(4-methoxyphenyl)-5-triisopropylsilanyloxypiperidine-1-carboxylate in analogy to the process described in method D and in Example 1b-c. Rf = 0.25 (EtOAc-heptane 3:1); Rt = 4.69 (Gradient I).
d) Benzyl (3R,4R,5S)-3-hydroxy-4-(4-methoxyphenyl)-5-triisopropylsilanyloxypiperidine-1-carboxylate
   The title compound is obtained as a yellowish resin from 25 g of benzyl (3R,4R,5S)-3-hydroxy-4-(4-hydroxyphenyl)-5-triisopropylsilanyloxypiperidine-1-carboxylate (Example 11 h) in analogy to Example 1 e. Rt = 6.35 (Gradient I).

### Example 20

### (R)-1-Methoxy-3-{(3S,4R,5R)-4-[4-(3-methoxypropoxy)-phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.260 g of benzyl (3S,4R,5R)-3-((R)-2-hydroxy-3-methoxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-3-((R)-2-hydroxy-3-methoxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   The title compound is obtained as a pale yellow resin from 0.580 g of benzyl (3S,4R,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate and 0.181 g of S-(+)-glycidyl methyl ether [64491-68-5] in analogy to method M. Rf = 0.15 (EtOAc-heptane 3:1); Rt = 4.92 (Gradient I).
b) Benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   The title compound is obtained as a yellow oil from 39.10 g of benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-triisopropylsilanyloxypiperidine-1-carboxylate in analogy to method J. Rf = 0.17 (EtOAc-heptane 4:1); Rt = 4.80 (Gradient I).
c) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-triisopropylsilanyloxypiperidine-1-carboxylate
   The title compound is obtained as a yellowish oil from 40.75 g of benzyl (3R,4R,5S)-4-[4-(3-methoxypropoxy)phenyl]-3-[4-(3-methoxypropyl)-3-oxo-3,4-dihydro-2H-benzo[1,4]oxazin-6-ylmethoxy]-5-triisopropylsilanyloxypiperidine-1-carboxylate in analogy to method K. Rf = 0.51 (EtOAc-heptane 1:1).
d) Benzyl (3R,4R,5S)-4-[4-(3-methoxyprolpoxy)phenyl]-3-[4-(3-methoxyprolpyl)-3-oxo-3,4-dihydro-2H-benzo[1,4]oxazin-6-ylmethoxy]-5-triisopropylsilanyloxypiperidine-1-carboxylate
   The title compound is obtained as a yellowish oil from 33.45 g of benzyl (3R,4R,5S)-3-hydroxy-4-[4-(3-methoxypropoxy)phenyl]-5-triisopropylsilanyloxypiperidine-1-carboxylate in analogy to method D. Rf = 0.40 (EtOAc-heptane 1:1); Rt = 7.05 (Gradient I).
e) Benzyl (3R,4R,5S)-3-hydroxy-4-[4-(3-methoxypropoxy)phenyl]-5-triisopropylsilanyloxypiperidine-1-carboxylate
   The title compound is obtained as a colourless oil from 32.00 g of benzyl (3R,4R,5S)-3-hydroxy-4-[4-hydroxyphenyl]-5-triisopropylsilanyloxypiperidine-1-carboxylate (Example 11h) in analogy to method F. Rf = 0.28 (EtOAc-heptane 1:2); Rt = 6.48 (Gradient I).

### Example 21

### 1-Methoxy-3-{(3S,4R,5R)-4-(4-methoxyphenyl-5-[4-(3-methoxypropyl)-3,4-dihydro-2H-benzo[1,4]oxazin-6-ylmethoxy]piperidin-3-yloxv}propan-2-one

The title compound is prepared from 0.123 g of benzyl (3S,4R,5R)-3-(3-methoxy-2-oxopropoxy)-4-(4-methoxyphenyl)-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-3-(3-methoxy-2-oxopropoxy)-4-(4-methoxyphenyl)-5-[4-(3-methoxy-propyl)-3,4-dihydro-2H-benzo[1,4]oxazin-6-ylmethoxy]piperidine-1-carboxylate
   1.00 ml of triethylamine is added dropwise to a solution of 0.820 g of benzyl (3S,4R,5R)-3-((R)-(2-hydroxy-3-methoxypropoxy)-4-(4-methoxyphenyl)-5-[4-(3-methoxypropyl)-3,4-dihydro-2H-benzo[1,4]oxazin-6-ylmethoxy]piperidine-1-carboxylate in 12 ml of dichloromethane-dimethyl sulfoxide 5:1 at 0-5°C. 0.981 g of sulfur trioxid-pyridine complex is added, and the reaction solution is stirred at room temperature for 16 hours. The reaction solution is poured into ice-water, adjusted to pH 2-3 with 1 M aqueous potassium bisulfate solution and extracted with diethyl ether. The combined organic extracts are washed with brine, dried with sodium sulfate and evaporated. The title compound is obtained as a pale yellow oil from the residue by flash chromatography (SiO₂ 60F). Rf = 0.26 (EtOAc-heptane 2:1); Rt = 4.97 (Gradient I).
b) Benzyl (3S,4R,5R)-3-((R)-(2-hydroxy-3-methoxypropoxy)-4-(4-methoxyphenyl)-5-[4-(3-methoxypropyl)-3,4-dihydro-2H-benzo[1,4]oxazin-6-ylmethoxy]piperidine-1-carboxylate
   The title compound is obtained as a colourless oil from 1.570 g of benzyl (3S,4S,5R)-3-hydroxy-4-(4-methoxyphenyl)-5-[4-(3-methoxypropyl)-3,4-dihydro-2H-benzo[1,4]oxazin-6-ylmethoxy]piperidine-1-carboxylate (Example 19c) and 0.181 g of S-(+)-glycidyl methyl ether [64491-68-5] in analogy to method M. Rf = 0.19 (EtOAc-heptane 2:1); Rt = 4.77 (Gradient I).

### Example 22

### 6-[(3R,4R,5S)-5-(2-Methanesulfonylethoxy)-4-(4-methoxyphenyl)piperdin-3-yloxymethyl]-4-(3-methoxypropyl)-3,4-dihydro-2H-benzo[1,4]oxazine

The title compound is prepared from 0.48 g of 6-[(3R,4R,5S)-5-(2-methanesulfonylethoxy)-4-(4-methoxyphenyl)-1-(toluene-4-sulfonyl)piperidin-3-yloxymethyl]-4-(3-methoxypropyl)-3,4-dihydro-2H-benzo[1,4]oxazine in analogy to method L.

The starting material is prepared as follows:
a) 6-[(3R,4R,5S)-5-(2-Methanesulfonylethoxy)-4-(4-methoxyphenyl)-1-(toluene-4-sulfonyl)-piperidin-3-yloxymethyl]-4-(3-methoxypropyl)-3,4-dihydro-2H-benzo[1,4]oxazine
   0.472 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) and 0.834 g of methyl vinyl sulfone [3680-02-2] are reacted in analogy to Example 14b. The title compound is obtained as a yellowish oil. Rf = 0.35 (EtOAc-heptane 2:1); Rt = 4.91 (Gradient I).

### Example 23

### 4-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxymethyl}tetrahydropyran-4-ol

The title compound is prepared from 0.270 g of 4-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxymethyl]tetrahydropyran-4-ol in analogy to method L.

The starting material is prepared as follows:
a) 4-[(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxymethyl]tetrahydropyran-4-ol
   0.04 g of sodium hydride (60% dispersion in oil) is added to a solution of 0.40 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) in 6 ml of 1,3-dimethyl-3,4,5,6-tetrahydro-2(1H)-pyrimidone (DMPU). The mixture is stirred at 60°C for 10 minutes and then a solution of 0.174 g of 1,6-dioxaspiro[2.5]octane [185-72-8] in 2 ml of DMPU is added. After one hour at 60°C, the reaction mixture is cooled at room temperature and diluted with 200 ml of tert-butyl methyl ether. The solution is washed successively with 20 ml of 1 N HCl, with 30 ml unsaturated aqueous sodium bicarbonate solution and 20 ml of brine. The organic phase is dried with sodium sulfate and evaporated. The title compound is obtained as a yellowish resin from the residue by flash chromatography (SiO₂ 60F). Rf = 0.23 (EtOAc-heptane 2:1); Rt = 4.87 (Gradient I).

The following compound is prepared in an analogous manner to the process described in Example 23:

### Example

### 137 6-[(3R,4R,5S)-5-[1-(2-Methoxy-ethoxy)-cyclopentylmethoxy]-4-(4-methoxyphenyl)-piperidin-3-yloxymethyl]-4-(3-methoxy-propyl)-3,4-dihydro-2H-benzo[1,4]oxazine

using 1-iodomethyl-1-(2-methoxy-ethoxy)-cyclopentane

The starting material is prepared as follows:
a) 1-lodomethyl-1-(2-methoxy-ethoxy)-cyclopentane
   To a solution of 25.98 mmol of 2-methoxyethanol [109-86-4] and 23.62 mmol of methylenecyclopentane [1528-30-9] in dry actetinitile is added 25.98 mmol of N-iodosuccinimid in one portion. The reaction mixture is stirred for 20 hours at room temperature under exclusion of light. The reaction mixture is poured into brine, extracted with diethyl ether (2X) and evaporated to a concentrated solution. The title compound is obtained as a yellow oil from the residue by flash chromatography (SiO₂ 60F). Rf = 0.42 (EtOAc-heptane 1:4).

### Example 24

### 2-{(3S,4R,5R)-4-(4-Methoxyphenyl)-5-[4-(3-methoxypropyl)-3,4-dihydro-2H-benzo[1,4]oxazin-6-ylmethoxyl]piperidin-3-yloxy}-N,N-dimethylacetamide

The title compound is prepared from 0.140 g of 2-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-N,N-dimethylacetamide in analogy to method L.

The starting material is prepared as follows:
a) 2-[(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-N,N-dimethylacetamide
   A solution of 0.145 g of methyl [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetate (Example 8c) in 5 ml of dimethylamide (33% in ethanol) is stirred at 50°C for 24 hours and then evaporated to dryness. The title compound is obtained as a yellow oil from the residue. Rt = 4.82 (Gradient I).

### Example 25

### (R,S)-1-Methoxy-3-{(3S,4R,5R)-4-(4-methoxyphenyl-5-[4-(3-methoxypropyl)-3,4-dihydro-2H-benzo[1,4]oxazin-6-ylmethoxy]piperidin-3-yloxy]-2-methylpropan-2-ol

The title compound is prepared from 0.381 g of benzyl (3S,4R,5R)-3-((R,S)-2-hydroxy-3-methoxy-2-methylpropoxy)-4-(4-methoxyphenyl)-5-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3S,4R,5R)-3-((R,S)-2-hydroxy-3-methoxy-2-methylpropoxy)-4-(4-methoxy-phenyl)-5-[4-(3-methoxypropyl)-3,4-dihydro-2H-benzo[1,4]oxazin-6-ylmethoxy]piperidine-1-carboxylate
   0.30 ml of methylmagnesium bromide solution (35% in diethyl ether) is added to a solution of 0.542 g of benzyl (3S,4R,5R)-3-(3-methoxy-2-oxopropoxy)-4-(4-methoxyphenyl)-5-[4-(3-methoxypropyl)-3,4-dihydro-2H-benzo[1,4]oxazin-6-ylmethoxy]piperidine-1-carboxylate (Example 21 a) in 5 ml of dry tetrahydrofuran at room temperature, and the mixture is stirred at room temperature for 2 hours. The reaction mixture is poured into 1 M aqueous potassium bisulfate solution and extracted with tert-butyl methyl ether. The combined organic extracts are washed with brine, dried with sodium sulfate and evaporated. The title compound is obtained as a pale yellow oil from the residue by flash chromatography (SiO₂ 60F). Rf = 0.15 (EtOAc-heptane 2:1); Rt = 5.03 (Gradient I).

### Example 26

### (R)-2-Fluoro-3-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy]propan-1-ol

0.10 ml of 2M aqueous HCl is added to a solution of 0.135 g of benzyl (3S,4R,5R)-3((R)-3-benzyloxy-2-fluoropropoxy)-4-(4-methoxyphenyl)-5-[4-(3-methoxypropyl)-3,4-dihydro-2H-benzo[1,4]oxazin-6-ylmethoxy]piperidine-1-carboxylate in 6 ml of 1:1 tetrahydrofuran-methanol, and hydrogenation is carried out in the presence of 60 mg of 10% Pd/C at 20°C for 6 hours. The reaction mixture is clarified by filtration through Hyflo and the filtrate is evaporated. The title compound is obtained from the residue by flash chromatography (SiO₂ 60F).

The starting materials are prepared as follows:
a) Benzyl (3S,4R,5R)-3((R)-3-benzyloxy-2-fluoropropoxy)-4-(4-methoxyphenyl)-5-[4-(3-methoxypropyl)-3,4-dihydro-2H-benzo[1,4]oxazin-6-ylmethoxy]piperidine-1-carboxylate
   A solution of 0.081 ml of diethylaminosulfur trifluoride in 5 ml of dry dichloromethane is cooled to -78°C. A solution of 0.418 g of benzyl (3S,4R,5R)-3((S)-3-benzyloxy-2-hydroxy-propoxy)-4-(4-methoxyphenyl)-5-[4-(3-methoxypropyl)-3,4-dihydro-2H-benzo[1,4]oxazin-6-ylmethoxy]piperidine-1-carboxylate in 5 ml of dichloromethane is added dropwise at this temperature. The reaction solution is stirred at -78°C for 1 hour and then warmed to room temperature over 3-4 hours. The reaction mixture is poured into a mixture of ice and saturated aqueous sodium bicarbonate solution and then extracted with dichloromethane. The combined organic phases are washed with water, dried with sodium sulfate and evaporated. The title compound is obtained as a pale yellow oil from the residue by flash chromatography (SiO₂ 60F). Rf = 0.29 (EtOAc-heptane 1:1); Rt = 5.70 (Gradient I).
b) Benzyl (3S,4R,5R)-3((S)-3-benzyloxy-2-hydroxypropoxy)-4-(4-methoxyphenyl)-5-[4-(3-methoxypropyl)-3,4-dihydro-2H-benzo[1,4]oxazin-6-ylmethoxy]piperidine-1-carboxylate
   The title compound is obtained as a yellow oil from 0.406 g of benzyl (3S,4S,5R)-3-hydroxy-4-(4-methoxyphenyl)-5-[4-(3-methoxypropyl)-3,4-dihydro-2H-benzo[1,4]oxazin-6-ylmethoxy]-piperidine-1-carboxylate (Example 19c) and 0.257 g of (R)-2-benzyloxymethyloxirane [14618-80-5] in analogy to method M. Rf = 0.38 (EtOAc-heptane 3:1); Rt = 5.33 (Gradient I).

The following compound is prepared in an analogous manner to the process described in Example 26:

### Example

### 27 (S)-2-Fluoro-3-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy]propan-1-ol

### Example 28

### 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-pyrrolidin-1-yl-ethanone

The title compound is prepared from 0.29 g of 2-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)-piperidin-3-yloxy]-1-pyrrolidin-1-yl-ethanone in analogy to method L.

The starting materials are prepared as follows:
a) 2-[(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-1-pyrrolidin-1-yl-ethanone
   A solution of 0.37 g of methyl [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetate and 0.39 g of pyrolidine is heated at 75°C for 2 hours. The solvent is evaporated in vacuo and the title compound is obtained in the form of white crystals from the residue by flash chromatography (SiO₂ 60F). Rf = 0.22 (EtOAc-heptane 3:1); Rt = 4.94 (Gradient I).
b) Methyl [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetate
   0.53 g of sodium hydride (60% dispersion in oil) is added to a solution of 3.2 g of ((3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) in 40 ml of tetrahydrofuran, and the mixture is stirred at room temperature for 1 hour. It is then cooled to -5°C and 2.49 g of methyl bromoacetate are added dropwise over the course of one hour. The reaction mixture is stirred at -5°C for 3 hours and then warmed to room temperature. It is then diluted with tert-butyl methyl ether and poured into 0.5M aqueous HCl. The resulting mixture is extracted three times with tert-butyl methyl ether. The combined organic phases are washed with brine, dried with sodium sulfate and evaporated. The title compound is obtained as a yellow oil from the residue by flash chromatography (SiO₂ 60F). Rf = 0.5 (EtOAc-heptane 2:1); Rt = 5.14 (Gradient I).

The following compounds are prepared in an analogous manner to the process described in Example 28:

### Examples

### 32 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-N-(tetrahydro-pyran-4-yl)acetamide

### 34 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-morpholin-4-ylethanone

### Example 29

### (S)-1-Methoxy-3-{(3S,4R,5R)-4-[4-(3-methoxypropoxy)phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.495 g of benzyl (3S,4R,5R)-3-((S)-2-hydroxy-3-methoxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3S,4R,5R)-3-((S)-2-hydroxy-3-methoxypropoxy)-4-[4-(3-methoxypropoxy)-phenyl]-5-[4-(3-methoxypropyl)-3,4-dihydro-2H-benzo[1,4]oxazin-6-ylmethoxy]piperidine-1-carboxylate
   0.635 g of benzyl (3S,4S,5R)-3-hydroxy-4-[4-(3-methoxypropoxy)phenyl]-5-[4-(3-methoxy-propyl)-3,4-dihydro-2H-benzo[1,4]oxazin-6-ylmethoxy]piperidine-1-carboxylate (Example 20b) and 0.202 g of R-(-)-glycidyl methyl ether [64491-70-9] are reacted in analogy to method M. The title compound is obtained as a yellow oil. Rf = 0.06 (EtOAc-heptane 1:1); Rt = 4.91 (Gradient I).

### Example 30

### 6-[(3R,4S,5S)-4-(4-Methoxyphenyl)-5-(3-morpholin-4-yl-propoxy)piperidin-3-yloxymethyl]-4-(3-methoxypropyl)-3,4-dihydro-2H-benzo[1,4]oxazine

The title compound is prepared from 0.26 g of 6-[(3R,4S,5S)-4-(4-methoxyphenyl)-5-(3-morpholin-4-yl-propoxy)-1-(toluene-4-sulfonyl)piperidin-3-yloxymethyl]-4-(3-methoxypropyl)-3,4-dihydro-2H-benzo[1,4]oxazine in analogy to method L.

The starting materials are prepared as follows:
a) 6-[(3R,4S,5S)-4-(4-Methoxyphenyl)-5-(3-morpholin-4-ylpropoxy)-1-(toluene-4-sulfonyl)-piperidin-3-yloxymethyl]-4-(3-methoxypropyl)-3,4-dihydro-2H-benzo[1,4]oxazine
   0.034 ml of acetic acid, 0.054 ml of morpholine and 0.181 g of sodium triacetoxyborohydride are successively added to a solution of 0.35 g of 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propionaldehyde in 3.5 ml of tetrahydrofuran. After one hour at room temperature, the reaction mixture is poured into 30 ml of ice-water and extracted with tert-butyl methyl ether (2 X 30 ml). The combined organic phases are washed successively with 30 ml of water and 30 ml of brine, dried with sodium sulfate and evaporated. The title compound is obtained as a colourless resin from the residue by flash chromatography (SiO₂ 60F). Rf = 0.33 (dichloromethane-methanol-25% conc. ammonia = 200:10:1); Rt = 4.46 (Gradient I).
b) 3-[(3S,4S,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]propionaldehyde
   0.328 g of pyridine-sulfur trioxide complex is added to a solution of 0.405 g of 3-[(3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-propan-1-ol (Example 16a) and 0.433 ml of triethylamine in 6 ml of 1:5 dimethyl sulfoxide-dichloromethane at 0°C. After 3 hours at 0°C, the reaction mixture is stirred at room temperature. After 1 hour, a further 0.1 g of pyridine-sulfur trioxide complex is added. After a further 1 hour, the reaction mixture is poured into 10 ml of ice-water, adjusted to pH 2.5 with 0.5 ml of 1 N potassium bisulfate solution and extracted with diethyl ether (3 X 20 ml). The combined organic phases are washed successively with 20 ml of water and 20 ml of 5% aqueous sodium bicarbonate solution, dried with sodium sulfate and evaporated. The crude title compound is obtained as a yellowish resin from the residue. Rf = 0.09 (EtOAc-heptane 1:2); Rt = 5.04 (Gradient I).

### Example 31

### 6-[(3R,4R,5S)-4-(4-Methoxyphenyl)-5-(2-morpholin-4-ylethoxy)piperidin-3-yloxymethyl]-4-(3-methoxypropyl)-3,4-dihydro-2H-benzo[1,4]oxazine

The title compound is prepared from 0.11 g of 6-[(3R,4R,5S)-4-(4-methoxyphenyl)-5-(2-morpholin-4-ylethoxy)-1-(toluene-4-sulfonyl)piperidin-3-yloxymethyl]-4-(3-methoxypropyl)-3,4-dihydro-2H-benzo[1,4]oxazine in analogy to method L.

The starting materials are prepared as follows:
a) 6-[(3R,4R,5S)-4-(4-Methoxyphenyl)-5-(2-morpholin-4-ylethoxy)-1-(toluene-4-sulfonyl)-piperidin-3-yloxymethyl]-4-(3-methoxypropyl)-3,4-dihydro-2H-benzo[1,4]oxazine
   0.122 g of [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetaldehyde and 0.0154 g of morpholine are reacted in analogy to Example 30a. The title compound is obtained as a yellowish resin. Rf = 0.38 (dichloromethane-methanol-25% conc. ammonia = 200:10:1); Rt = 4.45 (Gradient I).
b) [(3S,4R,5R)-4-(4-Methoxyphenyl)-5-[4-(3-methoxypropyl)-3,4-dihydro-2H-benzo-[1,4]oxazin-6-ylmethoxy]-1-(toluene-4-sulfonyl)piperidin-3-yloxy]acetaldehyde
   0.135 g of 2-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]ethanol is reacted in analogy to Example 30b. The title compound is obtained as a yellowish resin. Rf = 0.05 (EtOAc-heptane 1:2); Rt = 4.67 (Gradient I).
c) 2-[(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]ethanol
   0.19 g of 6-[(3R,4R,5S)-4-(4-methoxyphenyl)-1-(toluene-4-sulfonyl)-5-(2-triisopropylsilanyloxyethoxy)piperidin-3-yloxymethyl]-4-(3-methoxypropyl)-3,4-dihydro-2H-benzo[1,4]oxazine is reacted in analogy to Example 1 b. The title compound is obtained as a yellowish resin. Rf = 0.31 (EtOAc-heptane 1:2); Rt = 4.77 (Gradient I).
d) 6-[(3R,4R,5S)-4-(4-Methoxyphenyl)-1-(toluene-4-sulfonyl)-5-(2-triisopropylsilanyloxyethoxy)piperidin-3-yloxymethyl]-4-(3-methoxypropyl)-3,4-dihydro-2H-benzo[1,4]oxazine
   0.044 g of sodium hydride (60% dispersion in oil) is added to a solution of 0.50 g of (3S,4S,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-ol (Example 1b) and 0.303 g of (2-iodoethoxy)triisopropylsilane [93550-77-7] in 5 ml of tetrahydrofuran while stirring at room temperature. After 4 hours at 50°C, 0.303 g of (2-iodoethoxy)triisopropylsilane and 0.044 g of sodium hydride (60% dispersion in oil) are again added to the mixture. After 15 hours at 50°C, the reaction mixture is diluted at room temperature with tert-butyl methyl ether. The solution is washed successively with aqueous sodium bicarbonate solution, with water and brine, dried with sodium sulfate and evaporated. The title compound is obtained as a yellowish resin from the residue by flash chromatography (SiO₂ 60F). Rf = 0.25 (EtOAc-heptane 1:2).

### Example 33

### 6-{(3R,4S,5S)-5-(3-Methoxypropoxy)-4-[4-(3-methoxypropoxy)phenyl]piperidin-3-yloxymethyl}-4-(3-methoxypropyl)-3,4-dihydro-2H-benzo[1,4]oxazine

The title compound is obtained from 0.218 g of benzyl (3S,4S,5R)-3-(3-methoxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3S,4S,5R)-3-(3-methoxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   0.090 g of sodium hydride (60% dispersion in oil) is added to a solution of 0.955 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) in 7.0 ml of N,N-dimethylformamide while stirring at 0°C. The reaction mixture is stirred at 0°C for 1 hour. 0.258 g of 1-bromo-3-methoxypropane and 0.023 g of sodium iodide are successively added to the mixture. The reaction mixture is stirred at room temperature for 16 hours and then poured into 50 ml of water and extracted with tert-butyl methyl ether (3 X 50 ml). The organic phases are washed successively with water (2 X 50 ml) and brine (50 ml), dried with sodium sulfate and evaporated. The title compound is obtained as a colourless oil from the residue by flash chromatography (SiO₂ 60F). Rf = 0.23 (EtOAc-heptane 3:1); Rt = 5.45 (Gradient I).

### Example 35

### 3-{(3S,4S,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}propan-1-ol

The title compound is prepared from 0.300 g of benzyl (3S,4S,5R)-3-(3-hydroxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4S,5R)-3-(3-hydroxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   2.95 g of benzyl (3R,4S,5S)-4-[4-(3-methoxypropoxy)phenyl]-3-[4-(3-methoxypropyl)-3,4-dihydro-2H-benzo[1,4]oxazin-6-ylmethoxy]-5-(3-triisopropylsilanyloxypropoxy)piperidine-1-carboxylate are reacted in analogy to method J. The title compound is obtained as a colourless oil. Rf = 0.11 (EtOAc-heptane 2:1); Rt = 4.83 (Gradient I).
b) Benzyl (3R,4S,5S)-4-[4-(3-methoxypropoxy)phenyl]-3-[4-(3-methoxypropyl)-3,4-dihydro-2H-benzo[1,4]oxazin-6-ylmethoxy]-5-(3-triisopropylsilanyloxypropoxy)piperidine-1-carboxylate
   4.0 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) and 2.07 g of (3-bromopropoxy)triisopropylsilane are reacted in analogy to Example 31 d. The title compound is obtained as a colourless oil. Rf = 0.56 (EtOAc-heptane 2:1).

### Example 36

### (R)-1-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}propan-2-ol

The title compound is obtained from 0.340 g of benzyl (3R,4R,5S)-4-[4-(3-methoxypropoxy)-phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate in analogy to method B.

The starting material is prepared as follows:
a) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate
   0.065 g of sodium hydride (60% dispersion in oil) is added to a solution of 0.860 g of benzyl (3S,4S,5R)-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) in 5 ml of tetrahydrofuran. The mixture is stirred at 40°C for 45 minutes. A solution of 0.625 g of (R)-1-oxiranylmethyl toluene-4-sulfonate [113826-06-5] in 3 ml of tetrahydrofuran is added, and the reaction mixture is heated at 50°C for 3 hours. The reaction mixture is poured into saturated aqueous sodium bicarbonate solution, and the mixture is extracted with tert-butyl methyl ether. The combined organic extracts are washed with brine, dried with sodium sulfate and evaporated. The title compound is obtained as a colourless oil from the residue by flash chromatography (SiO₂ 60F). Rf = 0.25 (EtOAc-heptane 2:1); Rt = 5.26 (Gradient I).

The following compounds are prepared in an analogous manner to the process described in Example 36:

### Examples

### 44 (S)-1-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}propan-2-ol

starting from benzyl (3S,4S,5R)-hydroxy-4-[4-(3-methoxypropoxy)phenyl]-5-[4-(3-methoxy-propyl)-3,4-dihydro-2H-benzo[1,4]oxazin-6-ylmethoxy]piperidine-1-carboxylate (Example 20b) and 0.622 g of (S)-1-oxiranylmethyl toluene-4-sulfonate [70987-78-9]

### 140 (R)-1-{(3S,4R,5R)-4-[4-((S)-4-Methoxy-3-methyl-butoxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) (3S,4S,5R)-3-Hydroxy-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidine-1-carboxylic acid benzyl ester
   The title compound is obtained from benzyl (3R,4R,5S)-3-hydroxy-4-(4-hydroxyphenyl)-5-triisopropylsilanyloxypiperidine-1-carboxylate (Example 11 h) in analogy to the process described in Example 20 b, c, d, e using toluene-4-sulfonic acid (S)-4-methoxy-3-methyl-butyl ester (Example 144a). The title compound is identified from the residue on the basis of the Rf by flash chromatography (SiO₂ 60F).

### 141 (S)-1-{(3S,4R,5R)-4-[4-((S)-4-Methoxy-3-methyl-butoxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((S)-4-methoxy-3-methyl-butoxy)-phenyl]-5-[4-(3-methoxy-propyl)-3,4-dihydro-2H-benzo[1,4]oxazin-6-ylmethoxy]-piperidine-1-carboxylic acid benzyl ester (Example 140a) using (S)-1-oxiranymethylester [70987-78-9].

### 145 (R)-1-{(3S,4R,5R)-4-[4-((R)-4-Methoxy-pentyloxy)-phenyl]-5-[4-(3-methoxypropyl)-3,4-dihydro-2H-benzo[1,4]oxazin-6-ylmethoxy]-piperidin-3-yloxy}-propan-2-ol

starting from (3S,4S,5R)-3-hydroxy-4-[4-((R)-4-methoxy-pentyloxy)-phenyl]-5-[4-(3-methoxy-propyl)-3,4-dihydro-2H-benzo[1,4]oxazin-6-ylmethoxy]-piperidine-1-carboxylic acid benzyl ester.

The starting material is prepared as follows:
a) (3S,4S,5R)-3-Hydroxy-4-[4-((R)-4-methoxy-pentyloxy)-phenyl]-5-[4-(3-methoxypropyl)-3,4-dihydro-2H-benzo[1,4]oxazin-6-ylmethoxy]-piperidine-1-carboxylic acid benzyl ester
   The title compound is obtained from benzyl (3R,4R,5S)-3-hydroxy-4-(4-hydroxyphenyl)-5-triisopropylsilanyloxypiperidine-1-carboxylate (Example 11 h) in analogy to the proceedure described in Example 20 b, c, d, e using toluene-4-sulfonic acid (R)-4-methoxy-pentyl ester (Example 149a). The title compound is identified from the residue on the basis of the Rf by flash chromatography (SiO₂ 60F).

### 146 (S)-1-{(3S,4R,5R)-4-[4-((R)-4-Methoxy-3-methyl-butoxy)-phenyl]-5-[4-(3-methoxy-propyl)-3,4-dihydro-2H-benzo[1,4]oxazin-6-ylmethoxy]-piperidin-3-yloxy}-propan-2-ol

starting from (3S,4S,5R)-3-hydroxy-4-[4-((R)-4-methoxy-pentyloxy)-phenyl]-5-[4-(3-methoxy-propyl)-3,4-dihydro-2H-benzo[1,4]oxazin-6-ylmethoxy]-piperidine-1-carboxylic acid benzyl ester (Example 145a) using (S)-1-oxiranymethylester [70987-78-9].

### Example 37

### (3-{(3S,4S,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}propyl)dimethylamine

The title compound is prepared from 0.190 g of benzyl (3S,4S,5R)-3-(3-dimethylamino-propoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4S,5R)-3-(3-dimethylaminopropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   0.048 ml of acetic acid, 0.08 g of dimethylamine (in 0.5 ml of tetrahydrofuran) and 0.181 g of sodium triacetoxyborohydride are successively added to a solution of 0.530 g of benzyl (3R,4S,5S)-4-[4-(3-methoxypropoxy)phenyl]-3-[4-(3-methoxypropyl)-3,4-dihydro-2H-benzo[1,4]oxazin-6-ylmethoxy]-5-(3-oxopropoxy)piperidine-1-carboxylate in 5.3 ml of ethyl acetate at room temperature. The reaction mixture is stirred at room temperature for 2 hours and then poured into 1 M sodium bicarbonate solution (30 ml), and extracted with tert-butyl methyl ether (2 X 30 ml). The organic phases are washed successively with water (30 ml) and brine (30 ml), dried with sodium sulfate and evaporated. The title compound is obtained as a colourless oil from the residue by flash chromatography (SiO₂ 60F). Rf = 0.22 (dichloromethane-methanol-25% conc. ammonia = 200:20:1); Rt = 4.50 (Gradient I).
b) Benzyl (3R,4S,5S)-4-[4-(3-methoxypropoxy)phenyl]-3-[4-(3-methoxypropyl)-3,4-dihydro-2H-benzo[1,4]oxazin-6-ylmethoxy]-5-(3-oxopropoxy)piperidine-1-carboxylate
   0.46 g of pyridine-sulfur trioxide is added in portions to a solution of 0.600 g of benzyl (3S,4S,5R)-3-(3-hydroxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 35a) in 9 ml of 1:5 dimethyl sulfoxide-dichloromethane at 0°C. The reaction mixture is stirred at 0°C for 30 minutes and then poured into ice-water (10 ml). 1 M potassium bisulfite solution (0.5 ml) is added to the mixture, which is then extracted with diethyl ether (3 X 20 ml). The organic phases are washed successively with water (30 ml) and 0.5M sodium bicarbonate solution (20 ml), dried with sodium sulfate and evaporated. The crude title compound is obtained as a yellowish oil. Rt = 5.14 (Gradient I).

### Example 38

### 6-[(3R,4S,5S)-4-r4-(3-Methoxypropoxy)phenyl]-5-(3-[1,2,4]triazol-1-ylpropoxy)piperidin-3-yloxymethyl]-4-(3-methoxypropyl)-3,4-dihydro-2H-benzo[1,4]oxazine

The title compound is prepared from 0.390 g of benzyl (3R,4S,5S)-4-[4-(3-methoxypropoxy)-phenyl]-3-[4-(3-methoxypropyl)-3,4-dihydro-2H-benzo[1,4]oxazin-6-ylmethoxy]-5-(3-[1,2,4]triazol-1-ylpropoxy)piperidine-1-carboxylate in analogy to method B.

The starting materials are prepared as follows:
a) Benzyl 3R,4S,5S)-4-[4-(3-methoxypropoxy)phenyl]-3-[4-(3-methoxypropyl)-3,4-dihydro-2H-benzo[1,4]oxazin-6-ylmethoxy]-5-(3-[1,2,4]triazol-1-yl-propoxy)piperidine-1-carboxylate
   0.305 g of 1,2,4-triazole sodium salt [41253-21-8] is added to a solution of 0.510 g of benzyl (3R,4S,5S)-4-[4-(3-methoxypropoxy)phenyl]-3-[4-(3-methoxypropyl)-3,4-dihydro-2H-benzo[1,4]oxazin-6-ylmethoxy]-5-[3-(toluene-4-sulfonyloxy)propoxy]piperidine-1-carboxylate in 6 ml of N,N-dimethylformamide at 0°C, and the mixture is stirred at room temperature for 3 hours. The reaction mixture is poured into ice-water and extracted with tert-butyl methyl ether. The organic phases are washed with water and brine, dried with sodium sulfate and evaporated. The title compound is obtained as a colourless oil from the residue by flash chromatography (SiO₂ 60F). Rf = 0.63 (dichloromethane-methanol-25% conc. ammonia = 200:20:1). Rt = 4.80 (Gradient I).
b) Benzyl (3R,4S,5S)-4-[4-(3-methoxypropoxy)phenyl]-3-[4-(3-methoxypropyl)-3,4-dihydro-2H-benzo[1,4]oxazin-6-ylmethoxyl-5-[3-(toluene-4-sulfonyloxy)propoxylpiperidine-1-carboxylate
   0.500 g of benzyl (3S,4S,5R)-3-(3-hydroxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 35a) is reacted in analogy to method H. The title compound is obtained as a yellowish oil. Rf = 0.16 (EtOAc-heptane 1:1). Rt = 5.78 (Gradient I).

### Example 39

### N,N-Diethyl-2-{(3S,4R,5R)-4-[4-(3-methoxypropoxy)phenyl]-5-[4-(3-methoxypropyl)-3,4-dihydro-2H-benzo[1,4]oxazin-6-ylmethoxy]piperidin-3-yloxy}acetamide

The title compound is prepared from 0.44 g of benzyl (3S,4R,5R)-3-diethylcarbamoylmethoxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-3-diethylcarbamoylmethoxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   0.53 ml of propanephosphonic anhydride [68957-94-8, T3P] (50% in ethyl acetate) is added to a solution of 0.517 g of benzyl (3S,4R,5R)-3-carboxymethoxy-4-[4-(3-methoxypropoxy)-phenyl]-5-[4-(3-methoxypropyl)-3,4-dihydro-2H-benzo[1,4]oxazin-6-ylmethoxy]piperidine-1-carboxylate, 0.066 g of diethylamine and 0.52 ml of triethylamine in 8 ml of dichloromethane at 0°C, and the mixture is stirred at room temperature for 16 hours. The reaction mixture is diluted with dichloromethane, and 0.1 M aqueous HCl is added. The phases are separated and the aqueous phase is extracted twice more with dichloromethane. The combined organic phases are washed with brine, dried with sodium sulfate and evaporated. The title compound is obtained as yellow oil from the residue by flash chromatography (SiO₂ 60F). Rf = 0.2 (EtOAc-heptane 5:1); Rt = 5.20 (Gradient I).
b) Benzyl (3S,4R,5R)-3-carboxymethoxy-4-[4-(3-methoxylpropoxy)phenyl]-5-[4-(3-methoxy-propyl)-3,4-dihydro-2H-benzo[1,4]oxazin-6-ylmethoxy]piperidine-1-carboxylate
   8 ml of a 1.5M aqueous lithium hydroxide solution are added to a solution of 1.6 g of benzyl (3S,4R,5R)-3-methoxycarbonylmethoxy-4-[4-(3-methoxypropoxy)phenyl]-5-[4-(3-methoxy-propyl)-3,4-dihydro-2H-benzo[1,4]oxazin-6-ylmethoxy]piperidine-1-carboxylate in 6 ml of tetrahydrofuran and the mixture is stirred at room temperature for 2 hours. The reaction mixture is adjusted to pH 2 with 2M HCl. The resulting mixture is extracted twice with 150 ml of ethyl acetate each time. The combined organic phases are washed with brine, dried with sodium sulfate and evaporated. The title compound is obtained as a colourless oil and employed without further purification in the next stage. Rt = 4.78 (Gradient I).
c) Benzyl (3S,4R,5R)-3-methoxycarbonylmethoxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   0.29 g of sodium hydride (60% dispersion in oil) is added to a solution of 3.5 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) and the mixture is stirred at room temperature for 1 hour. It is then cooled to -5°C, and 1.30 g of methyl bromoacetate are added dropwise over the course of one hour. The reaction mixture is stirred at -5°C for 3 hours and then warmed to room temperature. It is then diluted with tert-butyl methyl ether and poured into 0.5M HCl. The resulting mixture is extracted 3 times with tert-butyl methyl ether. The combined organic phases are washed with brine, dried with sodium sulfate and evaporated. The title compound is obtained as a yellow oil from the residue by flash chromatography (SiO₂ 60F). Rf = 0.22 (EtOAc-heptane 2:1); Rt = 5.26 (Gradient I).

The following compounds are prepared in an analogous manner to the process described in Example 39:

### Examples

### 40 N-Ethyl-2-{(3S,4R,5R)-4-[4-(3-methoxypropoxy)phenyl]-5-[4-(3-methoxypropyl)-3,4-dihydro-2H-benzo[1,4]oxazin-6-ylmethoxy]piperidin-3-yloxy}-N-methylacetamide

### 41 2-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}-N-methyl-N-propylacetamide

### 46 2-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}-N-propylacetamide

### 47 2-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}-1-pyrrolidin-1-ylethanone

### 110 N-Ethyl-2-{(3S,4R,5R)-4-[4-(4-methoxybutoxy)phenyl]-5-[4-(3-methoxypropyl)-3,4-dihydro-2H-benzo[1,4]oxazin-6-ylmethoxy]piperidin-3-yloxy}-N-methylacetamide

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### 111 N,N-Diethyl-2-{(3S,4R,5R)-4-[4-(4-methoxybutoxy)phenyl]-5-[4-(3-methoxypropyl)-3,4-dihydro-2H-benzo[1,4]oxazin-6-ylmethoxy]piperidin-3-yloxy}acetamide

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### 112 2-{(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}-1-pyrrolidin-1-yl-ethanone

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### Example 42

### 6-[(3R,4R,5S)-4-[4-(3-Methoxypropoxy)phenyl]-5-(3-methyl-3H-imidazol-4-yl-methoxy)-piperidin-3-yloxymethyl]-4-(3-methoxypropyl)-3,4-dihydro-2H-benzo[1,4]oxazine

The title compound is obtained from 0.199 g of benzyl (3R,4R,5S)-4-[4-(3-methoxy-propoxy)-phenyl]-3-[4-(3-methoxypropyl)-3,4-dihydro-2H-benzo[1,4]oxazin-6-yl-methoxy]-5-(3-methyl-3H-imidazol-4-ylmethoxy)piperidine-1-carboxylate in analogy to method B.

The starting material is prepared as follows:
a) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5(3-methyl-3H-imidazol-4-ylmethoxy)piperidine-1-carboxylate
   0.123 g of sodium hydride (60% dispersion in oil) is added to a solution of 0.778 g of benzyl (3S,4S,5R)-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) and 0.269 g of 5-chloromethyl-1-methyl-1 H-imidazole hydrochloride [90773-41-4] in 5 ml of N,N-dimethylformamide at 0°C. 0.046 g of tetrabutylammonium iodide is added, and the reaction mixture is stirred at room temperature for 18 hours. The reaction mixture is poured into saturated aqueous sodium bicarbonate solution and extracted with tert-butyl methyl ether. The combined organic extracts are washed with brine, dried with sodium sulfate and evaporated. The title compound is obtained as a yellowish oil from the residue by flash chromatography (SiO₂ 60F). Rf = 0.20 (dichloromethane-methanol 95:5); Rt = 4.51 (Gradient I).

### Example 43

### 6-[(3R,4R,5S)-4-[4-(3-Methoxypropoxy)phenyl]-5-(2-[1,2,4]triazol-a-yl-ethoxy)piperidin-3-yloxymethyl]-4-(3-methoxypropyl)-3,4-dihydro-2H-benzo[1,4]oxazine

The title compound is prepared from 1.210 g of benzyl (3R,4R,5S)-4-[4-(3-methoxypropoxy)-phenyl]-3-[4-(3-methoxypropyl)-3,4-dihydro-2H-benzo[1,4]oxazin-6-ylmethoxy]-5-(2-triisopropylsilanyloxyethoxy)piperidine-1-carboxylate in analogy to the process described in Example 38.

The starting materials are prepared as follows:
a) Benzyl (3S,4R,5R)-3-(2-hydroxyethoxy)-4-[4-(3-methoxypropoxy)phenyl]-5-[4-(3-methoxypropyl)-3,4-dihydro-2H-benzo[1,4]oxazin-6-ylmethoxylpiperidine-1-carboxylate
   The title compound is obtained as a pale yellow resin from benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-(2-trisopropylsilanyloxyethoxy)piperidine-1-carboxylate in analogy to method J. Rf = 0.14 (EtOAc-heptane 3:1); Rt = 4.87 (Gradient I).
b) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-(2-trisopropylsilanyloxyethoxy)piperidine-1-carboxylate
   0.164 g of sodium hydride (60% dispersion in oil) is added to a solution of 2.000 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) in 15 ml of dry N,N-dimethylformamide. The reaction mixture is stirred at room temperature for 10 minutes and 2.276 g of (2-iodoethoxy)triisopropylsilane [93550-77-7] are added. The reaction mixture is stirred at room temperature for 18 hours, poured into saturated aqueous sodium bicarbonate solution and extracted with tert-butyl methyl ether. The combined organic phases are washed with brine, dried with sodium sulfate and evaporated. The title compound is obtained as an orange oil from the residue by flash chromatography (SiO₂ 60F). Rf = 0.61 (EtOAc-heptane 2:1).

### Example 45

### 4-(2-{(3S,4S,5R)-4-(4-Methoxyphenyl)-5-[4-(3-methoxypropyl)-3,4-dihydro-2H-benzo[1,4]oxazin-6-ylmethoxy]piperidin-3-yloxy}ethyl)tetrahydropyran-4-ol

0.0594 g of lithium aluminium hydride is added to a solution of 0.288 g of 6-[(3R,4R,5S)-5-(1,6-dioxaspiro[2.5]oct-2-ylmethoxy)-4-(4-methoxyphenyl)piperidin-3-yloxymethyl]-4-(3-methoxypropyl)-3,4-dihydro-2H-benzo[1,4]oxazine in 28 ml of diethyl ether. After 45 minutes at room temperature, the reaction mixture is diluted with 100 ml of tert-butyl methyl ether and cautiously quenched with 30 ml of 0.5N NaOH. The aqueous phase is again extracted with 100 ml of tert-butyl methyl ether. The combined organic phases are dried with sodium sulfate and evaporated. The title compound is obtained from the residue by flash chromatography (SiO₂ 60F).

The starting materials are prepared as follows:
a) 6-[(3R,4R,5S)-5-(1,6-Dioxasipiro[2,5]oct-2-ylmethoxy)-4-(4-methoxyphenyl)piperidin-3-yloxymethyl]-4-(3-methoxypropyl)-3,4-dihydro-2H-benzo[1,4]oxazine
   0.387 g of benzyl (3S,4R,5R)-3-(1,6-dioxaspiro[2.5]oct-2-ylmethoxy)-4-(4-methoxyphenyl)-5-[4-(3-methoxypropyl)-3,4-dihydro-2H-benzo[1,4]oxazin-6-ylmethoxy]piperidine-1-carboxylate are reacted in analogy to method B. The title compound is obtained as a brown oil. Rt = 3.48 (Gradient I).
b) Benzyl (3S,4R,5R)-3-(1,6-dioxaspiro[2.5]oct-2-ylmethoxy)-4-(4-methoxyphenyl)-5-[4-(3-methoxypropyl)-3,4-dihydro-2H-benzo[1,4]oxazin-6-ylmethoxy]piperidine-1-carboxylate
   0.548 g of benzyl (3S,4R,5R)-3-(1,6-dioxaspiro[2.5]oct-2-ylmethoxy)-4-(4-methoxyphenyl)-5-[4-(3-methoxypropyl)-3-oxo-3,4-dihydro-2H-benzo[1,4]oxazin-6-yl-methoxy]piperidine-1-carboxylate is reacted in analogy to method K. The title compound is obtained as a yellowish oil. Rf = 0.33 (EtOAc-heptane 2:1); Rt = 5.11 (Gradient I).
c) Benzyl (3S,4R,5R)-3-(1,6-dioxaspiro[2.5]oct-2-ylmethoxy)-4-(4-methoxyphenyl)-5-[4-(3-methoxypropyl)-3-oxo-3,4-dihydro-2H-benzo[1,4]oxazin-6-ylmethoxy]piperidine-1-carboxylate
   0.236 g of 3-chloroperoxybenzoic acid (70% mCPBA) is added to a solution of 0.61 g of benzyl (3R,4S,5S)-4-(4-methoxyphenyl)-3-[4-(3-methoxypropyl)-3-oxo-3,4-dihydro-2H-benzo[1,4]oxazin-6-ylmethoxy]-5-[2-(tetrahydropyran-4-ylidene)ethoxy]piperidine-1-carboxylate in 5 ml of dichloromethane. After 90 minutes at room temperature, the reaction mixture is diluted with 200 ml of tert-butyl methyl ether. The mixture is washed successively with 15 ml of saturated aqueous sodium carbonate solution, 15 ml of saturated aqueous sodium bicarbonate solution, 30 ml of water and 20 ml of brine. The organic phase is dried with sodium sulfate and evaporated. The title compound is obtained as a yellow oil from the residue by flash chromatography (SiO₂ 60F). Rf = 0.18 (EtOAc-heptane 2:1); Rt = 4.78 (Gradient I).
d) Benzyl (3R,4S,5S)-4-(4-methoxyphenyl)-3-[4-(3-methoxypropyl)-3-oxo-3,4-dihydro-2H-benzo[1,4]oxazin-6-ylmethoxyl-5-[2-(tetrahydropyran-4-ylidene)ethoxy]piperidine-1-carboxylate
   0.057 g of sodium hydride (60% dispersion in oil) is added to a stirred solution of 0.56 g of benzyl (3S,4S,5R)-3-hydroxy-4-(4-methoxyphenyl)-5-[4-(3-methoxypropyl)-3-oxo-3,4-dihydro-2H-benzo[1,4]oxazin-6-ylmethoxy]piperidine-1-carboxylate and 0.546 g of 4-(2-bromoethylidene)tetrahydropyran [21378-20-1] in 5 ml of tetrahydrofuran. After 2.5 hours at room temperature, the reaction mixture is diluted with 200 ml of tert-butyl methyl ether and washed successively with 15 ml of saturated aqueous sodium bicarbonate solution, 10 ml of water and 10 ml of brine. The organic phase is dried with sodium sulfate and evaporated. The title compound is obtained as a yellow oil from the residue by flash chromatography (SiO₂ 60F). Rf = 0.20 (EtOAc-heptane 2:1); Rt = 5.10 (Gradient I).
e) Benzyl (3S,4S,5R)-3-hydroxy-4-(4-methoxyphenyl)-5-[4-(3-methoxypropyl)-3-oxo-3,4-dihydro-2H-benzo[1,4]oxazin-6-ylmethoxy]piperidine-1-carboxylate
   1.0 g of benzyl (3R,4R,5S)-4-(4-methoxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted in analogy to Example 1 b. The title compound is obtained as a colourless resin. Rf = 0.29 (EtOAc-heptane 2:1); Rt = 4.36 (Gradient I).
f) Benzyl (3R,4R,5S)-4-(4-methoxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate
   26.59 g of benzyl (3R,4R,5S)-3-hydroxy-4-(4-methoxyphenyl)-5-triisopropylsilanyl-oxy-piperidine-1-carboxylate (Example 19d) and 17.09 g of 6-chloromethyl-4-(3-methoxypropyl)-4H-benzo[1,4]oxazin-3-one are reacted in analogy to method D. The title compound is obtained as a yellowish resin. Rf = 0.18 (EtOAc-heptane 1:2); Rt = 6.67 (Gradient I).

### Example 48

### (2-{(3S,4R,5R)-4-[4-(3-Methoxypropoxy)phenyl-5-[4-(3-methoxypropyl)-3,4-dihydro-2H-benzo[1,4]oxazin-6-ylmethoxy]piperidin-3-yloxy}ethyl)-2-dimethylamine

0.100 g of 4Å molecular sieve are added to a solution of 0.129 g of benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-(2-oxoethoxy)piperidine-1-carboxylate in 1.5 ml of tetrahydrofuran. A solution of 0.030 g of dimethylamine in 1 ml of tetrahydrofuran is added dropwise, and the reaction mixture is stirred at 20°C for 1 hour. The solid is filtered off through Hyflo and the filter cake is washed with tetrahydrofuran. The filtrate is degassed with argon for 15 minutes and, after addition of 0.025 g of 10% Pd/C, hydrogenated under atmospheric pressure at 20°C for 5 hours. The catalyst is filtered off and the filtrate is mixed with a further 0.025 g of Pd/C and 0.155 ml of 2M HCl and hydrogenated under atmospheric pressure at 20°C for 12 hours. The catalyst is filtered off through Hyflo and the filtrate is evaporated. The title compound is obtained as a brown resin from the residue by flash chromatography (SiO₂ 60F).

The starting material is prepared as follows:
a) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-(2-oxoethoxy)piperidine-1-carboxylate
   The title compound is prepared from 1.121 g of benzyl (3R,4R,5S)-4-[4-(3-methoxypropoxy)-phenyl]-3-[4-(3-methoxypropyl)-3,4-dihydro-2H-benzo[1,4]oxazin-6-ylmethoxy]-5-(2-triisopropylsilanyloxyethoxy)piperidine-1-carboxylate (Example 43a) in analogy to the process described in Example 37a-b. The title compound is obtained as a brown resin from the residue by flash chromatography (SiO₂ 60F). Rf = 0.18 (EtOAc-heptane 3:1); Rt = 4.74 (Gradient I).

### Example 49

### 6-{(3R,4S,5S)-4-(4-Methoxyphenyl)-5-[2-(4-methoxytetrahydropyran-4-yl)ethoxy]piperidin-3-yloxymethyl}-4-(3-methoxypropyl)-3,4-dihydro-2H-benzo[1,4]oxazine

The title compound is prepared from 0.169 g of benzyl (3R,4S,5S)-4-(4-methoxyphenyl)-3-[4-(3-methoxypropyl)-3,4-dihydro-2H-benzo[1,4]oxazin-6-ylmethoxy]-5-[2-(4-methoxy-tetrahydropyran-4-yl)ethoxy]piperidine-1-carboxylate in analogy to method B.

The starting materials are prepared as follows:
a) Benzyl (3R,4S,5S)-4-(4-methoxyphenyl)-3-[4-(3-methoxypropyl)-3,4-dihydro-2H-benzo[1,4]oxazin-6-ylmethoxy]-5-[2-(4-methoxytetrahydropyran-4-yl)ethoxy]piperidine-1-carboxylate
   0.014 g of sodium hydride (60% dispersion in oil) is added to a solution of 0.166 g of benzyl (3S,4S,5R)-3-[2-(4-hydroxytetrahydropyran-4-yl)ethoxy]-4-(4-methoxyphenyl)-5-[4-(3-methoxypropyl)-3,4-dihydro-2H-benzo[1,4]oxazin-6-ylmethoxy]piperidine-1-carboxylate and 0.074 ml of methyl iodide in 2.5 ml of 4:1 tetrahydofuran-N,N-dimethylformamide. After 3 hours at room temperature, a further 0.014 g of sodium hydride (60% dispersion in oil) and 0.074 ml of methyl iodide are added. After 14 hours at room temperature, the reaction mixture is diluted with tert-butyl methyl ether and washed with saturated aqueous sodium bicarbonate solution. The aqueous phase is extracted with tert-butyl methyl ether. The combined organic phases are washed with water and brine, dried with sodium sulfate and evaporated. The crude title compound is obtained as a cloudy oil from the residue. Rf = 0.75 (EtOAc); Rt = 5.31 (Gradient I).
b) Benzyl (3S,4S,5R)-3-[2-(4-hydroxytetrahvdropyran-4-yl)ethoxy]-4-(4-methoxyphenyl)-5-[4-(3-methoxypropyl)-3,4-dihydro-2H-benzo[1,4]oxazin-6-ylmethoxy]piperidine-1-carboxylate
   0.135 g of 4-(2-{(3S,4S,5R)-4-(4-methoxyphenyl)-5-[4-(3-methoxypropyl)-3,4-dihydro-2H-benzo[1,4]oxazin-6-ylmethoxy]piperidin-3-yloxy}ethyl)tetrahydropyran-4-ol (Example 45) is reacted in analogy to Example 11 h. The title compound is obtained as a brown oil. Rf = 0.43 (EtOAc); Rt = 4.87 (Gradient I).

### Example 50

### 6-[(3R,4R,5S)-4-[4-(3-Methoxypropoxy)phenyl]-5-(2-morpholin-4-ylethoxy)piperidin-3-yloxymethyl]-4-(3-methoxypropyl)-3,4-dihydro-2H-benzo[1,4]oxazine

The title compound is prepared from 0.191 g of benzyl (3R,4R,5S)-4-[4-(3-methoxy-propoxy)-phenyl]-3-[4-(3-methoxypropyl)-3,4-dihydro-2H-benzo[1,4]oxazin-6-yl-methoxy]-5-(2-morpholin-4-yl-ethoxy)piperidine-1-carboxylate in analogy to method B.

The starting materials are prepared as follows:
a) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxyl-5-(2-morpholin-4-ylethoxy)piperidine-1-carboxylate
   0.279 g of benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-(2-morpholin-4-yl-2-oxoethoxy)piperidine-1-carboxylate is reacted in analogy to method K. The methanolysis is carried out with 7 ml of methanol at 65°C for 24 hours. The title compound is obtained as a colourless oil. Rt = 4.46 (Gradient I).
b) Benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-(2-morpholin-4-yl-2-oxoethoxy)piperidine-1-carboxylate
   0.276 g of benzyl (3S,4R,5R)-3-carboxymethoxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 39b) and 0.045 ml of morpholine are reacted in analogy to Example 39a. The title compound is obtained as a yellowish oil. Rt = 4.87 (Gradient I).

The following compound is prepared in an analogous manner to the process described in Example 50:

### Example

### 54 6-[(3R,4R,5S)-4-[4-(3-Methoxypropoxy)phenyl]-5-(2-pyrrolidin-1-yl-ethoxy)piperidin-3-yloxymethyl]-4-(3-methoxypropyl)-3,4-dihydro-2H-benzo[1,4]oxazine

### Example 51

### (3S,4S,5R)-4-[4-(3-Methoxypropoxy)phenyl]-5-[4-(3-methoxypropyl)-3,4-dihydro-2H-benzo[1.4]oxazin-6-ylmethoxyl]piperidin-3-ol

The title compound is prepared from 0.360 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) in analogy to method B.

### Example 52

### 6-{(3R,4S,5S)-4-[4-(3-Methoxypropoxy)phenyl]-5-propoxypiperidin-3-yloxymethyl}-4-(3-methoxypropyl)-3,4-dihydro-2H-benzo[1,4]oxazine

The title compound is prepared from 0.255 g of benzyl (3S,4S,5R)-3-allyloxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3S,4S,5R)-3-allyloxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   The title compound is obtained as a yellow resin from 0.400 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) and 0.11 ml of allyl bromide in analogy to method D. Rf = 0.13 (EtOAc-heptane 1:1); Rt = 5.64 (Gradient I).

### Example 53

### 2-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}-N,N-dimethylacetamide

The title compound is prepared from 0.262 g of benzyl (3S,4R,5R)-3-dimethylcarbamoylmethoxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3S,4R,5R)-3-dimethylcarbamoylmethoxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   A solution of 0.266 g of benzyl (3S,4R,5R)-3-methoxycarbonylmethoxy-4-[4-(3-methoxy-propoxy)phenyl]-5-[4-(3-methoxypropyl)-3,4-dihydro-2H-benzo[1,4]oxazin-6-ylmethoxy]-piperidine-1-carboxylate (Example 39c) in 14 ml of dimethylamine (33% in ethanol) is stirred at 60°C for 24 hours. The reaction mixture is evaporated. The crude title compound is obtained as a yellow oil from the residue. Rt = 4.88 (Gradient I).

### Example 55

### 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-((S)-3-methylmorpholin-4-yl)ethanone

The title compound is prepared from 0.065 g of 2-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)-piperidin-3-yloxy]-1-((S)-3-methylmorpholin-4-yl)ethanone in analogy to method L.

The starting materials are prepared as follows:
a) 2-[(3S,4R,5R)-4-(4-Methoxyphenyl)-5-[4-(3-methoxylpropyl)-3,4-dihydro-2H-benzo[1,4]oxazin-6-ylmethoxy]-1-(toluene-4-sulfonyl)piperidin-3-yloxy]-1-((S)-3-methyl-morpholin-4-yl)ethanone
   0.389 ml of propanephosphonic anhydride [68957-94-8, T3P] (50% in ethyl acetate) is added to a solution of 0.40 g of [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetic acid, 0.0671 g of (S)-3-methylmorpholine [350595-57-2] and 0.385 ml of triethylamine in 8 ml of dichloromethane at 0°C, and the mixture is stirred at room temperature for 3 hours. The reaction mixture is diluted with dichloromethane, and 0.2M HCl is added. The phases are separated and the aqueous phase is extracted twice more with dichloromethane. The combined organic phases are washed with brine, dried with sodium sulfate and evaporated. The title compound is obtained as a yellow oil from the residue by flash chromatography (SiO₂ 60F). Rf = 0.53 (EtOAc); Rt = 4.93 (Gradient I).
b) [(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetic acid
   5 ml of a 1.5M aqueous lithium hydroxide solution are added to a solution of 0.75 g of methyl [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetate (Example 28b) in 5 ml of tetrahydrofuran, and the mixture is stirred at room temperature for 30 minutes. The reaction mixture is adjusted to pH 2 with 1 M HCl. The resulting mixture is extracted twice with 80 ml of ethyl acetate each time. The combined organic phases are washed with brine, dried with sodium sulfate and evaporated. The title compound is obtained as a yellow oil and is used without further purification in the next stage. Rf = 0.15 (EtOAc); Rt = 4.70 (Gradient I).

The following compounds are prepared in an analogous manner to the process described in Example 55:

### Examples

### 56 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-((S)-2-methylpiperidin-1-yl)-ethanone

### 57 1-((3S,5S)-3,5-Dimethylmorpholin-4-yl)-2-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}-ethanone

### 58 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-((R)-3-methylmorpholin-4-yl)-ethanone

### 61 1-((3S,5R)-3,5-Dimethylmorpholin-4-yl)-2-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}-ethanone

### 75 1-((2S,6R)-2,6-Dimethylpiperidin-1-yl)-2-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}-ethanone

### 79 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-((R)-2-methylpiperidin-1-yl)-ethanone

### 99 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-((R)-2-methylpyrrolidin-1-yl)-ethanone

### 100 N,N-Diisopropyl-2-{(3S,4R,5R)-4-(4-methoxyphenyl)-5-[4-(3-methoxypropyl)-3,4-dihydro-2H-benzo[1,4]oxazin-6-ylmethoxy]piperidin-3-yloxy}acetamide

### 101 2-{(3S,4R,5R)-4-(4-Methoxyphenyl)-5-[4-(3-methoxypropyl)-3,4-dihydro-2H-benzo[1,4]oxazin-6-ylmethoxy]piperidin-3-yloxy}-1-piperidin-1-ylethanone

### Example 59

### 6-{(3R,4R,5S)-4-(4-Methoxyphenyl)-5-[2-((S)-3-methylmorpholin-4-yl)ethoxy]piperidin-3-yloxymethyl}-4-(3-methoxypropyl)-3,4-dihydro-2H-benzo[1,4]oxazine

The title compound is prepared from 0.439 g of 6-[(3R,4R,5S)-4-(4-methoxyphenyl)-5-[2-((S)-3-methylmorpholin-4-yl)ethoxy]-1-(toluene-4-sulfonyl)piperidin-3-yloxymethyl]-4-(3-methoxy-propyl)-3,4-dihydro-2H-benzo[1,4]oxazine in analogy to method L.

The starting materials are prepared as follows:
a) 6-{(3R,4R,5S)-4-(4-Methoxyphenyl)-5-[2-((S)-3-methylmorpholin-4-yl)ethoxyl-1-(toluene-4-sulfonyl)piperidin-3-yloxymethyl]-4-(3-methoxypropyl)-3,4-dihydro-2H-benzo[1,4]oxazine
   2 ml of borane-tetrahydrofuran complex solution (1 M in tetrahydrofuran) are added to a solution of 0.493 g of 2-[(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]-1-((S)-3-methylmorpholin-4-yl)ethanone in 20 ml of tetrahydrofuran, and the mixture is stirred at 55°C for 16 hours. The reaction mixture is then mixed with 10 ml of methanol and heated at 65°C for 2 hours. The solution is evaporated in vacuo, and the title compound is obtained as a yellow oil from the residue by flash chromatography (SiO₂ 60F). Rf = 0.07 (EtOAc); Rt = 4.52 (Gradient I).
b) 2-[(3S,4R,5R)-4-(4-Methoxyphenyl)-5-[4-(3-methoxypropyl)-3,4-dihydro-2H-benzo[1,4]oxazin-6-ylmethoxyl-1-(toluene-4-sulfonyl)piperidin-3-yloxyl-1-((S)-3-methyl-moripholin-4-yl)ethanone
   0.389 ml of propanephosphonic anhydride [68957-94-8, T3P] (50% in ethyl acetate) is added to a solution of 0.40 g of [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetic acid, 0.0671 g of (3S)-3-methylmorpholine [350595-57-2] and 0.385 ml of triethylamine in 8 ml of dichloromethane at 0°C, and the mixture is stirred at room temperature for 3 hours. The reaction mixture is diluted with dichloromethane, and 0.2M HCl is added. The phases are separated and the aqueous phase is extracted twice more with dichloromethane. The combined organic phases are washed with brine, dried with sodium sulfate and evaporated. The title compound is obtained as a yellow oil from the residue by flash chromatography (SiO₂ 60F). Rf = 0.53 (EtOAc); Rt = 4.93 (Gradient I).
c) [(3S,4R,5R)-4-(4-Methoxyphenyl)-5-[4-(3-methoxypropyl)-3,4-dihydro-2H-benzo[1,4]oxazin-6-ylmethoxy]-1-(toluene-4-sulfonyl)piperidin-3-yloxylacetic acid
   5 ml of a 1.5M aqueous lithium hydroxide solution are added to a solution of 0.75 g of methyl [(3S,4R,5R)-4-(4-methoxyphenyl)-5-[4-(3-methoxypropyl)-3,4-dihydro-2H-benzo[1,4]oxazin-6-ylmethoxy]-1-(toluene-4-sulfonyl)piperidin-3-yloxy]acetate (Example 28b) in 5 ml of tetrahydrofuran, and the mixture is stirred at room temperature for 30 minutes. The reaction mixture is adjusted to pH 2 with 1 M HCl. The resulting mixture is extracted twice with 80 ml of ethyl acetate each time. The combined organic phases are washed with brine, dried with sodium sulfate and evaporated. The title compound is obtained as a yellow oil and is used without further purification in the next stage. Rf = 0.15 (EtOAc); Rt = 4.70 (Gradient I).

The following compounds are prepared in an analogous manner to the process described in Example 59:

### Examples

### 60 6-{(3R,4R,5S)-4-(4-Methoxyphenyl)-5-[2-((R)-3-methylmorpholin-4-yl)ethoxy]-piperidin-3-yloxymethyl}-4-(3-methoxypropyl)-3,4-dihydro-2H-benzo[1,4]oxazine

### 62 6-{(3R,4R,5S)-4-(4-Methoxyphenyl)-5-[2-((S)-2-methylpiperidin-1-yl)ethoxy]piperidin-3-yloxymethyl}-4-(3-methoxypropyl)-3,4-dihydro-2H-benzo[1,4]oxazine

### 63 6-[(3R,4R,5S)-5-[2-((3S,5S)-3,5-Dimethylmorpholin-4-yl)ethoxy]-4-(4-methoxy-phenyl)piperidin-3-yloxymethyl]-4-(3-methoxypropyl)-3,4-dihydro-2H-benzo[1,4]oxazine

### 68 6-[(3R,4R,5S)-5-[2-((3R,5S)-3,5-Dimethylmorpholin-4-yl)ethoxy]-4-(4-methoxyphenyl)piperidin-3-yloxymethyl]-4-(3-methoxypropyl)-3,4-dihydro-2H-benzo[1,4]oxazine

### 76 6-[(3R,4R,5S)-5-[2-((2S,6R)-2,6-Dimethylpiperidin-1-yl)ethoxy]-4-(4-methoxyphenyl)-piperidin-3-yloxymethyl]-4-(3-methoxypropyl)-3,4-dihydro-2H-benzo[1,4]oxazine

### Example 64

### ((R)-2-{(3S,4R,5R)-4-[4-(3-Methoxyproipoxy)phenyl]-5-[4-(3-methoxypropyl)-3,4-dihydro-2H-benzo[1,4]oxazin-6-ylmethoxy]piperidin-3-yloxy}-1-methylethyl)dimethylamine

The title compound is prepared from 0.118 g of benzyl (3S,4R,5R)-3-((R)-2-dimethyl-amino-propoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4R,5R)-3-((R)-2-dimethylaminoiproipoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   A solution of 0.177 g of benzyl (3S,4R,5R)-3-((S)-2-methanesulfonyloxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in 5 ml of dimethylamine (33% in ethanol) is stirred at 50°C for 20 hours and then evaporated. The residue is diluted with 100 ml of tert-butyl methyl ether and washed with 20 ml of saturated aqueous sodium bicarbonate solution. The aqueous phase is then extracted with 100 ml of tert-butyl methyl ether. The combined organic phases are dried with sodium sulfate and evaporated. The crude title compound is obtained as a yellow oil from the residue. Rt = 4.70 (Gradient I).
b) Benzyl (3S,4R,5R)-3-((S)-2-methanesulfonyloxypropoxy)-4-[4-(3-methoxypropoxy)-phenyl]-5-[4-(3-methoxypropyl)-3,4-dihydro-2H-benzo[1,4]oxazin-6-ylmethoxy]piperidine-1-carboxylate
   0.034 ml of methanesulfonyl chloride is added to a solution of 0.27 g of benzyl (3S,4R,5R)-3-((S)-2-hydroxypropoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate and 0.066 ml of triethylamine in 5 ml of dichloromethane at 0°C. After 1 hour at 0°C, a further 0.005 ml of methanesulfonyl chloride and 0.012 ml of triethylamine are added to the reaction solution. After 6 hours at room temperature, the reaction mixture is diluted with 200 ml of tert-butyl methyl ether and washed successively with 20 ml of 0.1 N HCl, 30 ml of saturated aqueous sodium bicarbonate solution, 20 ml of water and 10 ml of brine. The organic phase is dried with sodium sulfate and evaporated. The crude title compound is obtained as a yellow oil from the residue. Rf = 0.30 (EtOAc-heptane 2:1); Rt = 5.27 (Gradient I).
c) Benzyl (3S,4R,5R)-3-((S)-2-hydroxypropoxy)-4-[4-(3-methoxypropoxy)phenyl]-5-[4-(3-methoxvpropyl)-3,4-dihydro-2H-benzo[1,4]oxazin-6-ylmethoxy]piperidine-1-carboxylate
   0.347 g of (S)-1-{(3S,4R,5R)-4-[4-(3-methoxypropoxy)phenyl]-5-[4-(3-methoxypropyl)-3,4-dihydro-2H-benzo[1,4]oxazin-6-ylmethoxy]piperidin-3-yloxy}propan-2-ol (Example 44) are reacted in analogy to Example 11 h. The title compound is obtained as a colourless resin. Rf = 0.21 (EtOAc-heptane 2:1); Rt = 5.03 (Gradient I).

The following compound is prepared in an analogous manner to the process described in Example 64:

### Example

### 65 ((S)-2-{(3S,4R,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}-1-methylethyl)dimethyl-amine

### Example 66

### (R)-1-{(3S,4R,5R)-4-[4-(3-Methoxypropoxy)phenyl]-5-[4-(3-methoxypropyl)-3,4-dihydro-2H-benzo[1,4]oxazin-6-ylmethoxylpiperidin-3-yloxy}butan-2-ol

The title compound is obtained from 0.280 g of benzyl (3R,4R,5S)-3-((R)-2-hydroxybutoxy)-4-[4-(3-methoxypropoxy)phenyl]-3-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3R,4R,5S)-3-((R)-2-hydroxybutoxy)-4-[4-(3-methoxypropoxy)phenyl]-3-[4-(3-methoxypropyl)-3,4-dihydro-2H-benzo[1,4]oxazin-6-ylmethoxylpiperidine-1-carboxylate
   0.010 g of copper(I) cyanide are taken up in 5 ml of dry tetrahydrofuran under argon in a heat-dried Schlenk tube. The suspension is cooled to -78°C, and 0.30 ml of methylmagnesium bromide solution (35% in diethyl ether) is added dropwise. A solution of 0.475 g of benzyl (3R,4R,5S)-4-[4-(3-methoxypropoxy)phenyl]-3-[4-(3-methoxypropyl)-3,4-dihydro-2H-benzo[1,4]oxazin-6-ylmethoxy]-5-((R)-oxiranyl-methoxy)piperidine-1-carboxylate (Example 36a) in 4 ml of dry tetrahydrofuran is added, and the reaction mixture is stirred at -78°C for 30 minutes and then thawed to 20°C over 16 hours. The reaction mixture is poured into saturated aqueous ammonium chloride solution and adjusted to pH 10 with 25% aqueous ammonium hydroxide solution. The mixture is extracted with diethyl ether, and the combined organic extracts are washed with brine, dried with sodium sulfate and evaporated. The title compound is obtained as a colourless oil from the residue by flash chromatography (SiO₂ 60F). Rf = 0.15 (EtOAc-heptane 2:1); Rt = 5.22 (Gradient I).

The following compounds are prepared in an analogous manner to the process described in Example 66:

### Examples

### 67 (R)-1-{(3S,4R,5R)-4-[4-(3-Methoxypropoxy)phenyl]-5-[4-(3-methoxypropyl)-3,4-dihydro-2H-benzo[1,4]oxazin-6-ylmethoxylpiperidin-3-yloxy}butan-2-ol

using toluene-4-sulfonic acid (S)-1-oxiranylmethyl ester [70987-78-9]

### 106 (S)-1-{(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}butan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a) using toluene-4-sulfonic acid (S)-1-oxiranylmethyl ester [70987-78-9]

### 108 (R)-1-{(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}butan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### 113 (S)-1-{(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}pentan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a) using ethylmagnesium bromide solution (1 M in tetrahydrofuran) using toluene-4-sulfonic acid (S)-1-oxiranylmethyl ester [70987-78-9]

### 114 (R)-1-{(3S,4R,5R)-4-[4-(4-Methoxybutoxy)phenyl]-5-[4-(3-methoxypropyl)-3,4-dihydro-2H-benzo[1,4]oxazin-6-ylmethoxylpiperidin-3-yloxy}pentan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a) using ethylmagnesium bromide solution (1 M in tetrahydrofuran)

### 125 (S)-1-{(3S,4R,5R)-4-[4-(3-Methoxy-propoxy)-phenyl]-5-[4-(3-methoxy-propyl)-3,4-dihydro-2H-benzo[1,4]oxazin-6-ylmethoxyl-piperidin-3-yloxy}-pentan-2-ol

starting from (3S,4S,5R)-3-hydroxy-4-[4-(3-methoxy-propoxy)-phenyl]-5-[4-(3-methoxy-propyl)-3,4-dihydro-2H-benzo[1,4]oxazin-6-ylmethoxy]-piperidin-1-carboxylic acid benzyl ester (Example 20b) using toluene-4-sulfonic acid (S)-1-oxiranylmethyl ester [70987-78-9] and ethylmagnesium bromid solution (1M in tetrahydrofuran).

### 126

### (R)-1-{(3S,4R,5R)-4-[4-(3-Methoxy-propoxy)-phenyl]-5-[4-(3-methoxy-propyl)-3,4-dihydro-2H-benzo[1,4]oxazin-6-ylmethoxyl-piperidin-3-yloxy}-pentan-2-ol

starting from (3S,4S,5R)-3-hydroxy-4-[4-(3-methoxy-propoxy)-phenyl]-5-[4-(3-methoxy-propyl)-3,4-dihydro-2H-benzo[1,4]oxazin-6-ylmethoxy]-piperidin-1-carboxylic acid benzyl ester (Example 20b) using ethylmagnesium bromid solution (1M in tetrahydrofuran).

### Example 69

### (R)-1-Methoxy-3-[(3S,4R,5R)-5-[4-(3-methoxypropyl)-3,4-dihydro-2H-benzo[1,4]oxazin-6-ylmethoxy]-4-(4-methylsulfanylphenyl)piperidin-3-yloxy]propan-2-ol

3 ml of 40% aqueous potassium hydroxide solution are added to a solution of 0.047 g of benzyl (3S,4R,5R)-3-((R)-2-hydroxy-3-methoxypropoxy)-5-[4-(3-methoxypropyl)-3,4-dihydro-2H-benzo[1,4]oxazin-6-ylmethoxy]-4-(4-methylsulfanylphenyl)piperidine-1-carboxylate in 3 ml of methanol and 1 ml of dioxane. The reaction mixture is heated under reflux for 3 hours. It is then diluted with 40 ml of water and extracted three times with 40 ml of ethyl acetate each time. The combined organic phases are dried with sodium sulfate, filtered and evaporated in vacuo. The title compound is obtained from the residue by flash chromatography (SiO₂ 60F).

The starting materials are prepared as follows:
a) Benzyl (3S,4R,5R)-3-((R)-2-hydroxy-3-methoxypropoxy)-5-[4-(3-methoxypropyl)-3,4-dihydro-2H-benzo[1,4]oxazin-6-ylmethoxy]-4-(4-methylsulfanyliphenyl)piperidine-1-carboxylate
   0.068 g of benzyl (3S,4S,5R)-3-hydroxy-5-[4-(3-methoxypropyl)-3,4-dihydro-2H-benzo[1,4]oxazin-6-ylmethoxy]-4-(4-methylsulfanylphenyl)piperidine-1-carboxylate and 0.023 g of S-(+)-glycidyl methyl ether [64491-68-5] are reacted in analogy to method M. The title compound is obtained as a yellow oil. Rf = 0.20 (EtOAc-heptane 2:1); Rt = 5.04 (Gradient I).
b) Benzyl (3S,4S,5R)-3-hydroxy-5-[4-(3-methoxypropyl)-3,4-dihvdro-2H-benzo[1,4]oxazin-6-ylmethoxyl-4-(4-methylsulfanylphenyl)piperidine-1-carboxylate
   0.095 g of benzyl (3R,4R,5S)-3-[4-(3-methoxypropyl)-3,4-dihydro-2H-benzo[1,4]oxazin-6-ylmethoxy]-4-(4-methylsulfanylphenyl)-5-triisopropylsilanyl-oxypiperidine-1-carboxylate are reacted in analogy to method J. The title compound is obtained as a yellow oil. Rf = 0.13 (EtOAc-heptane 1:1); Rt = 4.95 (Gradient I).
c) Benzyl (3R,4R,5S)-3-[4-(3-methoxypropyl)-3,4-dihydro-2H-benzo[1,4]oxazin-6-ylmethoxyl-4-(4-methylsulfanylphenyl)-5-triisopropylsilanyloxypiperidine-1-carboxylate
   0.477 g of caesium fluoride is added to a degassed solution of 0.718 g of benzyl (3R,4R,5S)-3-[4-(3-methoxypropyl)-3,4-dihydro-2H-benzo[1,4]oxazin-6-ylmethoxy]-5-triisopropylsilanyloxy-4-(4-triisopropylsilanylsulfanylphenyl)piperidine-1-carboxylate in 15 ml of DMF under argon, and the mixture is stirred at room temperature for 2 hours. The mixture is then cooled to -12°C and, after addition of 0.060 ml of methyl iodide, stirred at this temperature for 3 hours. The reaction mixture is diluted with tert-butyl methyl ether and poured into water. The organic phase is dried with sodium sulfate, filtered and evaporated. The title compound is obtained as a yellow oil from the residue by flash chromatography (SiO₂ 60F). Rf = 0.56 (EtOAc-heptane 1:1).
d) Benzyl (3R,4R,5S)-3-[4-(3-Methoxypropyl)-3,4-dihydro-2H-benzo[1,4]oxazin-6-ylmethoxyl-5-triisopropylsilanyloxy-4-(4-triisopropylsilanylsulfanylphenyl)piperidine-1-carboxylate
   0.323 g of sodium tert-butoxide is added to 0.73 ml of triisopropylsilanethiol in 7 ml of toluene in a Schlenk tube at 0°C, and the mixture is stirred at room temperature for 45 minutes. In a second flask, 2.0 g of benzyl (3R,4R,5S)-3-[4-(3-methoxypropyl)-3,4-dihydro-2H-benzo[1,4]oxazin-6-ylmethoxy]-4-(4-trifluoromethanesulfonyloxy-phenyl)-5-triisopropylsilanyloxypiperidine-1-carboxylate (Example 72c) are dissolved in 7 ml of toluene and, under argon, 0.415 g of palladium(0) tetrakistriphenylphosphine is added. This suspension is added to the above "thiolate" solution which has been preheated to 90°C and is stirred under argon at 90°C overnight. The reaction mixture is diluted with tert-butyl methyl ether and poured into water. The organic phase is dried with sodium sulfate, filtered and evaporated. The title compound is obtained as a brown oil from the residue by flash chromatography (SiO₂ 60F). Rf = 0.39 (EtOAc-heptane 1:2).

The following compounds are prepared in an analogous manner to the process described in Example 69:

### Examples

### 70 (R)-1-Methoxy-3-{(3S,4R,5R)-4-[4-(2-methoxyethylsulfanyl)phenyl]-5-[4-(3-methoxy-propyl)-3,4-dihydro-2H-benzo[1,4]oxazin-6-ylmethoxy]piperidin-3-yloxy}propan-2-ol

### 71 (R)-1-Methoxy-3-{(3S,4R,5R)-5-[4-(3-methoxypropyl)-3,4-dihydro-2H-benzo[1,4]oxazin-6-ylmethoxy]-4-[4-(3-methoxypropylsulfanyl)phenyl]piperidin-3-yloxy}propan-2-ol

### 103 (R)-1-Methoxy-3-{(3S,4R,5R)-4-[4-(4-methoxybutoxy)phenyl]-5-[4-(3-methoxy-propyl)-3,4-dihydro-2H-benzo[1,4]oxazin-6-ylmethoxy]piperidin-3-yloxy}propan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### 104 (S)-1-Methoxy-3-{(3S,4R,5R)-4-[4-(4-methoxybutoxy)phenyl]-5-[4-(3-methoxypropyl)-3,4-dihydro-2H-benzo[1,4]oxazin-6-ylmethoxy]piperidin-3-yloxy}propan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a) using R-(-)-glycidyl methyl ether [64491-70-9].

### Example 72

### 4-{(3S,4S,5R)-3-Hydroxy-5-[4-(3-methoxypropyl)-3,4-dihydro-2H-benzo[1,4]oxazin-6-ylmethoxy]piperidin-4-yl}benzonitrile

The title compound is obtained from 0.0538 g of benzyl (3S,4S,5R)-4-(-cyanophenyl)-3-hydroxy-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4S,5R)-4-(4-cyanophenyl)-3-hydroxy-5-[4-(3-methoxypropyl)-3,4-dihydro-2H-benzo[1,4]oxazin-6-ylmethoxylpiperidine-1-carboxylate
   A mixture of 1.290 g of benzyl (3S,4S,5R)-3-hydroxy-5-[4-(3-methoxypropyl)-3,4-dihydro-2H-benzo[1,4]oxazin-6-ylmethoxy]-4-(trifluoromethanesulfonyloxyphenyl)piperidine-1-carboxylate, 0.440 g of zinc(II) cyanide and 0.193 g of palladium(0) tetrakistriphenylphosphine in 11 ml of dry N,N-dimethylformamide is heated at 120°C for 16 hours. the reaction mixture is poured into saturated aqueous sodium bicarbonate solution, and the mixture is then extracted with tert-butyl methyl ether. The combined organic phases are washed with brine, dried and evaporated. The title compound is obtained as a colourless resin from the residue by flash chromatography (SiO₂ 60F). Rf = 0.11 (EtOAc-heptane 3:2); Rt = 4.61 (Gradient I).
b) Benzyl (3S,4S,5R)-3-hydroxy-5-[4-(3-methoxypropyl)-3,4-dihydro-2H-benzo[1,4]oxazin-6-ylmethoxy]-4-(trifluoromethanesulfonyloxyphenyl)piperidine-1-carboxylate
   The title compound is obtained as a pale yellow resin from 2.340 g of benzyl (3S,4R,5R)-3-[4-(3-methoxypropyl)-3,4-dihydro-2H-benzo[1,4]oxazin-6-ylmethoxy]-4-(trifluoromethanesulfonyloxyphenyl)-5-triisopropylsilanyloxypiperidine-1-carboxylate in analogy to method J. Rf = 0.37 (EtOAc-heptane 2:1); Rt = 5.20 (Gradient I).
c) Benzyl (3S,4R,5R)-3-[4-(3-methoxypropyl)-3,4-dihydro-2H-benzo[1,4]oxazin-6-ylmethoxy]-4-(4-trifluoromethanesulfonyloxyphenyl)-5-triisopropylsilanyloxypiperidine-1-carboxylate
   0.544 ml of triethylamine is added to a solution of 2.590 g of benzyl (3R,4R,5S)-4-(4-hydroxy-phenyl)-3-[4-(3-methoxypropyl)-3,4-dihydro-2H-benzo[1,4]oxazin-6-ylmethoxy]-5-triisopropylsilanyloxypiperidine-1-carboxylate (Example 11d) and 1.353 g of N-phenyl-bis(trifluoromethanesulfonamide) in 20 ml of dry dichloromethane. The reaction solution is left to stand at room temperature for 3 hours and then evaporated to dryness. The title compound is obtained as a reddish oil from the residue by flash chromatography (SiO₂ 60F). Rf = 0.56 (EtOAc-heptane 1:1).

### Example 73

### 4-{(3S,4R,5R)-3((R)-2-Hydroxy-3-methoxypropoxy)-5-[4-(3-methoxypropyl)-3,4-dihydro-2H-benzo[1,4]oxazin-6-ylmethoxy]piperidin-4-yl}benzonitrile

The title compound is obtained from 0.065 g of benzyl (3S,4S,5R)-4-(4-cyanophenyl)-3-((R)-2-hydroxy-3-methoxypropoxy)-5-[4-(3-methoxypropyl)-3,4-dihydro-2H-benzo[1,4]oxazin-6-ylmethoxy]piperidine-1-carboxylate in analogy to method B.

The starting material is prepared as follows:
a) Benzyl (3S,4S,5R)-4-(4-cyanophenyl)-3-((R)-2-hydroxy-3-methoxypropoxy)-5-[4-(3-methoxypropyl)-3,4-dihydro-2H-benzo[1,4]oxazin-6-ylmethoxy]piperidine-1-carboxylate
   The title compound is obtained as a colourless resin from 0.100 g of benzyl (3S,4S,5R)-4-(4-cyanophenyl)-3-hydroxy-5-[4-(3-methoxypropyl)-3,4-dihydro-2H-benzo[1,4]oxazin-6-ylmethoxy]piperidine-1-carboxylate (Example 72a) and 0.061 g of S-(+)-glycidyl methyl ether [64491-68-5] in analogy to method M. Rf = 0.16 (EtOAc-heptane 2:1); Rt = 4.68 (Gradient I).

The following compound is prepared in an analogous manner to the process described in Example 73:

### Example

### 74 4-{(3S,4R,5R)-3((S)-2-Hydroxy-3-methoxypropoxy)-5-[4-(3-methoxypropyl)-3,4-dihydro-2H-benzo[1,4]oxazin-6-ylmethoxy]piperidin-4-yl}-benzonitrile

### Example 77

### 7-{(3R,4S,5S)-5-Hydroxy-4-[4-(3-methoxypropoxy)phenyl]piperidin-3-yloxymethyl}-3,3-dimethyl-1,3-dihydroindol-2-one

The title compound is prepared from 0.350 g of benzyl (3R,4S,5S)-3-(3,3-dimethyl-2-oxo-2,3-dihydro-1H-indol-7-ylmethoxy)-5-hydroxy-4-[4-(3-methoxypropoxy)phenyl]piperidine-1-carboxylate in analogy to method B.

The starting materials are prepared as follows:
a) Benzyl (3R,4S,5S)-3-(3,3-dimethyl-2-oxo-2,3-dihydro-1H-indol-7-ylmethoxy)-5-hydroxy-4-[4-(3-methoxypropoxy)phenyl]piperidine-1-carboxylate
   40 ml of tetrabutylammonium fluoride (1M solution in tetrahydrofuran) are added to a solution of 1.78 g of benzyl (3R,4R,5S)-3-[3,3-dimethyl-2-oxo-1-(2-trimethylsilanyl-ethoxymethyl)-2,3-dihydro-1H-indol-7-ylmethoxy]-4-[4-(3-methoxypropoxy)phenyl]-5-triisopropylsilanyloxypiperidine-1-carboxylate in 18 ml of tetrahydrofuran, and the mixture is stirred at the reflux temperature for 4 days. The reaction mixture is poured into ice-water and extracted with tert-butyl methyl ether. The organic phases are washed with water and brine, dried with sodium sulfate and evaporated. The title compound is obtained as a white foam from the residue by flash chromatography (SiO₂ 60F). Rf = 0.20 (EtOAc-heptane 1:1); Rt = 4.54 (Gradient I).
b) Benzyl (3R,4R,5S)-3-[3,3-dimethyl-2-oxo-1-(2-trimethylsilanylethoxymethyl)-2,3-dihydro-1H-indol-7-ylmethoxy]-4-[4-(3-methoxypropoxy)phenyl]-5-triisopropylsilanyloxypiperidine-1-carboxylate
   2.0 g of benzyl (3R,4R,5S)-3-hydroxy-4-[4-(3-methoxypropoxy)phenyl]-5-triisopropylsilanyloxypiperidine-1-carboxylate (Example 20e) and 1.60 g of 7-bromomethyl-3,3-dimethyl-1-(2-trimethylsilanylethoxymethyl)-1,3-dihydroindol-2-one [985278-97-8] are reacted in analogy to method D. The title compound is obtained as a colourless oil. Rf = 0.22 (EtOAc-heptane 1:4).

### Example 78

### (3S,4S,5R)-4-[4-(4-Methoxybutoxy)phenyl]-5-[4-(3-methoxypropyl)-3,4-dihydro-2H-benzo[1,4]oxazin-6-ylmethoxy]piperidin-3-ol

The title compound is prepared from 0.264 g of benzyl (3S,4S,5R)-3-hydroxy-4-[4-(4-methoxybutoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4S,5R)-3-hydroxy-4-[4-(4-methoxybutoxy)phenyl]-5-[4-(3-methoxypropyl)-3,4-dihydro-2H-benzo[1,4]oxazin-6-ylmethoxy]piperidine-1-carboxylate
   1.11 g of benzyl (3R,4R,5S)-4-[4-(4-methoxybutoxy)phenyl]-3-[4-(3-methoxypropyl)-3,4-dihydro-2H-benzo[1,4]oxazin-6-ylmethoxy]-5-triisopropylsilanyloxypiperidine-1-carboxylate are reacted in analogy to Example 1 b. The title compound is obtained as a cloudy white oil. Rf = 0.60 (EtOAc); Rt = 4.94 (Gradient I).
b) Benzyl (3R,4R,5S)-4-[4-(4-methoxybutoxy)phenyl]-3-[4-(3-methoxypropyl)-3,4-dihydro-2H-benzo[1,4]oxazin-6-ylmethoxy]-5-triisopropylsilanyloxypiperidine-1-carboxylate
   The title compound is prepared from 1.15 g of benzyl (3R,4R,5S)-3-hydroxy-4-(4-hydroxy-phenyl)-5-triisopropylsilanyloxypiperidine-1-carboxylate (Example 11h) and 0.461 g of 1-bromo-3-methoxypropane [4457-67-4] in analogy to the process described in Example 20c-e. Rf = 0.19 (EtOAc-heptane 1:1).

### Example 80

### (3S,4S,5R)-5-[4-(3-Methoxypropyl)-3,4-dihydro-2H-benzo[1,4]oxazin-6-ylmethoxy]-4-[4-(3-methylsulfanylpropoxy)phenyl]piperidin-3-ol

5 ml of 40% aqueous potassium hydroxide solution are added to a solution of 0.38 g of benzyl (3S,4S,5R)-3-hydroxy-5-[4-(3-methoxypropyl)-3,4-dihydro-2H-benzo[1,4]oxazin-6-ylmethoxy]-4-[4-(3-methylsulfanylpropoxy)phenyl]piperidine-1-carboxylate in 5 ml of methanol and 2 ml of dioxane. The reaction mixture is heated under reflux for 3 hours. It is then diluted with 40 ml of water and extracted three times with 40 ml of ethyl acetate each time. The combined organic phases are dried with sodium sulfate, filtered and evaporated in vacuo. The title compound is obtained from the residue by flash chromatography (SiO₂ 60F).

The starting materials are prepared as follows:
a) Benzyl (3S,4S,5R)-3-hydroxy-5-[4-(3-methoxypropyl)-3,4-dihydro-2H-benzo[1,4]oxazin-6-ylmethoxy]-4-[4-(3-methylsulfanylpropoxy)phenyl]piperidine-1-carboxylate
   1.2 g of benzyl (3R,4R,5S)-3-[4-(3-methoxypropyl)-3,4-dihydro-2H-benzo[1,4]oxazin-6-ylmethoxy]-4-[4-(3-methylsulfanylpropoxy)phenyl]-5-triisopropylsilanyloxypiperidine-1-carboxylate are reacted in analogy to method J. The title compound is obtained as a colourless resin. Rf = 0.18 (EtOAc-heptane 4:1); Rt = 5.15 (Gradient I).
b) Benzyl (3R,4R,5S)-3-[4-(3-methoxylpropyl)-3,4-dihydro-2H-benzo[1,4]oxazin-6-ylmethoxy]-4-[4-(3-methylsulfanylpropoxy)phenyl]-5-triisopropylsilanyloxypiperidine-1-carboxylate
   3.0 g of benzyl (3R,4R,5S)-4-(4-hydroxyphenyl)-3-[4-(3-methoxypropyl)-3,4-dihydro-2H-benzo[1,4]oxazin-6-ylmethoxy]-5-triisopropylsilanyloxypiperidine-1-carboxylate (Example 11 d) and 1.49 g of 3-methylsulfanylpropyl toluene-4-sulfonate [187722-18-5] are reacted in analogy to method G. The title compound is obtained as a yellow oil. Rf = 0.18 (EtOAc-heptane 1:2).

### Example 81

### (3S,4S,5R)-4-[4-(4-Methoxybutylsulfanyl)phenyl]-5-[4-(3-methoxypropyl)-3,4-dihydro-2H-benzo[1,4]oxazin-6-ylmethoxy]piperidin-3-ol

A solution of 0.33 g of benzyl (3R,4R,5S)-4-[4-(4-methoxybutylsulfanyl)phenyl]-3-[4-(3-methoxypropyl)-3,4-dihydro-2H-benzo[1,4]oxazin-6-ylmethoxy]-5-triisopropylsilanyloxypiperidine-1-carboxylate in 5 ml of dioxane, 6 ml of aqueous 40% potassium hydroxide solution and 6 ml of methanol is stirred at 90°C for 4 days. The reaction mixture is diluted at room temperature with 50 ml of tert-butyl methyl ether and mixed with 20 ml of water. The aqueous phase is then extracted with 2 X 50 ml of tert-butyl methyl ether. The combined organic phases are washed with 20 ml of brine, dried with sodium sulfate and evaporated. The title compound is obtained from the residue by flash chromatography (SiO₂ 60F).

The starting materials are prepared as follows:
a) Benzyl (3R,4R,5S)-4-[4-(4-methoxybutylsulfanyl)phenyl]-3-[4-(3-methoxypropyl)-3,4-dihydro-2H-benzo[1,4]oxazin-6-ylmethoxy]-5-triisopropylsilanyloxypiperidine-1-carboxylate
   The title compound is obtained as a colourless oil from benzyl (3R,4R,5S)-4-[4-(4-methoxy-butylsulfanyl)phenyl]-3-[4-(3-methoxypropyl)-3-oxo-3,4-dihydro-2H-benzo[1,4]oxazin-6-ylmethoxy]-5-triisopropylsilanyloxypiperidine-1-carboxylate in analogy to method K (temperature: 45°C). Rf = 0.40 (EtOAc-heptane 1:1).
b) Benzyl (3R,4R,5S)-4-[4-(4-methoxybutylsulfanyl)phenyl]-3-[4-(3-methoxypropyl)-3-oxo-3,4-dihydro-2H-benzo[1,4]oxazin-6-ylmethoxy]-5-triisopropylsilanyloxypiperidine-1-carboxylate
   The title compound is obtained as a yellow oil from benzyl (3R,4R,5S)-3-hydroxy-4-[4-(4-methoxybutylsulfanyl)phenyl]-5-triisopropylsilanyloxypiperidine-1-carboxylate in analogy to Example 16b. Rf = 0.45 (EtOAc-heptane 1:1); Rt = 7.38 (Gradient I).
c) Benzyl (3R,4R,5S)-3-hydroxy-4-[4-(4-methoxybutylsulfanyl)phenyl]-5-triisopropylsilanyloxypiperidine-1-carboxylate
   The title compound is obtained as a yellow oil from benzyl (3R,4R,5S)-3-hydroxy-5-triisopropylsilanyloxy-4-(4-triisopropylsilanylsulfanylphenyl)piperidine-1-carboxylate and 1-bromo-3-methoxypropane [4457-67-4] in analogy to Example 69c. Rf = 0.40 (EtOAc-heptane 1:1); Rt = 6.76 (Gradient I).
d) Benzyl (3R,4R,5S)-3-hydroxy-5-triisopropylsilanyloxy-4-(4-triisoiproipylsilanylsulfanyl-phenyl)piperidine-1-carboxylate
   The title compound is obtained as a red oil from benzyl (3R,4R,5S)-3-hydroxy-4-(4-trifluoromethanesulfonyloxyphenyl)-5-triisopropylsilanyloxypiperidine-1-carboxylate in analogy to Example 69d. Rf = 0.07 (EtOAc-heptane 1:4).
e) Benzyl (3R,4R,5S)-3-hydroxy-4-(4-trifluoromethanesulfonyloxyphenyl)-5-triisopropylsilanyloxypiperidine-1-carboxylate
   The title compound is obtained as a yellowish resin from benzyl (3R,4R,5S)-3-hydroxy-4-(4-hydroxyphenyl)-5-triisopropylsilanyloxypiperidine-1-carboxylate (Example 19d) in analogy to Example 72c. Rf = 0.58 (EtOAc-heptane 1:1); Rt = 6.61 (Gradient I).

The following compound is prepared in an analogous manner to the process described in Example 81:

### Example

### 82 (3S,4S,5R)-5-[4-(3-Methoxypropyl)-3,4-dihydro-2H-benzo[1,4]oxazin-6-ylmethoxy]-4-[4-(3-methoxypropylsulfanyl)phenyl]piperidin-3-ol

### Example 83

### (3S,4S,5R)-4-[4-((R)-4-Methoxy-3-methylbutylsulfanyl)phenyl]-5-[4-(3-methoxypropyl)-3,4-dihydro-2H-benzo[1,4]oxazin-6-ylmethoxy]piperidin-3-ol

A solution of 0.44 g of methyl (3S,4S,5R)-3-hydroxy-4-[4-((R)-4-methoxy-3-methylbutylsulfanyl)phenyl]-5-[4-(3-methoxypropyl)-3,4-dihydro-2H-benzo[1,4]oxazin-6-ylmethoxy]-piperidine-1-carboxylate in 1 ml of dioxane, 0.7 ml of aqueous 40% KOH and 1.2 ml of methanol is stirred at 90°C for 1 hour. The reaction mixture is diluted at room temperature with tert-butyl methyl ether and mixed with water. The aqueous phase is extracted with tert-butyl methyl ether (2X). The combined organic phases are washed with brine, dried with sodium sulfate and evaporated. The title compound is obtained from the residue by flash chromatography (SiO₂ 60F).

The starting materials are prepared as follows:
a) Methyl (3S,4S,5R)-3-hydroxy-4-[4-((R)-4-methoxy-3-methylbutylsulfanyl)phenyl]-5-[4-(3-methoxypropyl)-3,4-dihydro-2H-benzo[1,4]oxazin-6-ylmethoxy]piperidine-1-carboxylate
   0.31 g of methyl (3R,4R,5S)-4-[4-((R)-4-methoxy-3-methylbutylsulfanyl)phenyl]-3-[4-(3-methoxypropyl)-3,4-dihydro-2H-benzo[1,4]oxazin-6-ylmethoxy]-5-triisopropylsilanyloxypiperidine-1-carboxylate is reacted in analogy to Example 1b. The title compound is obtained as a colourless oil. Rf = 0.15 (EtOAc-heptane 2:1); Rt = 4.73 (Gradient I).
b) Methyl (3R,4R,5S)-4-[4-((R)-4-methoxy-3-methylbutylsulfanyl)phenyl]-3-[4-(3-methoxy-propyl)-3,4-dihydro-2H-benzo[1,4]oxazin-6-ylmethoxy]-5-triisopropylsilanyloxypiperidine-1-carboxylate
   The title compound is obtained as a colourless oil from 0.5 g of methyl (3R,4R,5S)-3-hydroxy-4-[4-((R)-4-methoxy-3-methylbutylsulfanyl)phenyl]-5-triisopropylsilanyloxypiperidine-1-carboxylate in analogy to the process described in Example 81 a-b. Rf = 0.53 (EtOAc-heptane 1:1).
c) Methyl (3R,4R,5S)-3-hydroxy-4-[4-((R)-4-methoxy-3-methylbutylsulfanyl)phenyl]-5-triisopropylsilanyloxypiperidine-1-carboxylate
   10.9 g of sodium methoxide are added to a solution of 2.09 g of benzyl (3R,4R,5S)-3-hydroxy-4-{4-[(R)-3-methyl-4-(toluene-4-sulfonyloxy)butylsulfanyl]phenyl}-5-triisopropylsilanyloxypiperidine-1-carboxylate in 11 ml of methanol and 15 ml of tetrahydrofuran. The reaction mixture is stirred at 50°C until conversion is complete and then diluted with 150 ml of tert-butyl methyl ether at room temperature. The mixture is washed with 40 ml of saturated aqueous sodium bicarbonate solution, 100 ml of water and 30 ml of brine. The organic phase is dried with sodium sulfate and evaporated. The crude title compound is obtained a s yellowish oil from the residue. Rf = 0.50 (EtOAc-heptane 1:1); Rt = 6.53 (Gradient I).
d) Benzyl (3R,4R,5S)-3-hydroxy-4-{4-[(R)-3-methyl-4-(toluene-4-sulfonyloxy)butylsulfanyl]-phenyl}-5-triisopropylsilanyloxypiperidine-1-carboxylate 1.5 g of benzyl (3R,4R,5S)-3-hydroxy-5-triisopropylsilanyloxy-4-(4-triisopropylsilanylsulfanyl-phenyl)piperidine-1-carboxylate (Example 81 d) and 1.5 g of (R)-2-methylbutane-1,4-diol bistoluenesulfonate [281214-26-4] are reacted in analogy to Example 81 c. The title compound is obtained as a yellow oil. Rf = 1.6 (EtOAc-heptane 1:2).

### Example 87

### Isopropyl-{(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}amine

The title compound is prepared starting from benzyl (3R,4R,5R)-3-(isopropylaminomethyl)-4-(4-methoxyphenyl)-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3R,4R,5R)-3-(isopropylaminomethyl)-4-(4-methoxyphenyl)-5-[4-(3-methoxy-propyl)-3,4-dihydro-2H-benzo[1,4]oxazin-6-ylmethoxy]piperidine-1-carboxylate
   A solution of 0.50 mmol of benzyl (3R,4R,5S)-4-(4-methoxyphenyl)-3-[4-(3-methoxypropyl)-3,4-dihydro-2H-benzo[1,4]oxazin-6-ylmethoxy]-5-(toluene-4-sulfonyloxymethyl)piperidine-1-carboxylate and 1.0 mmol of isopropylamine in 4 ml of 1-methylpyrrolidin-2-one (NMP) is stirred at 85°C for 8 hours. The reaction mixture is cooled to room temperature, diluted with water and extracted with dichloromethane (3X). The combined organic phases are washed with brine, dried with sodium sulfate and evaporated. The title compound is obtained as a yellow oil from the residue by flash chromatography (SiO2 60F). Rf = 0.29 (dichloromethane-methanol-25% conc. ammonia = 200:10:1); Rt = 4.45 (Gradient I).
b) Benzyl (3R,4R,5S)-4-(4-methoxyphenyl)-3-[4-(3-methoxypropyl)-3,4-dihydro-2H-benzo[1,4]oxazin-6-ylmethoxy]-5-(toluene-4-sulfonyloxymethyl)piperidine-1-carboxylate
   The title compound is obtained as a yellow oil starting from benzyl (3S,4R,5R)-3-hydroxymethyl-4-(4-methoxyphenyl)-5-[4-(3-methoxypropyl)-3,4-dihydro-2H-benzo[1,4]oxazin-6-ylmethoxy]piperidine-1-carboxylate in analogy to method H. The crude title compound is used in the next stage. Rf = 0.39 (EtOAc-heptane = 2:1).
c) Benzyl (3S,4R,5R)-3-hydroxymethyl-4-(4-methoxyphenyl)-5-[4-(3-methoxypropyl)-3,4-dihydro-2H-benzo[1,4]oxazin-6-ylmethoxy]piperidine-1-carboxylate
   The title compound is obtained as a yellow oil starting from benzyl (3S,4R,5R)-3-hydroxymethyl-4-(4-methoxyphenyl)-5-[4-(3-methoxypropyl)-3-oxo-3,4-dihydro-2H-benzo[1,4]oxazin-6-ylmethoxy]piperidine-1-carboxylate in analogy to the process described in Example 11 d. Rf = 0.18 (EtOAc-heptane = 2:1); Rt = 4.79 (Gradient I).
d) Benzyl (3S,4R,5R)-3-hydroxymethyl-4-(4-methoxyphenyl)-5-[4-(3-methoxypropyl)-3-oxo-3,4-dihydro-2H-benzo[1,4]oxazin-6-ylmethoxy]piperidine-1-carboxylate
   A solution of 1.87 g of benzyl (3R,4R,5S)-4-(4-methoxyphenyl)-3-[4-(3-methoxypropyl)-3-oxo-3,4-dihydro-2H-benzo[1,4]oxazin-6-ylmethoxy]-5-trityloxymethylpiperidine-1-carboxylate in 20 ml of methanol and 4 ml of tetrahydrofuran is mixed with 1.53 g of P-toluenesulfonic acid monohydrate and stirred at room temperature for 1.5 hours. The reaction mixture is poured into saturated aqueous sodium bicarbonate solution and extracted with dichloromethane (3X). The combined organic phases are dried with sodium sulfate and evaporated. The crude title compound is used in the next stage.
e) Benzyl (3R,4R,5S)-4-(4-methoxyphenyl)-3-[4-(3-methoxypropyl)-3-oxo-3,4-dihydro-2H-benzo[1,4]oxazin-6-ylmethoxy]-5-trityloxymethylpiperidine-1-carboxylate
   The title compound is obtained as a colourless wax starting from benzyl (3R,4R,5S)-3-hydroxy-4-(4-methoxyphenyl)-5-trityloxymethylpiperidine-1-carboxylate in analogy to method D. Rf = 0.26 (EtOAc-heptane = 1:1); Rt = 6.25 (Gradient I).
f) Benzyl (3R,4R,5S)-3-hydroxy-4-(4-methoxyphenyl)-5-trityloxymethylpiperidine-1-carboxylate
   The title compound is obtained as a yellow oil starting from (3R,4R,5S)-1-benzyl-4-(4-methoxyphenyl)-5-trityloxymethylpiperidin-3-ol (L)-(+)-mandelate [303043-54-1] in analogy to the process described in method B (3:1 methanol-tetrahydrofuran is used as solvent) and in Example 11 h. The crude title compound is used in the next stage. Rf = 0.25 (EtOAc-heptane = 1:1).

The following compounds are prepared in an analogous manner to the process described in Example 87:

### Examples

### 88 tert-Butyl-{(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}amine

### 89 (2-Methoxyethyl)-{(3R,4R,5R)-4-(4-methoxyphenyl)-5-[4-(3-methoxypropyl)-3,4-dihydro-2H-benzo[1,4]oxazin-6-ylmethoxy]piperidin-3-ylmethyl}amine

### 90 6-[(3R,4R,5S)-4-(4-Methoxyphenyl)-5-morpholin-4-ylmethylpiperidin-3-yloxymethyl]-4-(3-methoxypropyl)-3,4-dihydro-2H-benzo[1,4]oxazine

### Example 91

### N-{(3S,4R,5R)-4-(4-Methoxyphenyl)-5-[4-(3-methoxypropyl)-3,4-dihydro-2H-benzo[1,4]oxazin-6-ylmethoxy]piperidin-3-ylmethyl}acetamide

The title compound is prepared from benzyl (3R,4R,5R)-3-(acetylaminomethyl)-4-(4-methoxyphenyl)-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3R,4R,5R)-3-(acetylaminomethyl)-4-(4-methoxyphenyl)-5-[4-(3-methoxypropyl)-3,4-dihydro-2H-benzo[1,4]oxazin-6-ylmethoxy]piperidine-1-carboxylate
   3 mmol of triethylamine are added to a solution of 1 mmol of benzyl (3R,4R,5R)-3-aminomethyl-4-(4-methoxyphenyl)-5-[4-(3-methoxypropyl)-3,4-dihydro-2H-benzo[1,4]oxazin-6-ylmethoxy]piperidine-1-carboxylate and 1.1 mmol of acetyl chloride in 20 ml of dichloromethane at 0°C. After 1.5 hours, the reaction mixture is poured into 1 M sodium bicarbonate solution and extracted with tert-butyl methyl ether (3X), and the combined organic phases are washed with brine, dried with sodium sulfate and evaporated. The title compound is obtained as a yellowish oil from the residue by flash chromatography (SiO₂ 60F). Rf = 0.29 (dichloromethane-methanol-25% conc. ammonia = 200:20:1); Rt = 4.58 (Gradient I).
b) Benzyl (3R,4R,5R)-3-aminomethyl-4-(4-methoxyphenyl)-5-[4-(3-methoxypropyl)-3,4-dihydro-2H-benzo[1,4]oxazin-6-ylmethoxy]piperidine-1-carboxylate
   A solution of 0.195 ml of 25% conc. ammonia in 0.9 ml of methanol is added to a solution of 200 mg of benzyl (3S,4R,5R)-3-azidomethyl-4-(4-methoxyphenyl)-5-[4-(3-methoxypropyl)-3,4-dihydro-2H-benzo[1,4]oxazin-6-ylmethoxy]piperidine-1-carboxylate in 0.95 ml of tetrahydrofuran and 0.23 ml of water at room temperature. After addition of 128 mg of triphenylphosphine, the reaction mixture is stirred at room temperature for 16 hours. The mixture is diluted with ethyl acetate and washed with half-saturated aqueous sodium bicarbonate solution (2X), dried with sodium sulfate and evaporated. The title compound is obtained as a colourless oil from the residue by flash chromatography (SiO₂ 60F). Rf = 0.31 (dichloromethane-methanol-25% conc. ammonia = 200:20:1); Rt = 4.24 (Gradient I).
c) Benzyl (3S,4R,5R)-3-azidomethyl-4-(4-methoxyphenyl)-5-[4-(3-methoxypropyl)-3,4-dihydro-2H-benzo[1,4]oxazin-6-ylmethoxy]piperidine-1-carboxylate
   A solution of 0.50 mmol of benzyl (3R,4R,5S)-4-(4-methoxyphenyl)-3-[4-(3-methoxypropyl)-3,4-dihydro-2H-benzo[1,4]oxazin-6-ylmethoxy]-5-(toluene-4-sulfonyloxymethyl)piperidine-1-carboxylate (Example 87b) and 2 mmol of sodium azide in 5 ml of DMPU is stirred at 80°C for 4 hours. The reaction mixture is diluted with water and extracted with tert-butyl methyl ether (3X). The combined organic phases are dried with sodium sulfate and evaporated. The title compound is obtained as a yellowish oil from the residue by flash chromatography (SiO₂ 60F). Rf = 0.33 (EtOAc-heptane = 1:1); Rt = 5.61 (Gradient I).

The following compounds are prepared in an analogous manner to the process described in Example 91.

### Examples

### 92 N-{(3S,4R,5R)-4-(4-Methoxyphenyl)-5-[4-(3-methoxypropyl)-3,4-dihydro-2H-benzo[1,4]oxazin-6-ylmethoxy]piperidin-3-ylmethyl}propionamide

### 115 Morpholine-4-carboxylic acid {(3S,4R,5R)-4-(4-methoxyphenyl)-5-[4-(3-methoxy-propyl)-3,4-dihydro-2H-benzo[1,4]oxazin-6-ylmethoxy]piperidin-3-ylmethyl}amide

using morpholine-4-carbonyl chloride [15159-40-7]

### 116 Pentanoic acid {(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}amide

### 123 1-{(3S,4R,5R)-4-(4-Methoxyphenyl)-5-[4-(3-methoxypropyl)-3,4-dihydro-2H-benzo[1,4]oxazin-6-ylmethoxy]piperidin-3-ylmethyl}-3-propylurea

using propylcarbamoyl chloride [41891-16-1]

### 124 1-Cyclopentyl-3-{(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}urea

using cyclopentylcarbamoyl chloride [80413-82-7]

### 127 Cyclopentanecarboxylic acid {(3S,4R,5R)-4-(4-methoxy-phenyl)-5-[4-(3-methoxy-propyl)-3,4-dihydro-2H-benzo[1,4]oxazin-6-ylmethoxy]-piperidin-3-ylmethyl}-amide

using cyclopentanecarbonyl chlorid [4524-93-0]

### 128 N-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-ylmethyl}-2,2-dimethyl-propionamide

using pivaloyl chlorid [3282-30-2]

### 129 {(3S,4R,5R)-4-(4-Methoxy-phenyl)-5-[4-(3-methoxy-propyl)-3,4-dihydro-2H-benzo[1,4]oxazin-6-ylmethoxy]-piperidin-3-ylmethyl}-carbamic acid methyl ester

using methyl chloroformate [79-22-1]

### 133 1-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-ylmethyl}-3-methyl-urea

using N-succinimidyl N-methylcarbamate [18342-66-0]

### 134 3-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-ylmethyl}-1,1-dimethyl-urea

using N,N-dimethylcarbamoyl chloride [79-44-7]

### 130 N-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-ylmethyl}-butyramide

using butyryl chloride [141-75-3]

### 131 N-((R)-2-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-yloxy}-1-methyl-ethyl)-isobutyramide

starting from (3S,4R,5R)-3-((S)-2-methanesulfonyloxy-propoxy)-4-(4-methoxyphenyl)-5-[4-(3-methoxy-propyl)-3,4-dihydro-2H-benzo[1,4]oxazin-6-ylmethoxy]-piperidine-1-carboxylic acid benzyl ester using isobutyryl chloride [79-30-1]

The starting materials are prepared as follows:
a) (3S,4R,5R)-3-((S)-2-Methanesulfonyloxy-propoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester
   The title compound is obtained as a brown oil from (3S,4R,5R)-3-((S)-2-hydroxy-propoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester in analogy to the process used in Example 64b. Rt = 5.24 (Gradient I).
b) (3S,4R,5R)-3-((S)-2-Hydroxy-propoxy)-4-(4-methoxy-phenyl)-5-[4-(3-methoxypropyl)-3,4-dihydro-2H-benzo[1,4]oxazin-6-ylmethoxy]-piperidine-1-carboxylic acid benzyl ester
   A solution of 6.78 mmol (3R,4R,5S)-4-(4-methoxy-phenyl)-3-[4-(3-methoxy-propyl)-3,4-dihydro-2H-benzo[1,4]oxazin-6-ylmethoxy]-5-((S)-1-oxiranylmethoxy)-piperidine-1-carboxylic acid benzyl ester in 70 ml ethanol is treated with 20.36 mmol sodium borohydrid. The solution is stitrred at 45°C over night , cooled to room temperature, diluted with tert-butyl methyl ether and washed successively with saturated ammonium chloride solution, water and brine. The aqueous phases are extracted (3X) with dichloromethane. The combined organic phases are dried with sodium sulfate and evaporated. The title compound is obtained as a yellow oil from the residue by means of flash chromatography (SiO₂ 60F). Rf = 0.20 (EtOAc-heptane 2:1); Rt = 4.96 (Gradient I).
c) (3R,4R,5S)-4-(4-Methoxy-phenyl)-3-[4-(3-methoxy-propyl)-3,4-dihydro-2H-benzo[1,4]oxazin-6-ylmethoxy]-5-((S)-1-oxiranylmethoxy)-piperidine-1-carboxylic acid benzyl ester
   The title compound is obtained as a yellow oil from (3S,4S,5R)-3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (Example 19c) in analogy to the process used in Example 36a, using toluol-4-sulfonsäure (S)-1-oxiranymethyl ester [70987-78-9]. Rf = 0.18 (EtOAc-heptane 1:1); Rt = 5.12 (Gradient I).

### 132 N-((R)-2-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-yloxy}-1-methyl-ethyl)-propionamide

starting from (3S,4R,5R)-3-((S)-2-methanesulfonyloxy-propoxy)-4-(4-methoxyphenyl)-5-[4-(3-methoxy-propyl)-3,4-dihydro-2H-benzo[1,4]oxazin-6-ylmethoxy]-piperidine-1-carboxylic acid benzyl ester (Example 131a) using propanoyl chloride [79-03-8]

### 135 N-Ethyl-N-((R)-2-{(3S,4R,5R)-4-(4-methoxy-phenyl)-5-[4-(3-methoxy-propyl)-3,4-dihydro-2H-benzo[1,4]oxazin-6-ylmethoxy]-piperidin-3-yloxy}-1-methyl-ethyl)-acetamide

starting from (3S,4R,5R)-3-((R)-2-ethylamino-propoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester

The starting materials are prepared as follows:
a) (3S,4R,5R)-3-((R)-2-Ethylamino-propoxy)-4-(4-methoxy-phenyl)-5-[4-(3-methoxypropyl)-3,4-dihydro-2H-benzo[1,4]oxazin-6-ylmethoxy]-piperidine-1-carboxylic acid benzyl ester
   A solution of 1.82 mmol (3S,4R,5R)-3-((S)-2-methanesulfonyloxy-propoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (Example 131a) in 25 ml of ethanol is treated with 100.32 mmol of ethylamine. The solution is stirred at 50°C for 4 days, cooled to room temperature, evaporated and dissolved in tert-butyl methyl ether. The organic phase is washed successively with water and brine. The aqueous phases are extracted (3X) with tert-butyl methyl ether. The combined organic phases are dried with sodium sulfate and evaporated. The title compound is obtained as a yellow oil from the residue by means of flash chromatography (SiO₂ 60F). Rf = 0.16 (EtOAc-triethylamine 100:1); Rt = 4.61 (Gradient I).

### 136 N-((R)-2-{(3S,4R,5R)-4-(4-Methoxy-phenyl)-5-[4-(3-methoxy-propyl)-3,4-dihydro-2H-benzo[1,4]oxazin-6-ylmethoxy]-piperidin-3-yloxy}-1-methyl-ethyl)-N-propyl-acetamide

starting from (3R,4R,5S)-4-(4-methoxy-phenyl)-3-[4-(3-methoxy-propyl)-3,4-dihydro-2H-benzo[1,4]oxazin-6-ylmethoxy]-5-((R)-2-propylamino-propoxy)-piperidine-1-carboxylic acid benzyl ester.

The starting materials are prepared as follows:
a) (3R,4R,5S)-4-(4-Methoxy-phenyl)-3-[4-(3-methoxy-propyl)-3,4-dihydro-2H-benzo[1,4]oxazin-6-ylmethoxy]-5-((R)-2-propylamino-propoxy)-piperidine-1-carboxylic acid benzyl ester
   The title compound is obtained as a yellow oil from (3S,4R,5R)-3-((S)-2-methanesulfonyloxy-propoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (Example 131 a) in analogy to the process used in Example 135a, using propylamine. Rf = 0.19 (EtOAc-triethylamine 100:1); Rt = 4.75 (Gradient I).

### Example 93

### (R)-1-{(3S,4R,5R)-4-[4-((R)-4-Methoxy-3-methylbutylsulfanyl)phenyl]-5-[4-(3-methoxy-propyl)-3,4-dihydro-2H-benzo[1,4]oxazin-6-ylmethoxy]piperidin-3-yloxy}propan-2-ol

A solution of 0.4 mmol of methyl (3R,4R,5S)-4-[4-((R)-4-methoxy-3-methylbutylsulfanyl)-phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate in 4 ml of ethanol and 1 ml of tetrahydrofuran is mixed with 12 mmol of sodium borohydride. The reaction solution is stirred at 50°C for 15 hours and then mixed with 3 ml of dioxane, 3.6 ml of aqueous 40% potassium hydroxide solution and 1 ml of methanol. The reaction mixture is stirred at 90°C for 1 hour and then, at room temperature, poured into water and diluted with tert-butyl methyl ether. The aqueous phase is extracted with tert-butyl methyl ether (2X). The combined organic phases are washed with brine, dried with sodium sulfate and evaporated. The title compound is obtained from the residue by flash chromatography (SiO₂ 60F).

The starting material is prepared as follows:
a) Methyl (3R,4R,5S)-4-[4-((R)-4-methoxy-3-methylbutylsulfanyl)phenyl]-3-[4-(3-methoxy-propyl)-3,4-dihydro-2H-benzo[1,4]oxazin-6-ylmethoxy]-5-((R)-1-oxiranylmethoxy)piperidine-1-carboxylate
   0.5 mmol of methyl (3S,4S,5R)-3-hydroxy-4-[4-((R)-4-methoxy-3-methylbutyl-sulfanyl)-phenyl]-5-[4-(3-methoxypropyl)-3,4-dihydro-2H-benzo[1,4]oxazin-6-ylmethoxy]piperidine-1-carboxylate (Example 83a) and 1 mmol of (R)-1-oxiranyl-methyl toluene-4-sulfonate [113826-06-5] are reacted in analogy to Example 36a. The title compound is identified on the basis of the Rf.

The following compounds are prepared in an analogous manner to the process described in Example 93.

### Examples

### 94 (R)-1-{(3S,4R,5R)-5-[4-(3-Methoxypropyl)-3,4-dihydro-2H-benzo[1,4]oxazin-6-ylmethoxy]-4-[4-(3-methoxypropylsulfanyl)phenyl]piperidin-3-yloxy}propan-2-ol

### 95 (R)-1-{(3S,4R,5R)-4-[4-(4-Methoxybutylsulfanyl)phenyl]-5-[4-(3-methoxypropyl)-3,4-dihydro-2H-benzo[1,4]oxazin-6-ylmethoxy]piperidin-3-yloxy}propan-2-ol

### 102 (R)-1-{(3S,4R,5R)-5-[4-(3-Methoxypropyl)-3,4-dihydro-2H-benzo[1,4]oxazin-6-ylmethoxy]-4-[4-(3-methylsulfanylpropoxy)phenyl]piperidin-3-yloxy}propan-2-ol

starting from benzyl (3S,4S,5R)-3-hydroxy-5-[4-(3-methoxypropyl)-3,4-dihydro-2H-benzo[1,4]oxazin-6-ylmethoxy]-4-[4-(3-methylsulfanylpropoxy)phenyl]piperidine-1-carboxylate (Example 80a).

### Example 96

### (S)-4-{(3S,4S,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}butan-2-ol

The title compound is prepared from 0.63 g of benzyl (3S,4S,5R)-3-((S)-3-hydroxybutoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate in analogy to method B.

The starting materials are prepared as follows:
a) Benzyl (3S,4S,5R)-3-((S)-3-hydroxybutoxy)-4-[4-(3-methoxypropoxy)phenyl]-5-[4-(3-methoxypropyl)-3,4-dihydro-2H-benzo[1,4]oxazin-6-ylmethoxy]piperidine-1-carboxylate
   The title compound is obtained as a yellow oil from 0.807 g of benzyl (3S,4S,5R)-3-[(S)-3-(tert-butyldimethylsilanyloxy)butoxy]-4-[4-(3-methoxypropoxy)phenyl]-5-[4-(3-methoxy-propyl)-3,4-dihydro-2H-benzo[1,4]oxazin-6-ylmethoxy]piperidine-1-carboxylate in analogy to method J. Rf = 0.12 (EtOAc-heptane 2:1); Rt = 5.08 (Gradient I).
b) Benzyl (3S,4S,5R)-3-[(S)-3-(tert-butyldimethylsilanyloxy)butoxy]-4-[4-(3-methoxy-propoxy)phenyl]-5-[4-(3-methoxypropyl)-3,4-dihydro-2H-benzo[1,4]oxazin-6-ylmethoxy]piperidine-1-carboxylate
   0.15 g of sodium hydride (60% dispersion in oil) is added to a solution of 1.68 g of benzyl (3S,4S,5R)-3-hydroxy-4-[4-(3-methoxypropoxy)phenyl]-5-[4-(3-methoxypropyl)-3,4-dihydro-2H-benzo[1,4]oxazin-6-ylmethoxy]piperidine-1-carboxylate (Example 20b) in 10 ml of DMF at 0°C, and the mixture is stirred for 1 hour. It is then cooled to -5°C and 1.25 g of tert-butyl ((S)-3-iodo-1-methylpropoxy)dimethylsilane [134510-70-6] are added dropwise over the course of 1 hour. The reaction mixture is stirred at -5°C for 3 hours and then warmed to room temperature. It is then diluted with tert-butyl methyl ether and poured into ice-water. The resulting mixture is extracted three times with tert-butyl methyl ether. The combined organic phases are washed with brine, dried with sodium sulfate and evaporated. The title compound is obtained as a yellow oil from the residue by flash chromatography (SiO₂ 60F). Rf = 0.45 (EtOAc-heptane 2:1).

The following compounds are prepared in an analogous manner to the process described in Example 96:

### Examples

### 97 (R)-4-{(3S,4S,5R)-4-[4-(3-Methoxypropoxy)phenyl]-5-[4-(3-methoxypropyl)-3,4-dihydro-2H-benzo[1,4]oxazin-6-ylmethoxy]piperidin-3-yloxy}butan-2-ol

### 107 (S)-4-{(3S,4S,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}butan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### 109 (R)-4-{(3S,4S,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}butan-2-ol

starting from benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a)

### Example 98

### (R)-1-{(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}propan-2-ol

The title compound is prepared from 0.282 g of benzyl (3R,4R,5S)-4-[4-(4-methoxybutoxy)-phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate in analogy to method B.

The starting material is prepared as follows:
a) Benzyl (3R,4R,5S)-4-[4-(4-methoxybutoxy)phenyl]-3-[4-(3-methoxypropyl)-3,4-dihydro-2H-benzo[1,4]oxazin-6-ylmethoxy]-5-((R)-1-oxiranylmethoxy)piperidine-1-carboxylate
   0.32 g of benzyl (3S,4S,5R)-3-hydroxy-4-[4-(4-methoxybutoxy)phenyl]-5-[4-(3-methoxy-propyl)-3,4-dihydro-2H-benzo[1,4]oxazin-6-ylmethoxy]piperidine-1-carboxylate (Example 78a) and 0.227 g of (R)-1-oxiranylmethyl toluene-4-sulfonate [113826-06-5] are reacted in analogy to Example 36a. The title compound is obtained as a yellowish oil. Rf = 0.35 (EtOAc-heptan 2:1); Rt = 5.36 (Gradient I).

The following compound is prepared in an analogous manner to the process described in Example 98:

### Example

### 105 (S)-1-{(3S,4R,5R)-4-[4-(4-Methoxybutoxy)phenyl]-5-[4-(3-methoxypropyl)-3,4-dihydro-2H-benzo[1,4]oxazin-6-ylmethoxy]piperidin-3-yloxy}propan-2-ol

using (S)-1-oxiranylmethyl toluene-4-sulfonate [70987-78-9]

### Example 117

### Tetrahydropyran-4-carboxylic acid {(3S,4R,5R)-4-(4-methoxyphenyl)-5-[4-(3-methoxy-propyl)-3,4-dihydro-2H-benzo[1,4]oxazin-6-ylmethoxy]piperidin-3-ylmethyl}amide

The title compound is prepared from benzyl (3R,4R,5R)-4-(4-methoxyphenyl)-3-[4-(3-methoxypropyl)-3,4-dihydro-2H-benzo[1,4]oxazin-6-ylmethoxy]-5-{[(tetrahydropyran-4-carbonyl)amino]methyl}piperidine-1-carboxylate in analogy to method B.

The starting materials are prepared as follows:
a) Benzyl (3R,4R,5R)-4-(4-methoxyphenyl)-3-[4-(3-methoxypropyl)-3,4-dihydro-2H-benzo[1,4]oxazin-6-ylmethoxy]-5-{[(tetrahydropyran-4-carbonyl)amino]-methyl}piperidine-1-carboxylate
   5 mmol of triethylamine and 1 mmol of propanephosphonic anhydride [68957-94-8, T3P] (50% in ethyl acetate) are successively added to a solution of 1 mmol of benzyl (3R,4R,5R)-3-aminomethyl-4-(4-methoxyphenyl)-5-[4-(3-methoxypropyl)-3,4-dihydro-2H-benzo[1,4]oxazin-6-ylmethoxy]piperidine-1-carboxylate (Example 91 b) and 1.1 mmol of tetrahydropyran-4-carboxylic acid [5337-03-1] in 20 ml of dichloromethane at room temperature. After 12 hours, the reaction mixture is diluted with dichloromethane and washed successively with 1N HCl and brine, dried with sodium sulfate and evaporated. The title compound is obtained as a colourless oil from the residue by flash chromatography (SiO₂ 60F). Rf = 0.13 (EtOAc); Rt = 4.63 (Gradient I).

The following compounds are prepared in an analogous manner to the process described in Example 117:

### Examples

### 118 2-Cyclopentyl-N-{(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}acetamide

using cyclopentylacetic acid [1123-00-8]

### 119 (meso-1S,5R,6R)-3-Oxabicyclo[3.1.0]hexan-6-carboxylic acid {(3S,4R,5R)-4-(4-methoxyphenyl)-5-[4-(3-methoxypropyl)-3,4-dihydro-2H-benzo[1,4]oxazin-6-ylmethoxy]piperidin-3-ylmethyl}amide

using (meso-1S,5R,6R)-3-oxabicyclo[3.1.0]hexane-6-carboxylic acid [55780-88-6]

### 120 N-{(3S,4R,5R)-4-(4-Methoxyphenyl)-5-[4-(3-methoxypropyl)-3,4-dihydro-2H-benzo[1,4]oxazin-6-ylmethoxy]piperidin-3-ylmethyl}-2-(meso-1R,5S,6S)-3-oxa-bicyclo[3.1.0]hex-6-ylacetamide

using (meso-1R,5S,6S)-(3-oxabicyclo[3.1.0]hex-6-yl)acetic acid

The starting materials are prepared as follows:
a) (meso-1R,5S,6S)-(3-Oxabicyclo[3.1.0]hex-6-yl)acetic acid
   3 mmol of triethylamine and 0.5 mmol of silver trifluoroacetate are added to a solution of 1 mmol of 1-diazo-3-(meso-1R,5S,6S)-3-oxabicyclo[3.1.0]hex-6-ylpropan-2-one in 70 ml 10:1 tetrahydrofuran-water at -15°C. The reaction mixture is warmed to room temperature and stirred at room temperature for 2 hours. It is diluted with tert-butyl methyl ether, washed with 1M HCl and brine, dried with sodium sulfate and evaporated. The title compound is identified from the residue on the basis of the Rf by flash chromatography (SiO₂ 60F).
b) 1-Diazo-3-(meso-1R,5S,6S)-3-oxabicyclo[3.1.0]hex-6-yl-propan-2-one
   1.2 mmol of triethylamine and 1 mmol of ethyl chloroformate are added to a solution of 1 mmol of (meso-1S,5R,6R)-3-oxabicyclo[3.1.0]hexane-6-carboxylic acid [55780-88-6] in 60 ml of tetrahydrofuran at -15°C. The reaction mixture is warmed to -5°C and stirred at this temperature for 1 hour. It is cooled to -30°C, and 2.5 mmol of a diazomethane solution in ether are added, and the mixture is stirred overnight. It is diluted with tert-butyl methyl ether, washed with saturated aqueous sodium bicarbonate solution and brine, dried with sodium sulfate and evaporated. The title compound is identified from the residue on the basis of the Rf by flash chromatography (SiO₂ 60F).

### 121 4-Methoxycyclohexanecarboxylic acid {(3S,4R,5R)-4-(4-methoxyphenyl)-5-[4-(3-methoxypropyl)-3,4-dihydro-2H-benzo[1,4]oxazin-6-ylmethoxy]-piperidin-3-ylmethyl}-amide

using 4-methoxycyclohexanecarboxylic acid [99183-14-9]

### 122 N-{(3S,4R,5R)-4-(4-Methoxyphenyl)-5-[4-(3-methoxypropyl)-3,4-dihydro-2H-benzo[1,4]oxazin-6-ylmethoxy]piperidin-3-ylmethyl}-2-(tetrahydropyran-4-yl)acetamide

using (tetrahydropyran-4-yl)acetic acid [85064-61-5]

### Example 138

### N-{(3S,4R,5R)-4-[4-(4-Methoxy-butoxy)-phenyl]-5-[4-(3-methoxy-propyl)-3,4-dihydro-2H-benzo[1,4]oxazin-6-ylmethoxy]-piperidin-3-ylmethyl}-acetamide

The title compound is obtained from N-[(3R,4R,5R)-4-[4-(4-methoxy-butoxy)-phenyl]-5-[4-(3-methoxy-propyl)-3,4-dihydro-2H-benzo[1,4]oxazin-6-ylmethoxy]-1-(toluene-4-sulfonyl)-piperidin-3-ylmethyl]-acetamide in analogy to method L.

The starting materials are prepared as follows:
a) N-[(3R,4R,5R)-4-[4-(4-Methoxy-butoxy)-phenyl]-5-[4-(3-methoxy-propyl)-3,4-dihydro-2H-benzo[1,4]oxazin-6-ylmethoxy]-1-(toluene-4-sulfonyl)-piperidin-3-ylmethyl]-acetamide
   The title compound is obtained as a white foam from toluene-4-sulfonic acid (3S,4R,5R)-4-[4-(4-methoxy-butoxy)-phenyl]-5-[4-(3-methoxy-propyl)-3,4-dihydro-2H-benzo[1,4]oxazin-6-ylmethoxy]-1-(toluene-4-sulfonyl)-piperidin-3-ylmethyl ester in analogy to the process described in Example 91 a, b and c. Rf = 0.72 (Dichloromethane-methanol-25% conc. ammonia = 200:20:1); Rt = 4.86 (Gradient I).
b) Toluene-4-sulfonic acid (3S,4R,5R)-4-[4-(4-methoxy-butoxy)-phenyl]-5-[4-(3-methoxy-propyl)-3,4-dihydro-2H-benzo[1,4]oxazin-6-ylmethoxy]-1-(toluene-4-sulfonyl)-piperidin-3-ylmethyl ester
   The title compound is obtained as a yellowish oil from [(3S,4R,5R)-4-[4-(4-methoxy-butoxy)-phenyl]-5-[4-(3-methoxy-propyl)-3,4-dihydro-2H-benzo[1,4]oxazin-6-ylmethoxy]-1-(toluene-4-sulfonyl)-piperidin-3-yl]-methanol in analogy to method H. Rf = 0.46 (EtOAc-heptane = 2:1); Rt = 5.76 (Gradient I).
c) [(3S,4R,5R)-4-[4-(4-Methoxy-butoxy)-phenyl]-5-[4-(3-methoxy-propyl)-3,4-dihydro-2H-benzo[1,4]oxazin-6-ylmethoxy]-1-(toluene-4-sulfonyl)-piperidin-3-yl]-methanol
   The title compound is obtained as a yellow oil from 4-[(3S,4R,5R)-3-hydroxymethyl-5-[4-(3-methoxy-propyl)-3,4-dihydro-2H-benzo[1,4]oxazin-6-ylmethoxy]-1-(toluene-4-sulfonyl)-piperidin-4-yl]-phenol and 1-bromo-3-methoxy-propane [4457-67-4] in analogy to method F. Rf = 0.28 (EtOAc-heptane = 2:1); Rt = 5.07 (Gradient I).
d) 4-[(3S,4R,5R)-3-Hydroxymethyl-5-[4-(3-methoxy-propyl)-3,4-dihydro-2H-benzo[1,4]oxazin-6-ylmethoxy]-1-(toluene-4-sulfonyl)-piperidin-4-yl]-phenol
   A solution of 1 mmol of [(3S,4R,5R)-4-(4-methoxy-phenyl)-5-[4-(3-methoxy-propyl)-3,4-dihydro-2H-benzo[1,4]oxazin-6-ylmethoxy]-1-(toluene-4-sulfonyl)-piperidin-3-yl]-methanol in 5 ml of N,N-dimethylformamide is mixed with 5 mmol of sodium ethanethiolate, and the resulting suspension is heated at 130°C overnight. It is diluted with 1N HCl and extracted with ethyl acetate (2X). The combined organic phases are dried with sodium sulfate and evaporated. The title compound is obtaine as a yellow foam from the residue by flash chromatography (SiO₂ 60F). Rf = 0.13 (EtOAc-heptane = 2:1); Rt = 4.35 (Gradient I).
e) [(3S,4R,5R)-4-(4-Methoxy-phenyl)-5-[4-(3-methoxy-propyl)-3,4-dihydro-2H-benzo[1,4]oxazin-6-ylmethoxy]-1-(toluene-4-sulfonyl)-piperidin-3-yl]-methanol
   The title compound is obtained as a colourless oil from {(3S,4R,5R)-4-(4-methoxyphenyl)-5-[4-(3-methoxy-propyl)-3,4-dihydro-2H-benzo[1,4]oxazin-6-ylmethoxy]-piperidin-3-yl}-methanol in analogy to the process described in Example 1f. Rf = 0.25 (EtOAc-heptane = 2:1); Rt = 4.83 (Gradient I).
f) {(3S,4R,5R)-4-(4-Methoxy-phenyl)-5-[4-(3-methoxy-propyl)-3,4-dihydro-2H-benzo[1,4]oxazin-6-ylmethoxy]-piperidin-3-yl}-methanol
   The title compound is obtained as a yellow oil from (3S,4R,5R)-3-hydroxymethyl-4-(4-methoxy-phenyl)-5-[4-(3-methoxy-propyl)-3,4-dihydro-2H-benzo[1,4]oxazin-6-ylmethoxy]-piperidin-1-carboxylic acid benzyl ester (Example 87c) in analogy to method B. Rt = 3.34 (Gradient I).

The following compounds are prepared in an analogous manner to the process described in Example 138:

### Examples

### 144 N-{(3S,4R,5R)-4-[4-((S)-4-Methoxy-3-methyl-butoxy)-phenyl]-5-[4-(3-methoxy-propyl)-3,4-dihydro-2H-benzo[1,4]oxazin-6-ylmethoxy]-piperidin-3-ylmethyl}-acetamide

using toluene-4-sulfonic acid (S)-4-methoxy-3-methyl-butyl ester

The starting materials are prepared as follows:
a) Toluene-4-sulfonic acid (S)-4-methoxy-3-methyl-butyl ester
   The title compound is prepared from (S)-4-methoxy-3-methyl-butan-1-ol in analogy to method H and identified from the residue on the basis of the Rf by flash chromatography (SiO₂ 60F).
b) (S)-4-Methoxy-3-methyl-butan-1-ol
   A solution of 1 mmol of (S)-4-methoxy-3-methyl-butyronitrile in 10 ml of methanol and 10 ml of a 2M aqueous sodium hydroxid solution is stirred for 16 hours at 80°. The methanol is mostly evaporated and the remaining aqueous phase is acidified to pH 2 with 2M HCl solution and extracted with ethylacetat (3 x 50 ml). The combined organic phases are washed with brine, dried with sodium sulfate and evaporated. The title compound is identified from the residue on the basis of the Rf by flash chromatography (SiO₂ 60F).
c) (S)-4-Methoxy-3-methyl-butyronitrile
   A mixture of 1 mmol of methanesulfonic acid (R)-3-methoxy-2-methyl-propyl ester and 5 mmol of sudium cyanid in 5 ml of dimethylsulfoxid is stirred for 16 hours at 60°C, then cooled to room temperature and 10 ml of water are added. The reaction mixture is extracted with tert-butyl methyl ether (3 x 50 ml), dried with sodium sulfate and evaporated. The title compound is identified from the residue on the basis of the Rf by flash chromatography (SiO₂ 60F).
d) Methanesulfonic acid (R)-3-methoxy-2-methyl-propyl ester
   To a solution of 1 mmol of (S)-3-methoxy-2-methyl-propan-1-ol [913969-31-0] and 5 mmol of triethylamin in 10 ml of dichloromethan are added dropwise 2 mmol of methanesulfonyl chlorid at 0°C. The reaction mixture is stirred for 3 hours at 0°C, then, the mixture is washed with water and 1 M aqueous citric acid solution, dried with sodium sulfate and evaporated. The title compound is obtained as a colourless oil from the residue by flash chromatography (SiO₂ 60F) and used crude in the next step. Rf = 0.57 (diethyl ether).

### 149 N-{(3S,4R,5R)-4-[4-((R)-4-Methoxy-pentyloxy)-phenyl]-5-[4-(3-methoxy-propyl)-3,4-dihydro-2H-benzo[1,4]oxazin-6-ylmethoxy]-piperidin-3-ylmethyl}-acetamide

using toluene-4-sulfonic acid (R)-4-methoxy-pentyl ester

The starting material is prepared as follows:
a) Toluene-4-sulfonic acid (R)-4-methoxy-pentyl ester
   The title compound is obtained from (R)-3-methoxy-butan-1-ol [119784-98-4] in analogy to the process described in Example 144a, b, c and d and identified from the residue on the basis of the Rf by flash chromatography (SiO₂ 60F).

### Example 139

### 3-{(3S,4S,5R)-4-[4-(4-Methoxy-butoxy)-phenyl]-5-[4-(3-methoxy-propyl)-3,4-dihydro-2H-benzo[1,4]oxazin-6-ylmethoxy]-piperidin-3-yloxy}-propan-1-ol

The title compound is prepared from (3S,4S,5R)-3-(3-hydroxy-propoxy)-4-[4-(4-methoxy-butoxy)-phenyl]-5-[4-(3-methoxy-propyl)-3,4-dihydro-2H-benzo[1,4]oxazin-6-ylmethoxy]-piperidine-1-carboxylic acid benzyl ester in analogy to method B.
a) (3S,4S,5R)-3-(3-Hydroxy-propoxy)-4-[4-(4-methoxy-butoxy)-phenyl]-5-[4-(3-methoxy-propyl)-3,4-dihydro-2H-benzo[1,4]oxazin-6-ylmethoxy]-piperidine-1-carboxylic acid benzyl ester
   The title compound is obtained as a colourless oil from (3R,4S,5S)-4-[4-(4-methoxy-butoxy)-phenyl]-3-[4-(3-methoxy-propyl)-3,4-dihydro-2H-benzo[1,4]oxazin-6-ylmethoxy]-5-(3-triisopropylsilanyloxy-propoxy)-piperidine-1-carboxylic acid benzyl ester in analogy to method J. Rf = 0.06 (EtOAc-heptane 2:1); Rt = 5.01 (Gradient I).
b) (3R,4S,5S)-4-[4-(4-Methoxy-butoxy)-phenyl]-3-[4-(3-methoxy-propyl)-3,4-dihydro-2H-benzo[1,4]oxazin-6-ylmethoxy]-5-(3-triisopropylsilanyloxy-propoxy)-piperidine-1-carboxylic acid benzyl ester
   The title compound is obtained as a colourless oil from (3S,4S,5R)-3-hydroxy-4-[4-(4-methoxy-butoxy)-phenyl]-5-[4-(3-methoxy-propyl)-3,4-dihydro-2H-benzo[1,4]oxazin-6-ylmethoxy]-piperidine-1-carboxylic acid benzyl ester (Example 78a) in analogy to the process described in Example 33a. Rf = 0.25 (EtOAc-heptane 1:1); Rt = 8.04 (Gradient III).

### Example 150:

### {(3S,4R,5R)-4-[4-((R)-4-Methoxy-pentyloxy)-phenyl]-5-[4-(3-methoxy-propyl)-3,4-dihydro-2H-benzo[1,4]oxazin-6-ylmethoxy]-piperdin-3-yl}-methanol

According to general procedure L, 1.0 mmol of [(3S,4R,5R)-4-[4-((R)-4-methoxy-pentyloxy)-phenyl]-5-[4-(3-methoxy-propyl)-3,4-dihydro-2H-benzo[1,4]oxazin-6-ylmethoxy]-1-(toluene-4-sulfonyl)-piperidin-3-yl]-methanol are reacted to afford the title compound as a colourless oil. Rf = 0.09 (CH₂Cl₂/MeOH/25% conc. NH₃ 200:20:1); Rt = 3.76 (gradient I).

The starting material(s) is (are) prepared as follows:
a) [(3S,4R,5R)-4-[4-((R)-4-Methoxy-pentyloxy)-phenyl]-5-[4-(3-methoxy-propyl)-3,4-dihydro-2H-benzo[1,4]oxazin-6-ylmethoxy]-1-(toluene-4-sulfonyl)-piperidin-3-yl]-methanol
   A solution of 1.0 mmol of 6-[(3R,4R,5S)-4-[4-((R)-4-methoxy-pentyloxy)-phenyl]-1-(toluene-4-sulfonyl)-5-trityloxymethyl-piperidin-3-yloxymethyl]-4-(3-methoxy-propyl)-3,4-dihydro-2H-benzo[1,4]oxazine and 2.0 mmol of para-toluenesulfonic acid in 20 ml of methanol and 5 ml of tetrahydrofuran is stirred at room temperature for 6 hours. The reaction mixture is quenched with saturated aqueous sodium bicarbonate solution and the methanol is removed under reduced pressure. The residue is diluted with water and extracted with dichloromethane (3x). The combined organic extracts are washed with brine, dried over sodium sulfate, and concentrated under reduced pressure. The residue is purified by flash chromatography to afford the title compound as a yellow oil. Rf = 0.11 (EtOAc/heptane 1:1); Rt = 5.21 (gradient I).
b) 6-[(3R,4R,5S)-4-[4-((R)-4-Methoxy-pentyloxy)-phenyl]-1-(toluene-4-sulfonyl)-5-trityloxymethyl-piperidin-3-yloxymethyl]-4-(3-methyl-propyl)-3,4-dihydro-2H-benzo[1,4]oxazine
   According to general method K, 1.0 mmol of 6-[(3R,4R,5S)-4-[4-((R)-4-methoxy-pentyloxy)-phenyl]-1-(toluene-4-sulfonyl)-5-trityloxymethyl-piperidin-3-yloxymethyl]-4-(3-methoxy-propyl)-4H-benzo[1,4]oxazin-3-one are reacted to afford the title compound as a turbid oil. Rf = 0.29 (EtOAc/heptane 1:1); Rt = 30.16 (gradient II).
c) 6-[(3R,4R,5S)-4-[4-((R)-4-Methoxy-pentyloxy)-phenyl]-1-(toluene-4-sulfonyl)-5-trityloxymethyl-piperidin-3-yloxymethyl]-4-(3-methoxy-propyl)-4H-benzo[1,4]oxazin-3-one
   According to general procedure D, 1.0 mmol of (3R,4R,5S)-4-[4-((R)-4-methoxy-pentyloxy)-phenyl]-1-(toluene-4-sulfonyl)-5-trityloxymethyl-piperidin-3-ol are reacted with 1.2 mmol of 6-chloromethyl-4-(3-methoxy-propyl)-4H-benzo[1,4]oxazin-3-one [857272-02-7] to afford the title compound as a yellow foam. Rf = 0.24 (EtOAc/heptane 1:1); Rt = 6.38 (gradient I).
d) (3R,4R,5S)-4-[4-((R)-4-Methoxy-pentyloxy)-phenyl]-1-(toluene-4-sulfonyl)-5-trityloxymethyl-piperidin-3-ol
   A solution of 1.0 mmol of (3R,4R,5S)-5-hydroxymethyl-4-[4-((R)-4-methoxy-pentyloxy)-phenyl]-1-(toluene-4-sulfonyl)-piperidin-3-ol, 1.0 mmol of trityl chloride, 0.05 mmol of 4-dimethylaminopyridine and 25 mmol of pyridine is stirred at 70°C for 20 hours. The reaction mixture is cooled to room temperature, and partitioned between cold 0.5 M aqueous HCl and tert-butyl methyl ether. The aqueous phase is re-extracted with tert-butyl methyl ether, the combined organic extracts are washed with saturated aqueous sodium bicarbonate solution, then with brine, dried over sodium sulfate and concentrated under reduced pressure. The residue is purified by flash chromatography (SiO₂ 60F) to afford the title compound as a white foam. Rf = 0.60 (EtOAc/heptane 2:1); Rt = 6.00 (gradient I).
e) (3R,4R,5S)-5-Hydroxymethyl-4-[4-((R)-4-methoxy-pentyloxy)-phenyl]-1-(toluene-4-sulfonyl)-piperidin-3-ol
   According to general method I, 1.0 mmol of (3R,4R,5S)-5-hydroxymethyl-4-(4-hydroxy-phenyl)-1-(toluene-4-sulfonyl)-piperidin-3-ol [912347-32-1] and 1.1 mmol of toluene-4-sulfonic acid (R)-4-methoxy-pentyl ester are reacted to afford the title compound as a white solid. Rf = 0.26 (EtOAc/heptane 2:1); Rt = 4.07 (gradient I).
f) Toluene-4-sulfonic acid (R)-4-methoxy-pentyl ester
   According to general method H, 1.0 mmol of (R)-4-methoxy-pentan-1-ol are reacted to afford the title compound as a colourless liquid. Rf = 0.62 (EtOAc/heptane 2:1); Rt = 4.48 (gradient I).
g) (R)-4-Methoxy-pentan-1-ol
   To a solution of 1.0 mmol of (R)-4-methoxy-pentanoic acid in 1 ml of tetrahydrofuran are added 1.5 mmol of borane-tetrahydrofuran complex (1 M in tetrahydrofuran), and the reaction mixture is refluxed for 20 hours. It is then cooled at 0°C, quenched with methanol, and the solvents are removed under reduced pressure. The residue is purified by flash chromatography to afford the title compound as a colourless liquid. Rf = 0.27 (EtOAc/heptane 2:1).
h) (R)-4-Methoxy-pentanoic acid
   A solution of 1.0 mmol of (R)-4-methoxy-pentanenitrile in 2.5 ml of ethanol, 0.5 ml of tetrahydrofuran and 3 ml of 2N aqueous NaOH is stirred at 80°C for 18 hours. The reaction mixture is cooled to room temperature, and the organic solvents are removed under reduced pressure. The residue is extracted with tert-butyl methyl ether (3x). The basic aqueous layer is acidified by addition of 4N aqueous HCl. The aqueous layer is extracted with ethyl acetate (3x), the combined extracts are dried over sodium sulfate, and concentrated under reduced pressure, to afford the title compound as light yellow liquid. Rf = 0.17 (EtOAc/heptane 1:1).
i) (R)-4-Methoxy-pentanenitrile
   To a solution of 1.0 mmol of (R)-4-hydroxy-pentanenitrile in 1.5 ml of tetrahydrofuran at 0°C are added 1.2 mmol of sodium hydride (55% dispersion in oil). The reaction mixture is stirred at 0°C for 1 hour, then 1.1 mmol of methyl iodide are added, and the mixture is stirred at 0°C for 2.5 hours. Saturated aqueous ammunium chloride is added, and the aqueous phase is extracted with tert-butyl methyl ether (3x). The combined organic extracts are dried over sodium sulfate and concentrated under reduced pressure to afford the crude title compound as a light yellow oil, Rf = 0.46 (EtOAc/heptane 1:1).
j) (R)-4-Hydroxy-pentanenitrile
   According to general procedure J, 1.0 mmol of (R)-4-triisopropylsilanyloxypentanenitrile are reacted to afford the title compound as a light yellow liquid. Rf = 0.09 (EtOAc/heptane 1:2).
k) (R)-4-Triisopropylsilanyloxy-pentanenitrile
   A solution of 1.0 mmol of toluene-4-sulfonic acid (R)-3-triisopropylsilanyloxy-butyl ester and 3.5 mmol of sodium cyanide in 1 ml of dimethylsulfoxid is stirred for 20 hours at 60°C. The reaction mixture is cooled to room temperature, diluted with tert-butyl methyl ether and washed with water (3x). The organic phase is dried over sodium sulfate, and concentrated under reduced pressure, affording the title compound as a colourless liquid. Rf = 0.66 (EtOAc/heptane 1:2).
l) Toluene-4-sulfonic acid (R)-3-triisoopropylsilanyloxy-butyl ester
   A solution of 1.0 mmol of toluene-4-sulfonic acid (R)-3-hydroxy-butyl ester [75351-36-9] in 2 ml of dichloromethane is cooled to 0°C, 1.35 mmol of 2,6-lutidine are added, followed by 0.95 mmol of triisopropylsilyl trifluoromethanesulfonate. The reaction mixture is stirred at 0°C for 1.5 hours, diluted with dichloromethane and washed with 0.5N aqueous HCl. The aqueous phase is re-extracted with dichloromethane (3x). The combined organic extracts are dried over sodium sulfate, and concentrated under reduced pressure. The residue is purified by flash chromatography to afford the title compound as a colourless liquid. Rf = 0.52 (EtOAc/heptane 1:2); Rt = 6.63 (gradient I).

## Claims

1. A compound of formula 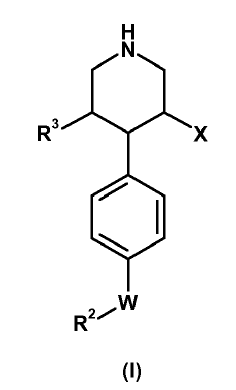 or its pharmaceutically acceptable salt for the inhibition of beta-secretase, cathepsin D, plasmepsin II and/or HIV-protease, in which
(A) R¹ is heterocyclyl substituted by oxo or oxide or as indicated under (B) or (C), in particular azepanyl, benzo[1,3]dioxolyl, benzofuranyl, benzoimidazolyl, 4H-benzo[1,4]oxazinyl, benzooxazolyl, 4H-benzo[1,4]thiazinyl, quinolinyl, chromenyl, dihydro-benzo[e][1,4]diazepinyl, dihydrobenzofuranyl, 3,4-dihydro-2H-benzo[1,4]oxazinyl, dihydro-3H-benzo[1,4]oxazinyl, dihydro-benzo[d][1,3]oxazinyl, 3,4-dihydro-2H-benzo[1,4]thiazinyl, dihydro-2H-1λ6-benzo[1,4]thiazinyl, dihydro-1H-quinazolinyl, 1a,7b-dihydro-1H-cyclo-propa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1H-pyrido[2,3-b][1,4]oxazinyl, pyridyl, 1H-pyrrolizinyl, 1H-pyrrolo[2,3-b]pyridyl, pyrrolyl, tetrahydrobenzo[e][1,4]diazepinyl, 3H-thieno[2,3-d]pyrimidinyl, tetrahydroquinoxalinyl, 1,1a,2,7b-tetrahydrocyclopropa[c]chromenyl, tetrahydropyranyl or triazinyl; or
(B) R¹ is aryl which is substituted by 1-4-acetamidinyl-C₁₋₈alkyl, acyl-C₁₋₈alkoxy-C₁₋₈alkyl, (N-acyl)-C₁₋₈alkoxy-C₁₋₈alkylamino, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkyl-carbamoyl, C₁₋₈alkoxy-C₁₋₈alkylcarbonyl, C₁₋₈alkoxy-C₁₋₈alkylcarbonylamino, 1-C₁₋₈alkoxy-C₁₋₈alkylimidazol-2-yl, 2-C₁₋₈alkoxy-C₁₋₈alkyl-4-oxoimidazol-1-yl, 1-C₁₋₈alkoxy-C₁₋₈-alkyltetrazol-5-yl, 5-C₁₋₈alkoxy-C₁₋₈-alkyltetrazol-1-yl, 6-alkoxy-aminocarbonyl-C₁₋₈alkoxy, C₁₋₈alkoxyaminocarbonyl-C₁₋₈alkyl, C₁₋₈alkoxycarbonyl, C₁₋₈alkoxycarbonyl-C₁₋₈alkoxy, C₁₋₈alkoxycarbonyl-C₁₋₈alkyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₁₋₈alkoxy-C₁₋₈alkylcarbamoyl, (N-C₁₋₈alkyl)-C₁₋₈alkoxy-C₁₋₈alkylcarbonylamino, (N-C₁₋₈alkyl)-C₁₋₈alkoxycarbonylamino, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₁₋₈alkylsulfonylamino-C₁₋₈alkoxy, (N-C₁₋₈alkyl)-C₁₋₈alkylsulfonylamino-C₁₋₈alkyl, C₁₋₈alkylamidinyl, C₁₋₈alkylaminocarbonyl-C₁₋₈alkoxy, di-C₁₋₈alkylaminocarbonyl-C₁₋₈alkoxy, C₁₋₈alkylaminocarbonyl-C₁₋₈alkoxy-C₁₋₈alkyl, C₁₋₈alkylaminocarbonyl-C₁₋₈alkyl, C₁₋₈alkylaminocarbonylamino-C₁₋₈alkoxy, C₁₋₈alkylaminocarbonylamino-C₁₋₈alkyl, di-C₁₋₈alkylaminocarbonyl-C₁₋₈alkyl, C₁₋₈alkylamino-C₂₋₈alkoxy, di-C₁₋₈alkylamino-C₂₋₈alkoxy, C₁₋₈alkylamino-C₁₋₈alkyl, di-C₁₋₈alkylamino-C₁₋₈alkyl, C₁₋₈alkylcarbamoyl, di-C₁₋₈alkylcarbamoyl, C₀₋₈alkylcarbonylamino-C₁₋₈alkoxy, C₀₋₈alkylcarbonylamino, C₀₋₈alkylcarbonylamino-C₁₋₈alkyl, C₁₋₈alkylcarbonyloxy-C₁₋₈alkoxy, C₁₋₈alkylcarbonyloxy-C₁₋₈alkyl, C₁₋₈alkylsulfonyl, C₁₋₈alkylsulfonyl-C₁₋₈alkoxy, C₁₋₈alkylsulfonyl-C₁₋₈alkyl, C₁₋₈alkylsulfonylamino-C₁₋₈alkoxy, C₁₋₈alkylsulfonylamino-C₁₋₈alkyl, carbamoyl, carbamoyl-C₁₋₈alkoxy, carbamoyl-C₁₋₈alkyl, carboxy-C₁₋₈alkoxy, carboxy-C₁₋₈alkoxy-C₁₋₈alkyl, carboxy-C₁₋₈alkyl, cyano, cyano-C₁₋₈alkoxy, cyano-C₁₋₈alkyl, C₃₋₈cycloalkyl-C₁₋₈alkoxy, C₃₋₈cycloalkyl-C₁₋₈alkyl, C₃₋₈cycloalkylcarbonylamino-C₁₋₈alkoxy, C₃₋₈cycloalkylcarbonylamino-C₁₋₈alkyl, O,N-dimethylhydroxylamino-C₁₋₈alkyl, halogen, hydroxy-C₁₋₈alkoxy-C₁₋₈alkoxy, hydroxy-C₁₋₈alkoxy-C₁₋₈alkyl, hydroxy-C₁₋₈alkyl, (N-hydroxy)-C₁₋₈alkylaminocarbonyl-C₁₋₈alkoxy, (N-hydroxy)-C₁₋₈alkylaminocarbonyl-C₁₋₈alkyl, (N-hydroxy)aminocarbonyl-C₁₋₈alkoxy, (N-hydroxy)aminocarbonyl-C₁₋₈alkyl, 2-oxooxazolidinyl-C₁₋₈alkoxy, 2-oxooxazolidinyl-C₁₋₈alkyl, O-methyloximyl-C₁₋₈alkyl, polyhalo-C₁₋₈alkoxy or polyhalo-C₁₋₈alkyl; or
(C) R¹ is aryl which is substituted by 3-acetamidomethylpyrrolidinyl, 3-C₁₋₈alkoxy-C₁₋₈alkyl-pyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolyl-alkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]-triazol-1-yl-alkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl or thiomorpholinyl; or
(D) R¹ is aryl when X is -O-CHR⁶-CO-NR⁴-R¹ or -O-CHR⁶-CO-NR⁴-Z, where Z is Alk-R¹ where Alk is C₁₋₈alkylene; or
(E) R¹ is aryl when X is -O-Z, where Z is Alk-NR⁴-R¹ or X is -Z, where Z is -Alk-NR⁴-R¹, where Alk is C₁₋₈alkylene;
R²
a) is absent when W is cyano; or
b) is C₁₋₈alkyl, C₂₋₈alkenyl, C₂₋₈alkynyl, C₁₋₈alkoxy-C₁₋₈alkyl, C₁₋₈alkoxy-C₃₋₈cycloalkyl-C₁₋₈-alkyl, C₁₋₈alkylsulfanyl-C₁₋₈alkyl, C₁₋₈alkylsulfonyl-C₁₋₈alkyl when W is -O- or -S-;
R³
a) is halogen- and/or hydroxy-substituted C₁₋₈alkoxy, halogen- and/or hydroxy-substituted C₁₋₈alkoxy-C₁₋₈alkoxy, branched C₁₋₈alkoxy-C₁₋₈alkoxy, optionally N-mono- or N,N-di-C₁₋₈-alkylated amino-C₁₋₈alkoxy, optionally N-C₁₋₈alkylated C₁₋₈alkoxy-C₁₋₈alkylamino-C₁₋₈alkoxy, optionally N-mono- or N,N-di-C₁₋₈alkylated amino-C₀₋₈-alkylcarbonyl-C₁₋₈alkoxy, hydroxy-C₀₋₈-alkylcarbonyl-C₀₋₈alkoxy, C₁₋₈alkoxy-C₀₋₈alkylcarbonyl-C₀₋₈alkoxy, C₁₋₈-alkylcarbonylamino-C₁₋₈alkoxy, cyano-C₁₋₈alkoxy, substituted C₃₋₈cycloalkyl-C₀₋₈alkoxy, heterocyclyl-C₀₋₈alkoxy, optionally N-C₁₋₈alkylated heterocyclyl-C₀₋₈alkylamino-C₀₋₈alkylcarbonyl-C₀₋₈alkoxy, C₁₋₈alkyl-sulfonyl-C₁₋₈alkoxy, C₂₋₈alkynyloxy, heterocyclyl-C₂₋₈alkynyloxy, optionally N-mono- or N,N-di-C₁₋₈alkylated amino-C₂₋₈alkynyloxy, N-mono- or N,N-di-C₁₋₈alkylated aminocarbonyl-C₂₋₈alkynyloxy, heterocyclylcarbonyl-C₀₋₈alkoxy, optionally N-mono- or N,N-di-C₁₋₈alkylated amino-C₁₋₈alkyl, optionally N-C₁₋₈alkylated C₁₋₈alkoxy-C₁₋₈alkylamino-C₁₋₈alkyl, optionally N-mono- or N,N-di-C₁₋₈alkylated and optionally hydroxy-substituted amino-C₀₋₈alkyl-carbonyl-C₀₋₈alkyl, optionally N-C₁₋₈alkylated heterocydyl-C₀₋₈alkylamino-C₀₋₈alkylcarbonyl-C₀₋₈alkyl, optionally halogen- or hydroxy-substituted C₁₋₈alkoxy-C₁₋₈alkyl, optionally halogen-or hydroxy-substituted hydroxy-C₁₋₈alkyl, optionally N-C₁₋₈alkylated hydroxy-C₁₋₈alkylamino-C₁₋₈alkyl, heterocyclylcarbonyl-C₀₋₈alkyl, heterocyclylcarbonyl-C₀₋₈alkylamino-C₁₋₈alkyl, heterocyclyl-C₁₋₈alkyl, C₁₋₈alkoxycarbonylamino-C₁₋₈alkyl, optionally halogen-substituted heterocyclyl-C₀₋₈alkylcarbonylamino-C₁₋₈alkyl, optionally halogen-substituted C₃₋₈cycloalkyl-C₀₋₈alkylcarbonylamino-C₁₋₈alkyl or optionally halogen-substituted C₁₋₈alkylcarbonylamino-C₁₋₈alkyl; or additionally
b) is hydroxy, unsubstituted C₁₋₈alkoxy, unsubstituted, unbranched C₁₋₈alkoxy-C₁₋₈-alkoxy or unsubstituted C₃₋₈cycloalkyl-C₀₋₈alkoxy if -W-R² is not C₁₋₈alkoxy;
R⁴ is acyl, C₁₋₈alkoxy-C₁₋₈alkyl, C₁₋₈alkyl, aryl-C₁₋₈alkyl, C₃₋₈cycloalkyl-C₀₋₈alkyl, or hydrogen;
R⁵ is C₁₋₈alkoxycarbonyl-C₁₋₈alkyl, C₁₋₈alkyl, carboxy-C₁₋₈alkyl or hydrogen;
R⁶ is acyl, C₂₋₈alkenyl, C₁₋₈alkyl, aryl-C₁₋₈alkyl or hydrogen;
X is Z, -O-Z or -S-Z, where the bond originating from an oxygen or sulfur atom leads to a saturated C atom of the group Z, or is a group -CHR⁶-Z, -CHOR⁴-Z, -O-CO-Z, -O-CO-R¹, -CO-Z, -C=NOR⁵-Z, -O-CHR⁶-Z, -O-CHR⁶-CO-NR⁴-Z, -O-CHR⁶-CO-NR⁴-R¹, or -O-CHR⁶-R¹;
W is -O-, -S- or cyano;
Z is C₁₋₈-Alk-R¹, C₂₋₈alkenylene-R¹, hydroxy-substituted -Alk-R¹, -O-R¹, -S-R¹, -O-Alk-R¹, -S-Alk-R¹, -Alk-O-R¹, -Alk-S-R¹ or -Alk-NR⁴-R¹, where Alk is C₁₋₈alkylene; and where
(a) X is -CH-R⁶-Z if Z is -O-R¹ or -S-R¹
(b) X is -CH-R⁶-Z if Z is -O-Alk-R¹ or -S-Alk-R¹; and
(c) Z is C₂₋₈alkenylene-R¹, -Alk-O-R¹, -Alk-S-R¹ or -Alk-NR⁴-R¹ if X is Z;
or pharmaceutically acceptable salt or prodrug thereof, or where one or more atoms are replaced by their stable, non-radioactive isotopes;
for the manufacture of a medicament for the inhibition of beta-secretase, cathepsin D, plasmepsin II and/or HIV-protease.

2. Compound according to claim 1 of formula (IA) 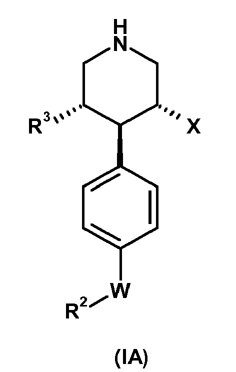 or its pharmaceutically acceptable salt, wherein R², R³, W and X are each as defined in claim 1.

3. Compound according to any one of claims 1 to 2, wherein
X is -CHR⁶-Alk-R¹, -Alk-NR⁴-R¹, -Alk-O-R¹, -Alk-S-R¹, C₂₋₈-Alkenylen-R¹, -CH(OR⁴)-Alk-R¹, -CHR⁶-Alk-R¹, -CHR⁶-O-R¹, -CHR⁶-O-Alk-R¹, -CHR⁶-S-R¹, -CHR⁶-S-Alk-R¹, -CO-Alk-R¹, -C(=NOR⁵)-Alk-R¹, -O-Alk-NR⁴-R¹, -O-Alk-R¹, -O-Alk-O-R¹, -O-CO-R¹, -O-CO-Alk-R¹, -O-CHR⁶-R¹, -O-CHR⁶-Alk-R¹, -O-CHR⁶-CO-NR⁴-R¹ or -O-CHR⁶-CO-NR⁴-Alk-R¹, where Alk is C₁₋₈alkylene.

4. Compound according to any one of claims 1 to 3, wherein
R³
a) is halogen- and/or hydroxy-substituted C₁₋₈alkoxy, halogen- and/or hydroxy-substituted C₁₋₈alkoxy-C₁₋₈alkoxy, branched C₁₋₈alkoxy-C₁₋₈alkoxy, optionally N-mono- or N,N-di-C₁₋₈alkylated amino-C₁₋₈alkoxy, optionally N-mono- or N,N-di-C₁₋₈alkylated amino-C₀₋₈alkylcarbonyl-C₁₋₈alkoxy, substituted C₃₋₈cycloalkyl-C₀₋₈alkoxy, optionally C₁₋₈alkoxy or hydroxy-substituted heterocyclyl-C₀₋₈alkoxy, heterocyclylcarbonyl-C₀₋₈alkoxy, heterocyclylcarbonyl-C₀₋₈alkyl, optionally halogen-substituted heterocyclyl-C₀₋₈alkyl-carbonylamino-C₁₋₈alkyl, optionally halogen-substituted C₃₋₈cycloalkyl-C₀₋₈alkylcarbonyl-amino-C₁₋₈alkyl or optionally halogen-substituted C₁₋₈alkylcarbonylamino-C₁₋₈alkyl; or additionally
b) hydroxy, unsubstituted C₁₋₈alkoxy, unsubstituted, unbranched C₁₋₈alkoxy-C₁₋₈alkoxy or unsubstituted C₃-₈cycloalkyl-C₀₋₈alkoxy if -W-R² is not C₁₋₈alkoxy.

5. Compound according to any one of claims 1 to 4, wherein
R² is C₁₋₈alkyl, C₂₋₈alkenyl, C₁₋₈alkoxy-C₁₋₈alkyl, C₁₋₈alkoxy-C₃₋₈cycloalkyl-C₁₋₈alkyl, C₃₋₈cycloalkyl-C₀₋₈alkoxy-C₁₋₈alkyl-, C₁₋₈alkylsulfanyl-C₁₋₈alkyl, C₁₋₈alkylsulfonyl-C₁₋₈alkyl;
R³
a) hydroxy-substituted C₁₋₈alkoxy, hydroxy-substituted C₁₋₈alkoxy-C₁₋₈alkoxy, branched C₁₋₈alkoxy-C₁₋₈alkoxy or C₁₋₈alkylcarbonylamino-C₁₋₈alkyl; or additionally
b) hydroxy, unsubstituted C₁₋₈alkoxy or unsubstituted, unbranched C₁₋₈alkoxy-C₁₋₈alkoxy if R² is not C₁₋₈alkyl;
and
W is -O-

6. Compound according to any one of claims 1 to 5, wherein
R¹ is substituted chromenyl or 3,4-dihydro-2H-benzo[1,4]oxazinyl;
R² is C₁₋₈alkyl, C₂₋₈alkenyl, C₁₋₈alkoxy-C₁₋₈alkyl, C₁₋₈alkoxy-C₃₋₈cycloalkyl-C₁₋₈alkyl, C₃₋₈cycloalkyl-C₀₋₈alkoxy-C₁₋₈alkyl, C₁₋₈alkylsulfanyl-C₁₋₈alkyl;
R³
a) hydroxy-substituted C₁₋₈alkoxy, hydroxy-substituted C₁₋₈alkoxy-C₁₋₈alkoxy, branched C₁₋₈alkoxy-C₁₋₈alkoxy or C₁₋₈alkylcarbonylamino-C₁₋₈alkyl; or additionally
b) hydroxy, unsubstituted C₁₋₈alkoxy or unsubstituted, unbranched C₁₋₈alkoxy-C₁₋₈alkoxy if R² is not C₁₋₈alkyl;
R⁶ is C₁₋₈alkyl or hydrogen;
X is -CHR⁶-Alk-R¹ or -O-Alk-R¹, where Alk is C₁₋₈alkylene; and
W is -O-.

7. A compound of the general formula (I) or (IA), or a pharmaceutically acceptable salt thereof, according to any one of claims 1 to 6 for the prevention, delay of progression or treatment of Alzheimer Disease, malaria or HIV infection.

8. A pharmaceutical preparation for the prevention, delay of progression or treatment of Alzheimer disease, malaria or HIV infection, whereby said preparation contains a compound of the general formula (I) or (IA), or a pharmaceutically acceptable salt thereof, according to any one of claims 1 to 6, as well as commonly used ingredients.


SEQUENCE LISTING

<110>  Speedel Experimenta AG

<120>  Substituted piperidines as therapeutic compounds

<130>  SP-P2231EP01

<160>  4

<170>  PatentIn version 3.3

<210>  1
<211>  9
<212>  PRT
<213>  Artificial

<220>
<223>  beta-secretase peptide substrate

<400>  1

Glu Val Asn Leu Asp Ala Glu Phe Lys
1               5


<210>  2
<211>  7
<212>  PRT
<213>  Artificial

<220>
<223>  cathepsin D peptide substrate

<400>  2

Pro Thr Glu Phe Phe Arg Leu
1               5


<210>  3
<211>  8
<212>  PRT
<213>  Artificial

<220>
<223>  plasmepsin peptide substrate


<220>
<221>  X
<222>  (3)..(3)
<223>  X is Norleucine

<400>  3

Glu Arg Xaa Phe Leu Ser Phe Pro
1               5


<210>  4
<211>  11
<212>  PRT
<213>  Artificial

<220>
<223>  HIV protease peptide substrate

<220>
<221>  X
<222>  (10)..(10)
<223>  X is Norleucine

<400>  4

His Lys Ala Arg Val Leu Tyr Glu Ala Xaa Ser
1               5                   10